(19) Europäisches Patentamt
European Patent Office
Office européen des brevets

(11) EP 0 937 044 B1

(12) **FASCICULE DE BREVET EUROPEEN**

(45) Date de publication et mention de la délivrance du brevet:
**30.01.2002 Bulletin 2002/05**

(21) Numéro de dépôt: **97936724.0**

(22) Date de dépôt: **31.07.1997**

(51) Int Cl.[7]: **C07D 213/30**, C07D 213/38, C07D 213/40, C07D 215/14, C07D 215/12, C07D 471/04, A61K 31/47, C07D 213/36, C07D 471/14, A61K 31/475

(86) Numéro de dépôt international:
**PCT/FR97/01436**

(87) Numéro de publication internationale:
**WO 98/05642 (12.02.1998 Gazette 1998/06)**

(54) **COMPOSES ACTIVATEURS DU CANAL CFTR, ET COMPOSITIONS PHARMACEUTIQUES LES CONTENANT**

AKTIVATOREN DES CFRT-KANALS UND SIE ENTHALTENDE PHARMAZEUTISCHE ZUSAMMENSETZUNGEN

CFTR CHANNEL ACTIVATOR COMPOUNDS AND PHARMACEUTICAL COMPOSITIONS CONTAINING SAME

(84) Etats contractants désignés:
**CH DE FR GB IT LI**

(30) Priorité: **01.08.1996 FR 9609721**

(43) Date de publication de la demande:
**25.08.1999 Bulletin 1999/34**

(73) Titulaire: **CENTRE NATIONAL DE LA RECHERCHE SCIENTIFIQUE (CNRS)**
**75016 Paris (FR)**

(72) Inventeurs:
- **BECQ, Frédéric**
  **F-13100 Aix en Provence (FR)**
- **METTEY, Yvette**
  **F-86280 Saint-Benoît (FR)**
- **VIERFOND, Jean-Michel**
  **F-94700 Maisons Alfort (FR)**
- **VERRIER, Bernard**
  **F-13360 Roquevaire (FR)**
- **GOLA, Maurice**
  **F-13400 Aubagne (FR)**

(74) Mandataire: **Grosset-Fournier, Chantal Catherine**
**Grosset-Fournier & Demachy s.a.r.l. 20, rue de Maubeuge**
**75009 Paris (FR)**

(56) Documents cités:
**EP-A- 0 480 717**
**WO-A-94/03431**
**WO-A-96/16084**

- **CHEMICAL ABSTRACTS, vol. 110, no. 15, 10 avril 1989 Columbus, Ohio, US; abstract no. 135039, XP002021980 & I.Y. MAINAGASHEV ET AL.: KHIM. GETEROTSIKL. SOEDIN., no. 4, 1988, pages 514-520,**
- **CHEMICAL ABSTRACTS, vol. 106, no. 7, 16 février 1987 Columbus, Ohio, US; abstract no. 49539, XP002021985 & V.V. LAPACHEV ET AL.: KHIM. GETEROSIKL. SOEDIN., no. 6, 1986, pages 802-809,**
- **CHEMICAL ABSTRACTS, vol. 93, no. 5, 4 août 1980 Columbus, Ohio, US; abstract no. 46363, XP002021990 & T. KONAKAHARA ET AL.: HETEROCYCLES, vol. 14, no. 4, 1980, pages 393-396,**
- **CHEMICAL ABSTRACTS, vol. 92, no. 3, 21 janvier 1980 Columbus, Ohio, US; abstract no. 21823k, XP002045767 & E.H. SUND ET AL.: TEX. J. SCI., vol. 31, no. 3, 1979, pages 294-295,**
- **CHEMICAL ABSTRACTS, vol. 91, no. 21, 19 novembre 1979 Columbus, Ohio, US; abstract no. 175163m, XP002045768 & J.-M. VIERFOND ET AL.: J. HETEROCYCL. CHEM., vol. 16, no. 4, 1979, pages 753-755,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 937 044 B1

- **CHEMICAL ABSTRACTS, vol. 81, no. 11, 16 septembre 1974 Columbus, Ohio, US; abstract no. 63583k, XP002045769 & J.F. WOLFE ET AL.: JOURNAL OF ORGANIC CHEMISTRY, vol. 39, no. 14, 1974, EASTON US, pages 2006-2010,**
- **A.R.E. CAREY ET AL.: JOURNAL OF THE CHEMICAL SOCIETY, PERKIN TRANSACTIONS 2, vol. 11, 1993, LETCHWORTH GB, pages 2285-2296, XP002045764**
- **T. KONAKAHARA ET AL.: JOURNAL OF THE CHEMICAL SOCIETY, PERKIN TRANSACTIONS 1, vol. 7, 1987, LETCHWORTH GB, pages 1489-1493, XP002045765**
- **R.A.M. O'FERRALL ET AL.: JOURNAL OF THE CHEMICAL SOCIETY, PERKIN TRANSACTIONS 2, vol. 12, 1994, LETCHWORTH GB, pages 2461-2470, XP002045766**
- **PATENT ABSTRACTS OF JAPAN vol. 096, no. 012, 26 décembre 1996 & JP 08 198875 A (CHUGAI PHARMACEUT. CO., LTD.), 6 août 1996,**
- **CHEMICAL ABSTRACTS, vol. 120, no. 19, 9 mai 1994 Columbus, Ohio, US; abstract no. 241028s, XP002045770 & M.J. STUTTS ET AL.: J. BIOL. CHEM., vol. 269, no. 12, 1994, pages 8667-8674,**
- **F. BECQ ET AL.: PROC. NATL. ACAD. SCI. USA, vol. 91, septembre 1994, pages 9160-9164, XP000611989 cité dans la demande**


Remarques:

Le dossier contient des informations techniques présentées postérieurement au dépôt de la demande et ne figurant pas dans le présent fascicule.

## Description

La présente invention a pour objet des composés activateurs du canal CFTR, des compositions pharmaceutiques contenant ces derniers, ainsi que leur utilisation dans le cadre du traitement de pathologies telles que la mucoviscidose.

Dans une cellule épithéliale, les transports d'eau et d'électrolytes sont associés à une augmentation des perméabilités membranaires pour les ions $K^+$, $Na^+$ et $Cl^-$. Ces mouvements sont liés à l'activité des canaux ioniques, à savoir des protéines spécialisées, intégrés dans la membrane permettant la diffusion passive des ions. Les techniques d'électrophysiologie moléculaire (patch-clamp) permettent l'enregistrement au niveau unitaire des ouvertures et des fermetures d'un canal ionique et rendent possible l'étude des transports ioniques transépithéliaux, de leurs régulations et de leurs dérèglements pathologiques.

Parmi les nombreuses pathologies associées à la physiologie des cellules épithéliales, la mucoviscidose est considérée également comme une pathologie des canaux ioniques dans la mesure où la protéine impliquée est un canal chlorure, le canal CFTR pour "Cystic Fibrosis Transmembrane conductance Regulator". La mucoviscidose ou Cystic Fibrosis (CF) dans la terminologie Anglo-saxonne est la maladie génétique autosomique récessive la plus commune dans les populations caucasiennes. Aux Etats-Unis et dans la plupart des pays d'Europe, la fréquence des porteurs du gène CF est de 1 sur 20 à 1 sur 30. La mucoviscidose affecte les glandes exocrines de l'organisme humain. Les principaux sites d'expression de la protéine CFTR sont le pancréas exocrine, les poumons, les glandes sudoripares, l'intestin et le tissu cardiaque. L'intérêt porté à cette maladie a eu des conséquences importantes pour la compréhension des mécanismes sécrétoires des cellules épithéliales normales. Les cellules épithéliales des glandes exocrines de l'intestin, du pancréas ou des poumons contrôlent le transport de sel et d'eau dans ces organes. Dans la mucoviscidose, des mutations du gène CF altèrent les propriétés et la fonction du canal CFTR. Le transport électrolytique devient alors anormal et conduit à des troubles obstructifs chroniques pulmonaires, à une insuffisance pancréatique, à des affections bactériennes pulmonaires, à une sécrétion sudoripare anormalement concentrée et à une infertilité masculine. Le défaut de sécrétion est lié au fonctionnement de canaux ioniques sélectifs pour les ions chlorure (canaux CFTR), localisés dans la membrane apicale des cellules et dont l'activité est contrôlée par la voie de l'AMP cyclique.

La protéine CFTR est une glycoprotéine de 1480 amino-acides, d'un poids moléculaire de 170 kD répartis en cinq domaines (Riordan et al., 1989), deux segments transmembranaires avec chacun 6 hélices alpha (numérotées de 1 à 12 comportant chacune de 21 à 22 amino acides), deux domaines de fixation des nucléotides (NBF1 et 2) et un large domaine hydrophile de régulation (domaine R). La protéine CFTR, de par sa structure moléculaire appartient à la famille des transporteurs membranaires (ABC pour ATP-binding cassette).

Les transporteurs ABC constituent une famille de protéines membranaires très conservées dans l'évolution. Elles sont impliquées dans la translocation de substrats variés à travers les membranes cellulaires. Cependant, alors que chez les procaryotes de nombreux couples transporteurs/substrats ont été définis, ces informations sont plus rares chez les eucaryotes. Chez les mammifères, la plupart des transporteurs ABC sont associés à une pathologie. Citons la protéine CFTR impliquée dans la mucoviscidose, la P glycoprotéine (MDR ; Multi Drug Resistance) impliquée dans le rejet de drogues cytotoxiques antitumorales et la protéine ABC1, nouvellement décrite qui joue un rôle essentiel dans l'endocytose de corps apoptotiques par le macrophage. La CFTR contrôle le transport de chlorure transépithélial et l'hydratation des compartiments muqueux, alors qu'une des isoformes de la MDR est impliquée dans la translocation de la phosphatidylcholine. Ces trois protéines ABC qui ont une structure à deux fois 6 segments transmembranaires, disposent de deux domaines qui lient et hydrolysent les nucléotides (NBF) et d'un domaine régulateur. La régulation de la CFTR a été particulièrement étudiée.

Deux processus complexes contrôlent l'activité du canal CFTR : la phosphorylation du domaine R par des protéines kinases et la fixation (et peut-être l'hydrolyse) d'ATP sur les deux domaines NBF. La déphosphorylation du canal CFTR entraîne une perte d'activité du canal jusqu'à sa fermeture (Tabcharani et al., 1991, Becq et al., 1993a, Becq et al., 1994). En outre, le canal CFTR est associé à une phosphatase membranaire qui contrôle l'activité et l'état de phosphorylation du canal (Becq et al., 1993b, Becq et al., 1994).

Le gène codant pour la protéine CFTR, a été isolé par clonage moléculaire et identifié sur le chromosome 7 (Kerem et al., 1989, Riordan et al., 1989). L'identification du gène et son implication dans la mucoviscidose ont été confirmés par la localisation d'une délétion de trois paires de bases dans une région codante (exon 10) du gène CF provenant de patients CF. Cette mutation correspond à la délétion d'une phénylalanine en position 508 ($\Delta$F508) de la protéine dans le NBFI. La fréquence d'apparition de cette mutation est de 70% en moyenne dans les analyses génétiques (Tsui & Buchwald, 1991). Les conséquences de cette mutation sont dramatiques car la protéine anormale issue de la transcription du gène muté ($\Delta$F508) n'est plus capable d'assurer ses fonction dans le transport de chlorure des cellules épithéliales affectées. L'absence de courant chlore après stimulation des cellules épithéliales des glandes exocrines par l'AMPc est la principale caractéristique montrant la présence d'une anomalie sur le gène CF et notamment de la mutation ($\Delta$F508). Plus de 300 mutations ont été identifiées à ce jour sur le gène CF. La densité de mutations la

plus élevé se trouve dans les deux domaines de fixation des nucléotides. La mutation (ΔF508) se retrouve dans 70% des cas et 50% des patients sont homozygotes pour cette mutation. Sept autres mutations importantes sont présentes avec des fréquences supérieures à 1%. La mutation G551D correspond à la substitution d'un résidu glycine (G) en position 551 de la protéine par un acide aspartique (D). Les patients porteurs de ce mutant ont une pathologie sévère avec une insuffisance pancréatique et des troubles pulmonaires graves (Cutting et al., 1990). La fréquence d'observation et cette mutation atteint 3 à 5 % chez certaines populations CF. A l'inverse de la délétion ΔF508, la protéine CFTR portant la mutation G551D est mature et est incorporée dans la membrane (Gregory et al., 1991). Cependant, la mutation entraîne une imperméabilité membranaire et la stimulation de la voie de l'AMPc n'ouvre pas le canal associé à l'expression de ce mutant (Gregory et al., 1991, Becq et al., 1994).

D'autres mutations comme R117H, R334W et R347P apparaissent avec des fréquences basses de 0.8, 0.4 et 0.5% respectivement et sont associées à une pathologie moins grave (Sheppard et al., 1993). L'expression de ces trois mutants génère une forme mature glycosylée de la protéine en accord avec son insertion dans la membrane.

Les trois mutants sont capables toutefois de répondre à une stimulation de la voie de l'AMP par l'ouverture des canaux. L'amplitude des courants, la conductance unitaire et la probabilité d'ouverture du canal associé avec chacun des trois mutants sont modifiées par rapport au canal CFTR normal (Sheppard et al., 1993, Becq et al., 1994). La régulation par les kinases/phosphatases semble toutefois normale pour ces différents mutants, y compris les mutants G551D et ΔF508 (Becq et al., 1994).

Ainsi, ces observations montrent qu'il est possible pharmacologiquement d'activer un grand nombre de mutants CFTR, y compris G551D et ΔF508. Malgré un défaut d'adressage de la protéine ΔF508 dans les membranes des cellules épithéliales affectées par la mucoviscidose plusieurs groupes ont montré que cette protéine pouvait être présente de façon fonctionnelle dans les membranes (Dalemans et al., 1991, Drumm et al., 1991, Becq et al., 1994). Ainsi, il apparaît nécessaire et primordial de développer une stratégie d'ouvreurs du canal CFTR pour optimiser les chance de succès d'une thérapie mais aussi pour remplacer la thérapie génique lorsque celle-ci n'est pas nécessaire (mutations autres que ΔF508).

Malgré les progrès réalisés sur la génétique de la mucoviscidose, la biologie et la biochimie de la protéine CFTR, la pharmacologie des ouvreurs du canal CFTR est peu développée. Trois familles de molécules sont aujourd'hui mise en avant pour leurs propriétés d'activateurs ou d'ouvreurs du canal CFTR ; les phénylimidazothiazoles (lévamisole et bromotétramisole), les benzimidazolones (NSOO4) et les xanthines (IBMX, théophylline...).

1) les phénylimidazothiazoles (lévamisole et bromotétramisole)

Il a été récemment montré que le lévamisole et le bromotétramisole permettent, en inhibant une phosphatase membranaire, un contrôle de l'activité et du niveau de phosphorylation du canal CFTR (Becq et al., 1994,). Ces composés ouvrent le canal CFTR de manière dose-dépendante (Becq et al 1996) et agissent sur le canal CFTR présentant des mutations à l'origine de la maladie (Becq et al., 1994). Le mode d'action de ces activateurs est encore incertain. Ces molécules n'agissent pas par les voies classiques de l'AMPc ou du calcium intracellulaire. Les composés de la famille du bromotétramisole ont déjà une utilisation thérapeutique (Grem, 1990) et le lévamisole est utilisé dans certaines thérapies pulmonaires (Van Eygen et al., 1976, Dils, 1979). Ces dernières propriétés représentent un avantage certain pour amorcer des tests en clinique. Toutefois ces molécules ne semblent pas pouvoir agir dans toutes les cellules. Les cellules intestinales répondent mal et l'ouverture du CFTR après expression dans l'ovocyte de *Xenope* ne peut pas être déclenchée. De plus, dans un modèle de souris transgéniques présentant la mutation G551D/G551D, le bromotétramisole n'a pas l'effet activateur attendu. Les effets de ces molécules semblent donc limités.

2) Les benzimidazolones (NSOO4)

Récemment, Gribkoff et al., 1994 ont montré que le NS004, un composé (benzimidazolone) dérivé du noyau imidazole comme le lévamisole peut dans certaines conditions (lorsque le CFTR a été phosphorylé) ouvrir le canal. Les benzimidazolones sont toutefois également activateurs de nombreux canaux potassium (Olesen et al., 1994) et sont de ce fait peu spécifiques pour le CFTR.

3) Les xanthines (IBMX, théophylline ...).

Les xanthines comme l'IBMX (3-isobutyl-1-méthylxanthine) sont des activateurs du CFIR. Le mécanisme d'action est encore mal connu et plusieurs possibilités existent. Les xanthines peuvent en inhibant les phosphodiesterases intracellulaires (enzymes de dégradation de l'AMPc) faire augmenter le taux d'AMPc et donc activer CFTR. D'autres possibilités sont aujourd'hui avancées comme la fixation des xanthines sur les sites de fixation pour les nucléotides (NBF) du CFTR.

La présente invention a pour but de fournir des composés activateurs du canal CFTR qui sont davantage

spécifiques du CFTR que les composés activateurs du canal CFTR décrits jusqu'à maintenant.

A ce titre, la présente invention a pour but de fournir de nouveaux médicaments destinés au traitement de pathologies associées à des troubles des flux ioniques transmembranaires, notamment de chlore, dans les cellules épithéliales d'un organisme humain ou animal.

La présente invention a plus particulièrement pour but de fournir de nouveaux médicaments susceptibles d'être utilisés dans le cadre du traitement de la mucoviscidose, de la prévention du rejet de drogues cytotoxiques (notamment antitumorales), ou de la prévention ou du traitement des obstructions des voies bronchiques ou des voies digestives (notamment pancréatique ou intestinale) ou encore dans le cadre du traitement de maladies cardio-vasculaires.

Un autre but de la présente invention est celui de fournir un procédé de préparation des composés et compositions pharmaceutiques de l'invention.

La présente invention a pour objet l'utilisation de composés correspondant a des dérivés de benzo[c]quinolizisium de formule générale (III) suivante :

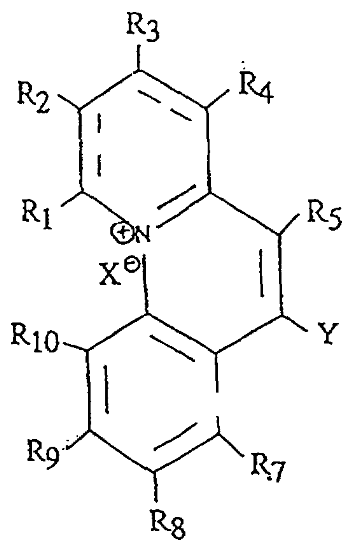

(III)

dans laquelle :

- l'hétérocycle A est aromatique ou non, étant entendu que dans ce dernier cas l'atome d'azote de cet hétérocycle est lié par une double liaison au carbone en position 4a,
- $R_1$, $R_2$, $R_3$, $R_4$, $R_5$, $R_7$, $R_8$, $R_9$ et $R_{10}$, représentent, indépendamment les uns des autres :
  . un atome d'hydrogène, ou de brome, ou de fluor, ou
  . un atome d'halogène, notamment un atome de chlore, ou
  . un groupe alkyle, alkoxy, carbonyle, ou oxycarbonyle, linéaire ou ramifié, d'environ 1 à environ 10 atomes de carbone, ces groupes étant le cas échéant substitués, notamment par un halogène, et/ou par un hydroxyle, et/ou par une amine (primaire, secondaire ou tertiaire), et/ou par un cycle aromatique et/ou aliphatique, d'environ 5 à environ 10 atomes de carbone dans le cycle, ces cycles étant eux-mêmes, le cas échéant, substitués notamment par un halogène, et/ou par un hydroxyle, et/ou par une amine (primaire, secondaire ou tertiaire), et/ou par un groupe alkyle, alkoxy, carbonyle, ou oxycarbonyle, ces groupes étant tels que définis ci-dessus, ou
  . un cycle aromatique ou aliphatique, d'environ 5 à environ 10 atomes de carbone dans le cycle, ce cycle étant lui-même, le cas échéant, substitué notamment par un halogène, et/ou par un hydroxyle, et/ou par une amine (primaire, secondaire ou tertiaire), et/ou par un groupe alkyle, alkoxy, carbonyle, ou oxycarbonyle, ces groupes étant tels que définis ci-dessus, ou
  . un groupe $-OR_a$, $R_a$ représentant un atome d'hydrogène, ou un groupe alkyle, carbonyle, ou oxycarbonyle, linéaire ou ramifié, ces groupes étant tels que définis ci-dessus, ou un cycle aromatique ou aliphatique, ces cycles étant tels que définis ci-dessus, ou
  . un groupe $-NR_bR_c$, $R_b$ et $R_c$, indépendamment l'un de l'autre, représentant un groupe alkyle, alkoxy, carbonyle, ou oxycarbonyle, linéaire ou ramifié, ces groupes étant tels que définis ci-dessus, ou un cycle aromatique ou

aliphatique, ces cycles étant tels que définis ci-dessus, ou

. lorsque $R_1$ et $R_2$, ou $R_3$ et $R_4$, et/ou $R_4$ et $R_5$, et/ou $R_7$ et $R_8$, et/ou $R_8$ et $R_9$, et/ou $R_9$ et $R_{10}$, ne représentent pas les différents atomes ou groupes ou cycles mentionnés ci-dessus, alors $R_1$ en association avec $R_2$, ou $R_2$ en association avec $R_3$, et/ou $R_3$ en association avec $R_4$, et/ou $R_4$ en association avec $R_5$, et/ou $R_7$ en association avec $R_8$, et/ou $R_8$ en association avec $R_9$, et/ou $R_9$ en association avec $R_{10}$, forment respectivement avec $C_1$ et $C_2$, ou avec $C_2$ et $C_3$, ou avec $C_3$ et $C_4$, ou avec $C_4$, $C_{4a}$ et $C_5$, ou avec $C_7$ et $C_8$, ou avec $C_8$ et $C_9$, ou avec $C_9$ et $C_{10}$, un cycle aromatique ou aliphatique de 5 à 10 atomes de carbone, ce cycle étant le cas échéant substitué, notamment par un halogène, et/ou par un groupe alkyle, alkoxy, carbonyle, ou oxycarbonyle, et/ou par un cycle aromatique ou aliphatique, ces groupes ou cycles étant tels que définis ci-dessus, ou

. lorsque $R_3$ et $R_4$ ne représentent pas les différents atomes ou groupes ou cycles mentionnés ci-dessus, alors $R_3$ en association avec $R_4$ forment un groupe indole de formule

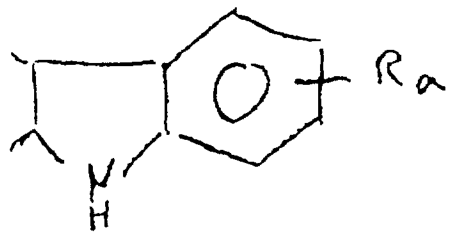

dans laquelle $R_a$ est tel que défini ci-dessus,

- Y représente :

. un groupe $-OR_d$, $R_d$ représentant un atome d'hydrogène, ou un groupe alkyle, carbonyle, ou oxycarbonyle, linéaire ou ramifié, ces groupes étant tels que définis ci-dessus, ou un cycle aromatique ou aliphatique, ces cycles étant tels que définis ci-dessus, ou

. un groupe $-NR_eR_f$, $R_e$ et $R_f$ indépendamment l'un de l'autre, représentant un groupe alkyle, alkoxy, carbonyle, ou oxycarbonyle, linéaire ou ramifié, ces groupes étant tels que définis ci-dessus, ou un cycle aromatique ou aliphatique, ces cycles étant tels que définis ci-dessus,

. étant entendu que lorsque $R_d$, ou l'un au moins de $R_e$ ou de $R_f$, ne représentent pas l'un des différents atomes ou groupes ou cycles mentionnés ci-dessus, alors $R_d$, ou l'un au moins de $R_e$ ou de $R_f$, en association avec $R_5$, ou en association avec $R_7$, forment respectivement avec $C_5$ et $C_6$, ou avec $C_6$, $C_{6a}$ et $C_7$, un hétérocycle aromatique ou aliphatique de 5 à 10 atomes de carbone, le cas échéant substitué, notamment par un halogène, et/ou par un groupe alkyle, alkoxy, carbonyle, ou oxycarbonyle, et/ou par un cycle aromatique ou aliphatique, ces groupes ou cycles étant tels que définis ci-dessus,

- X représente un atome sous forme anionique, tel qu'un atome d'halogène, notamment un atome de brome ou de chlore, ou un groupe d'atomes sous forme anionique, tel qu'un perchlorate, et l'azote de l'hétérocycle A de la formule (I) est sous forme quaternaire et est lié d'une part par liaison covalente au carbone en position 11, et, d'autre part, par liaison ionique à X défini ci-dessus,

* étant entendu que lorsque $R_1$ et $R_{10}$ ne représentent pas l'un des différents atomes ou groupes ou cycles mentionnés ci-dessus, alors $R_1$ en association avec $R_{10}$ forment avec $C_1$, l'azote de l'hétérocycle A de la formule (I), $C_{11}$, et $C_{10}$, un hétérocycle aromatique ou aliphatique de 5 à 10 atomes de carbone, le cas échéant substitué, notamment par un halogène, et/ou par un groupe alkyle, alkoxy, carbonyle; ou oxycarbonyle, et/ou par un cycle aromatique ou aliphatique, ces groupes ou cycles étant tels que définis ci-dessus, pour la préparation de médicaments destinés au traitement de pathologies, notamment pulmonaires, digestives ou cardiaques, liées à des troubles des flux ioniques transmembranaires, notamment de chlore et, le cas échéant, de bicarbonate, dans l'organisme (humain ou animal), notamment pour la préparation de médicaments destinés au traitement de la mucoviscidose, ou à la prévention du rejet de drogues cytotoxiques (notamment antitumorales), ou au traitement des obstructions des voies bronchiques ou des voies digestives (notamment pancréatique ou intestinale)

L'invention concerne plus particulièrement encore l'utilisation telle que décrite ci-dessus, de composés de formule générale (IIIa) suivante :

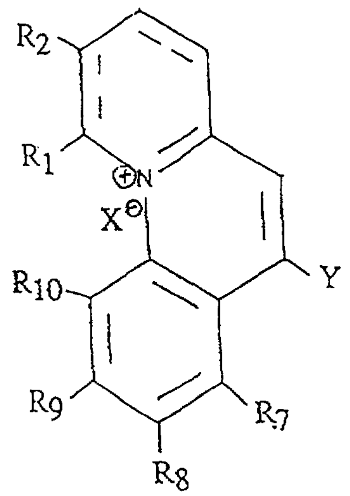

(IIIa)

dans laquelle :

- $R_1$ et $R_2$ représentent un atome d'hydrogène, ou forment en association avec $C_1$ et $C_2$ un cycle aromatique à 6 atomes de carbone,
- Y représente un groupe -OH ou $-NH_2$, ou $-NHCOCH_3$,
- $R_7$, $R_8$, $R_9$ et $R_{10}$ représentent un atome d'hydrogène, ou l'un de $R_7$, $R_8$, $R_9$ ou $R_{10}$, représente un atome d'halogène, notamment un atome de chlore. de brome ou de fluor,
- X représente un atome d'halogène sous forme anionique, notamment un atome de brome $Br^-$, ou de chlore $Cl^-$, ou un groupe d'atomes sous forme anionique, notamment un perchlorate $ClO_4^-$.

Des composés particulièrement préférés dans le cadre de la présente invention sont ceux de formule (IIIa) dans laquelle :

- $R_1$ et $R_2$ représentent un atome d'hydrogène,
- Y représente un groupe -OH,
- X représente un atome d'halogène sous forme anionique, notamment un atome de brome $Br^-$, ou de chlore $Cl^-$, ou un groupe d'atomes sous forme anionique, notamment un perchlorate $ClO_4^-$,
- $R_7$, $R_8$, $R_9$ et $R_{10}$ représentent indépendamment les uns des autres un atome d'hydrogène ou un atome d'halogène, notamment un atome de chlore, de brome ou de fluor.

Des composés de formule générale (IIIa) avantageusement utilisés dans le cadre de la présente invention, sont ceux choisis parmi les suivants :

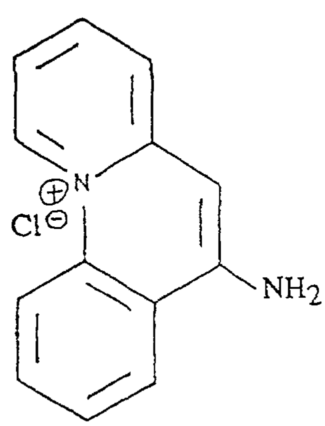

(composé 11 ou MPB-26)

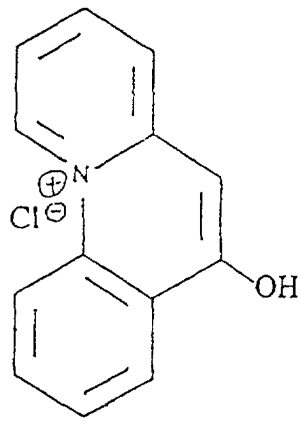

(composé 12 ou MPB-05)

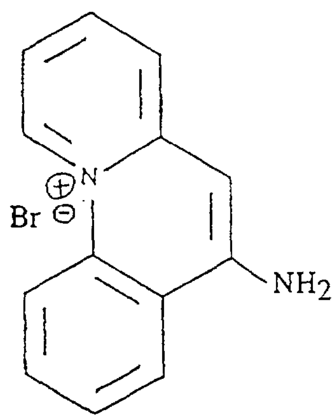

(composé 13 ou MPB-01)

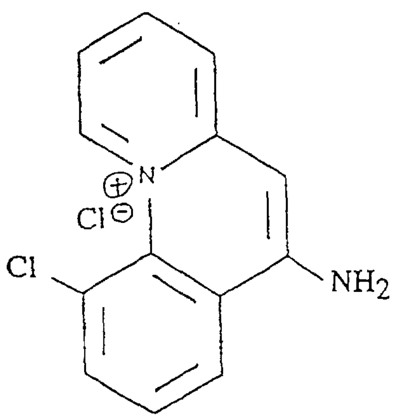

(composé 14 ou MPB-02)

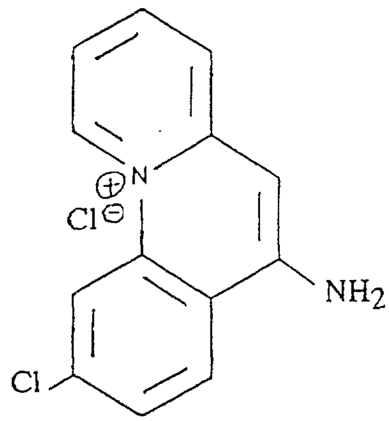

(composé 15 ou MPB-03)

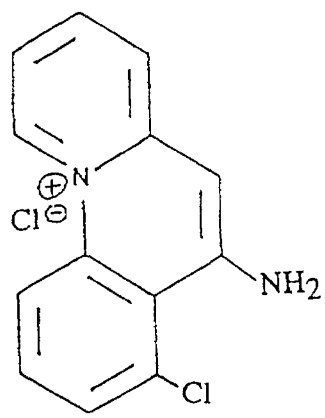

(composé 16)

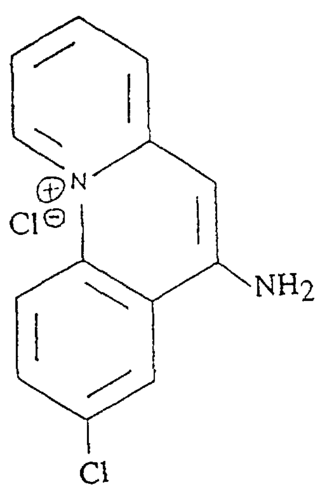

(composé 17)

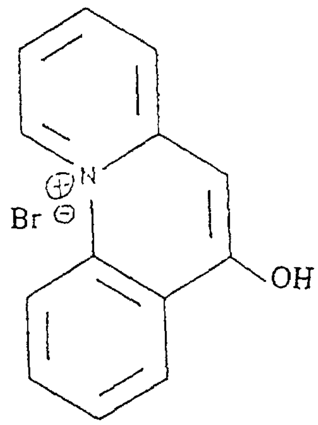

(composé 18 ou MPB-06)

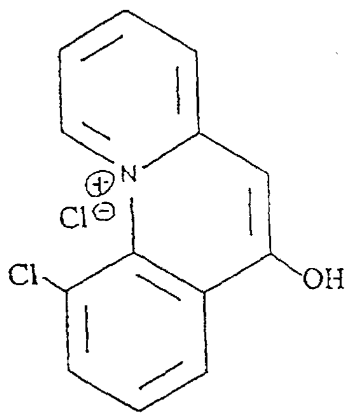

(composé 19 ou MPB-07)

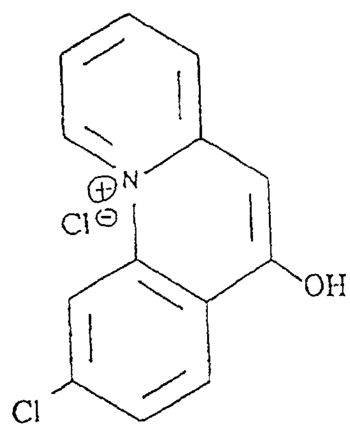

(composé 20 ou MPB-08)

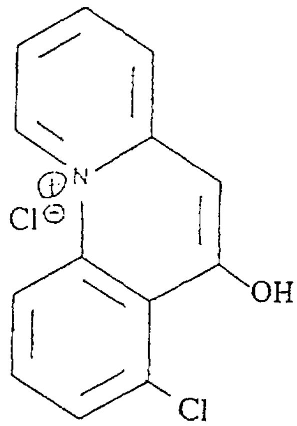

(composé 21 ou MPB-27)

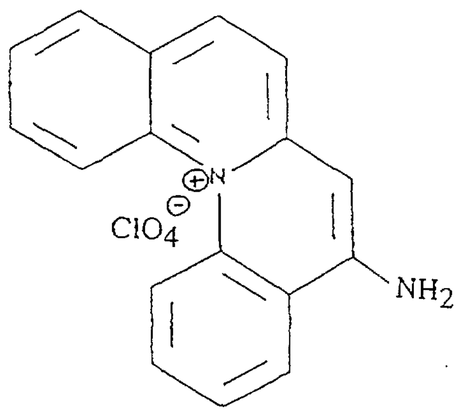

(composé 22)

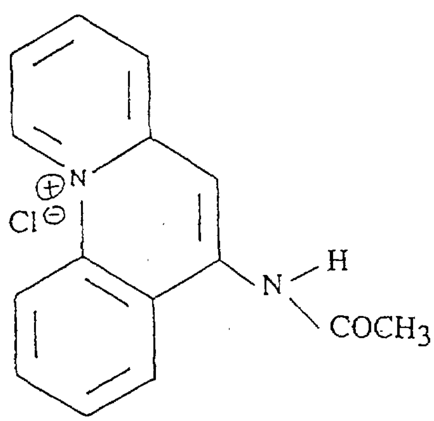

(composé 23)

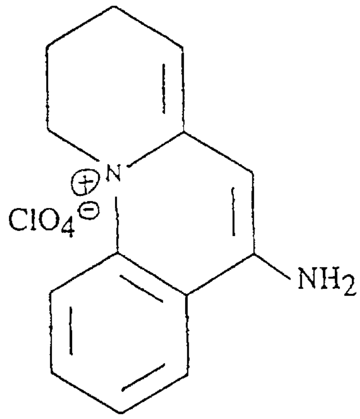

(composé 24)

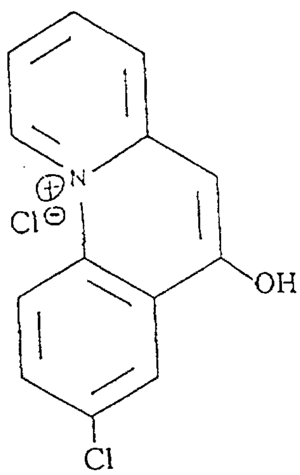

(composé 25 ou MPB-30)

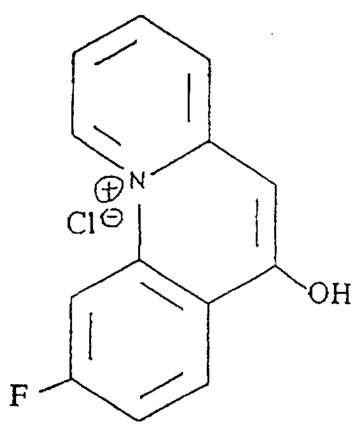

(composé 26 ou MPB-29)

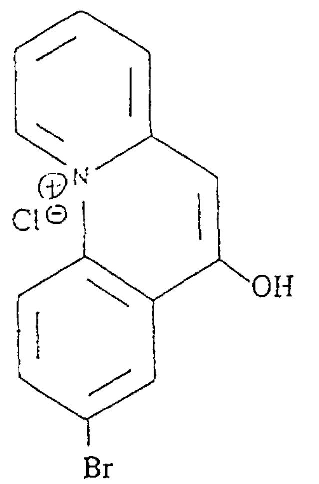

(composé 27 ou MPB-32)

L'invention concerne plus particulièrement encore l'utilisation, telle que décrite ci-dessus, de composés de formule générale (IIIb) suivante :

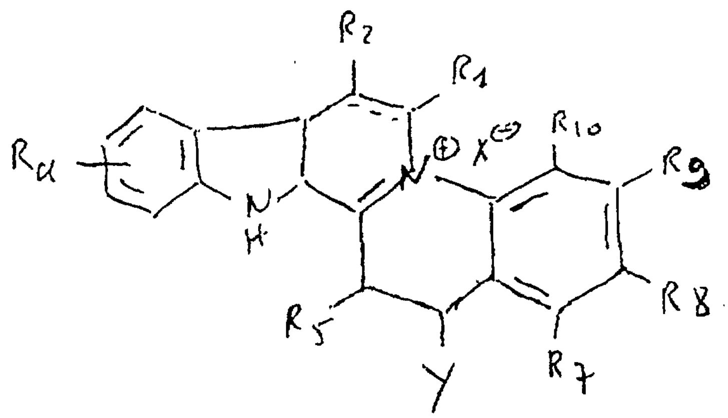

(IIIb)

dans laquelle $R_a$, $R_1$, $R_2$, $R_5$, $R_7$, $R_8$, $R_9$, $R_{10}$, X et Y sont tels que définis ci-dessus, et notamment les composés de formule (IIIb) dans laquelle :

- $R_a$ représente un atome d'hydrogène,
- $R_1$ et $R_2$ représentent un atome d'hydrogène, et il n'y a pas de double liaison entre les deux carbones portant $R_1$ et $R_2$,
- $R_5$ représente un atome d'hydrogène,
- $R_7$, $R_8$, $R_9$ et $R_{10}$ représentent un atome d'hydrogène, ou l'un de $R_7$, $R_8$, $R_9$ ou $R_{10}$ représente un atome d'halogène, notamment un atome de chlore, de brome ou de fluor,
- Y représente $-NH_2$,
- X représente un atome d'halogène sous forme anionique, notamment un atome de brome $Br^-$, ou de chlore $Cl^-$, ou un groupe d'atomes sous forme anionique, notamment un perchlorate $ClO_4^-$.

Des composés de formule (IIIb) avantageusement utilisés dans le cadre de la présente invention, sont ceux choisis parmi les suivants :

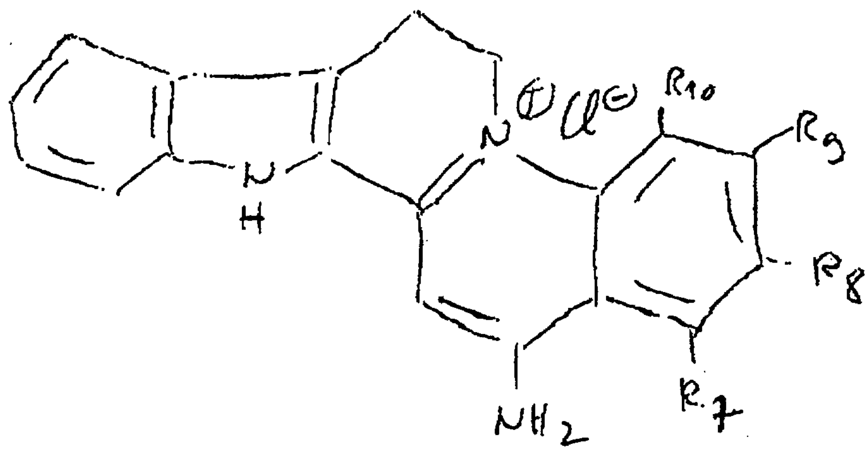

- composé G : $R_7$ = Cl, $R_8$ = $R_9$ = $R_{10}$ = H,
- composé H : $R_7$ = $R_8$ = $R_9$ = $R_{10}$ = H,
- composé 1 : $R_8$ = Cl, $R_7$ = $R_9$ = $R_{10}$ = H,
- composé J : $R_9$ = Cl, R7 = $R_8$ = $R_{10}$ = H,
- composé K : $R_{10}$ = Cl, $R_7$ = $R_8$ = $R_9$ = H,
- composé L : $R_9$ = Br, $R_7$ = $R_8$ = $R_{10}$ = H.

L'invention a également pour objet toute composition pharmaceutique comprenant, à titre de principe(s) actif(s), au moins un des composés de formule générale (III) décrite ci-dessus, en association avec un véhicule physiologiquement acceptable.

L'invention a plus particulièrement pour objet toute composition pharmaceutique telle que décrite ci-dessus, comprenant, à titre de principe(s) actif(s), au moins un des composés de formule (III), et plus particulièrement de formule (IIIa), tels que définis ci-dessus, et notamment au moins un des composés 11 à 27 décrits ci-dessus, et plus particulièrement encore de formule (IIIb), tels que définis ci-dessus, et notamment au moins un des composés G à L décrits ci-dessus.

Des compositions pharmaceutiques préférées de l'invention sont celles comprenant le composé 19 (encore désigné MPB-07), le cas échéant en association avec un (ou plusieurs) autre(s) composé(s) de l'invention décrit(s) ci-dessus.

Avantageusement les compositions pharmaceutiques selon l'invention se présentent sous une forme administrable par voie orale, notamment sous forme de comprimés, ou de gélules, ou sous une forme administrable par voie parentérale, notamment sous forme de préparations injectables par voie intraveineuse, intramusculaire, ou sous-cutanée, ou encore par voie aérienne, notamment par voie pulmonaire sous forme d'aérosols.

Avantageusement encore, les compositions pharmaceutiques selon l'invention, sont caractérisées en ce que les quantités de principe(s) actif(s) sont telles que la posologie journalière en principe(s) actif(s) est d'environ 0,1 mg/kg à 5 mg/kg, notamment d'environ 3 mg/kg, en une ou plusieurs prises.

L'invention concerne également les composés de formule générale (III) décrite ci-dessus, en tant que tels, à l'exclusion des composés de formules suivantes:

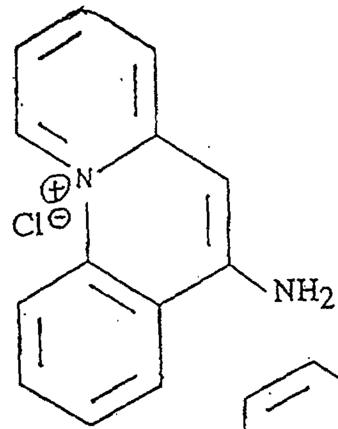

(composé 11 ou MPB-26)

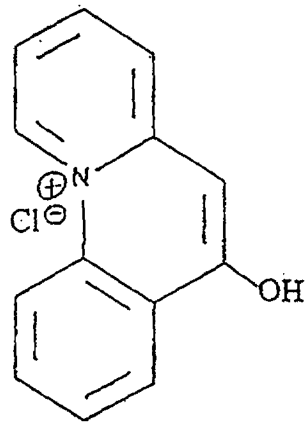

(composé 12 ou MPB-05)

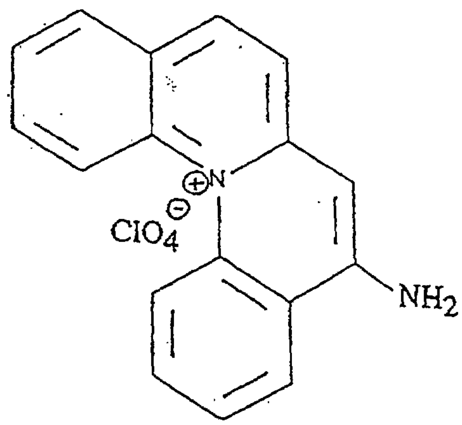

(composé 22)

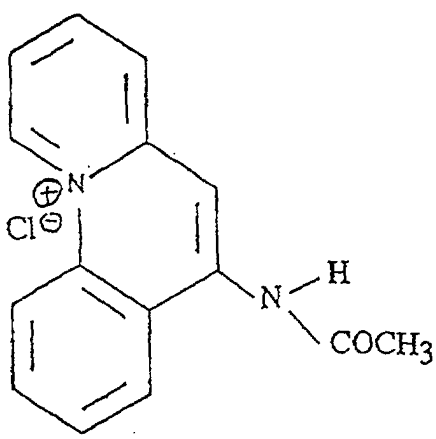

(composé 23)

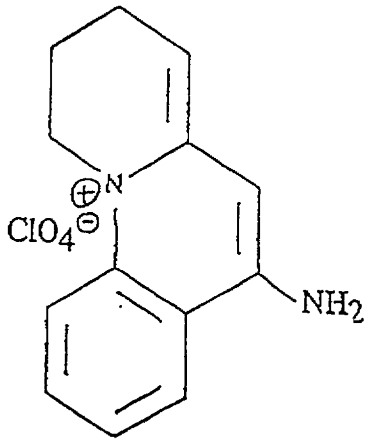

(composé 24)

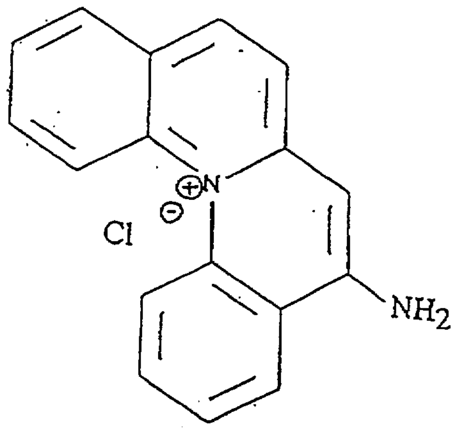

L'invention concerne plus particulièrement les composés de formule générale (IIIa) décrite ci-dessus, dont notamment les composés 13, 14, 15, 16, 17, 18, 19, 20, 21, 25, 26 et 27 décrits ci-dessus.

L'invention concerne plus particulièrement encore les composés de formule générale (IIIb) décrite ci-dessus, dont notamment les composés G à L décrits ci-dessus.

L'invention a également pour objet un procédé de préparation des composés de formule générale (III), caractérisé en ce qu'il comprend les étapes suivantes :

- traitement du dérivé de formule (A) dans laquelle $R_1$, $R_2$, $R_3$, $R_4$; et $R_5$, sont tels que définis dans la formule (III), par le phényllithium ou le diisopropyl amidure de lithium, avantageusement dans l'éther ou le THF, ce qui conduit à l'obtention de dérivés de formule (B) dans laquelle $R_1$, $R_2$, $R_3$, $R_4$, et $R_5$, sont tels que définis dans la formule (III), selon le schéma réactionnel suivant:

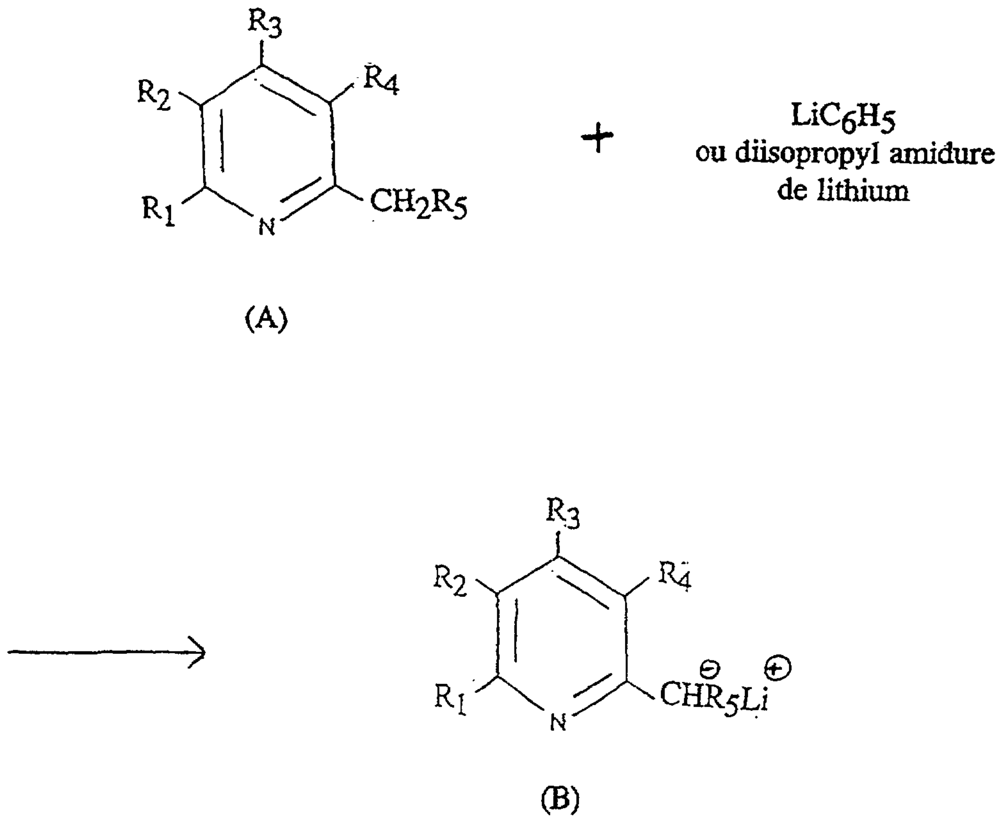


(A)

(B)

- condensation du dérivé de formule (B) obtenu à l'étape précédente avec le dérivé de formule (C) dans laquelle $R_7$, $R_8$, $R_9$, $R_{10}$, et X sont tels que définis dans la formule (III), ce qui conduit à l'obtention de dérivés de formule (B) dans laquelle $R_1$, $R_2$, $R_3$, $R_4$, $R_5$, $R_7$, $R_8$, $R_9$, $R_{10}$, et X sont tels que définis dans la formule (III), selon le schéma réactionnel suivant :

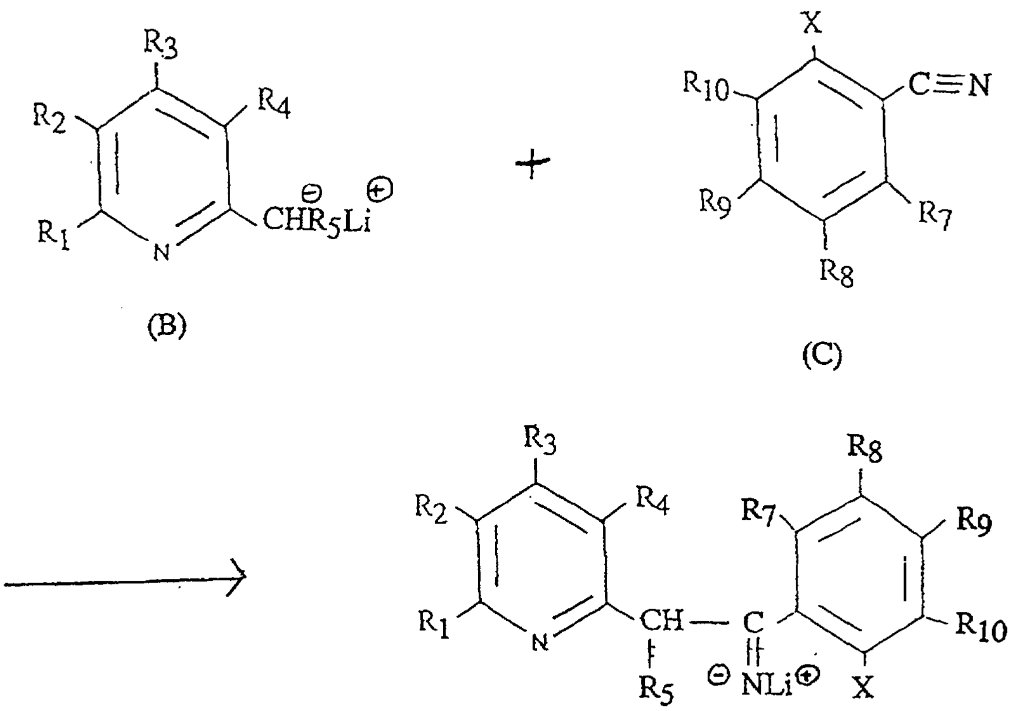

(B) (C)

(D)

- traitement du composé de formule (D) par addition d'$H_2O$, ce qui conduit à l'obtention du dérivé de formule (II) suivante, dans laquelle $R_1$ à $R_5$, $R_7$ à $R_{10}$, et X sont tels que définis dans la formule (III), et Y représente $-NH_2$,

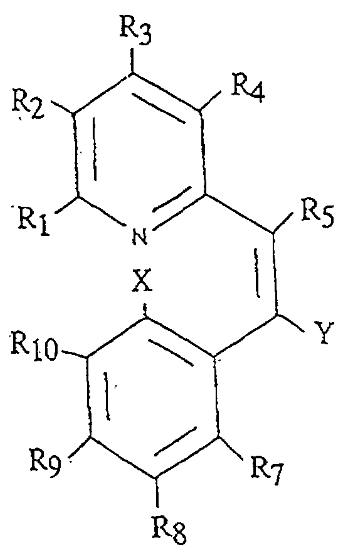

- le cas échéant, traitement du composé de formule (II) susmentionnée, par un dérivé comportant les groupes $R_e$ et $R_f$ tels que définis dans la formule (III) ce dérivé étant susceptible de réagir avec l'atome d'azote lié au carbone en position 6 du composé de formule (II) susmentionnée, notamment par un halogénure de $R_e$ et/ou de $R_f$ tout en ayant, si nécessaire, pris soin de protéger au préalable celles des autres fonctions présentes sur le composé de formule (II) susmentionnée et susceptibles de réagir avec le dérivé comportant les groupes $R_e$ et $R_f$ susmentionnés, ce qui conduit à l'obtention du composé de formule (II) suivante dans laquelle $R_1$ à $R_5$, $R_7$ à $R_{10}$, et X sont tels que définis ci-dessus, et Y représente un groupe $-NR_eR_f$ tel que défini dans la formule (III)

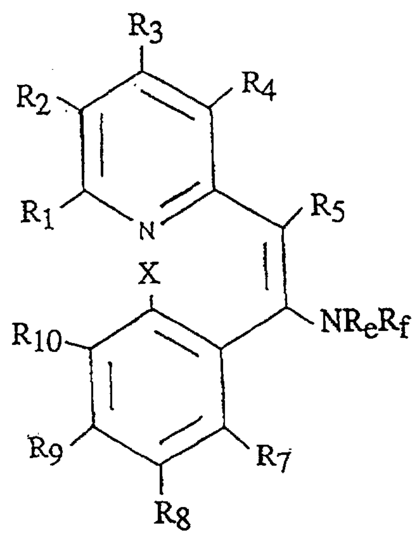

- le cas échéant, hydrolyse, notamment par action de l'acide sulfurique (pH3) à 40°C, du composé de formule (II) susmentionnée dans laquelle Y représente $NH_2$, ce qui conduit à l'obtention du composé de formule (II) suivante, correspondant à un dérivé de formule (II) décrite ci-dessus, dans laquelle $R_1$ à $R_5$, $R_7$ à $R_{10}$, et X sont tels que définis dans la formule (III) et Y représente un groupe -OH,

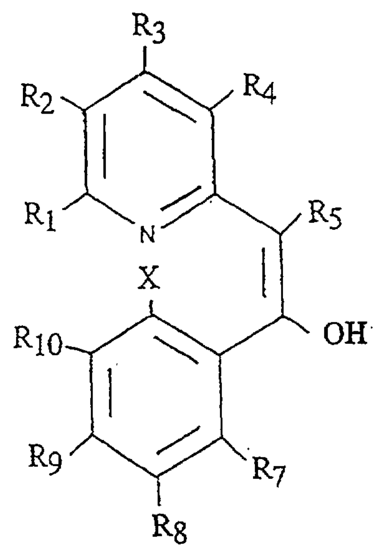

- le cas échéant, traitement du composé de formule (II) susmentionnée, dans laquelle Y représente un groupe -OH, par un dérivé comportant le groupe $R_d$, tel que défini dans la formule (III), ce dérivé étant susceptible de réagir avec l'atome d'oxygène lié au carbone en position 7 du composé de formule (II) susmentionnée, notamment par un halogénure de $R_d$, tout en ayant, si nécessaire, pris soin de protéger au préalable celles des autres fonctions présentes sur le composé de formule (II) susmentionnée et susceptibles de réagir avec le dérivé comportant le groupe $R_d$ susmentionné, ce qui conduit à l'obtention du composé de formule (II) suivante dans laquelle $R_1$ à $R_5$, $R_7$ à $R_{10}$, et X sont tels que définis ci-dessus, et Y représente un groupe $-OR_d$ tel que défini dans la formule (III),

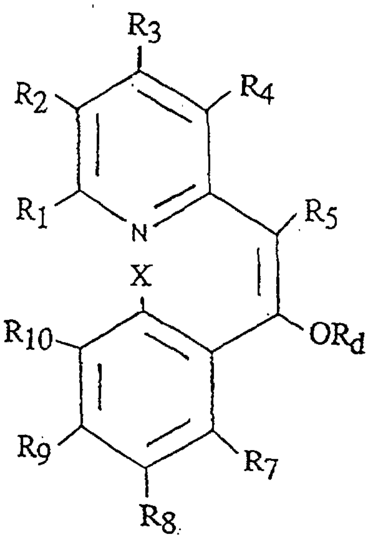

- le cas échéant, chauffage, avantageusement à 200°C, des composés de formule (II) susmentionnée dans laquelle Y représente $-NH_2$ ou -OH, ce qui conduit respectivement aux composés de formules (III) suivantes, correspondant aux composés de formule (III) décrite ci-dessus, dans laquelle $R_1$ à $R_5$, $R_7$ à $R_{10}$, et X sont tels que définis dans la formule (III), et Y représente un groupe $-NH_2$ ou -OH,

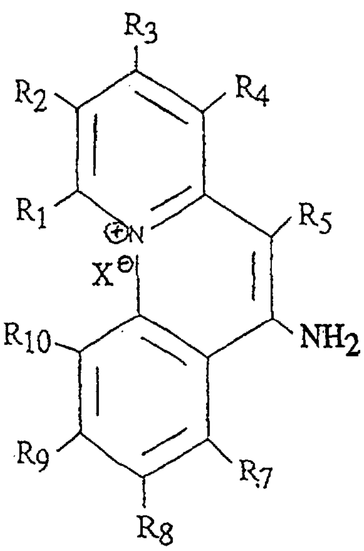

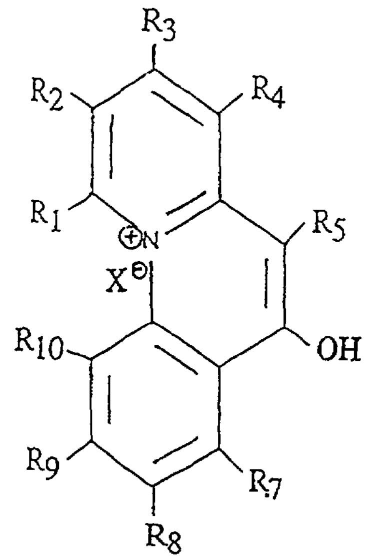

- le cas échéant, traitement du composé de formule (III) susmentionnée, dans laquelle Y représente un groupe -$NH_2$, par un dérivé comportant les groupes $R_e$ et $R_f$ tels que définis dans la formule (III), ce dérivé étant susceptible de réagir avec l'atome d'azote lié au carbone en position 6 du composé de formule (III) susmentionnée, notamment par un halogénure de $R_e$ et/ou de $R_f$, tout en ayant, si nécessaire, pris soin de protéger au préalable celles des autres fonctions présentes sur le composé de formule (III) susmentionnée et susceptibles de réagir avec le dérivé comportant les groupes $R_e$ et $R_f$ susmentionnés, ce qui conduit à l'obtention du composé de formule (III) suivante dans laquelle $R_1$ à $R_5$, $R_7$ à $R_{10}$, et X sont tels que définis ci-dessus, et Y représente un groupe -$NR_eR_f$ tel que défini dans la formule (III),

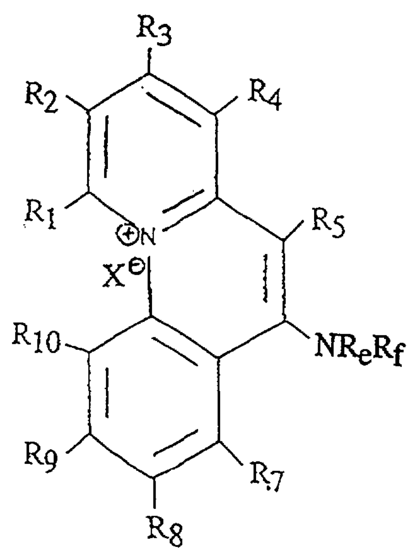

- le cas échéant, traitement du composé de formule (III) susmentionnée, dans laquelle Y représente un groupe -OH, par un dérivé comportant le groupe $R_d$, tel que défini dans la formule (III), ce dérivé étant susceptible de réagir avec l'atome d'oxygène lié au carbone en position 7 du composé de formule (III) susmentionnée, notamment par un halogénure de $R_d$, tout en ayant, si nécessaire, pris soin de protéger au préalable celles des autres fonctions présentes sur le composé de formule (III) susmentionnée et susceptibles de réagir avec le dérivé comportant le groupe $R_d$ susmentionné, ce qui conduit à l'obtention du composé de formule (III) suivante dans laquelle $R_1$ à $R_5$, $R_7$ à $R_{10}$, et X sont tels que définis ci-dessus, et Y représente un groupe -$OR_d$ tel que défini dans la formule (III),

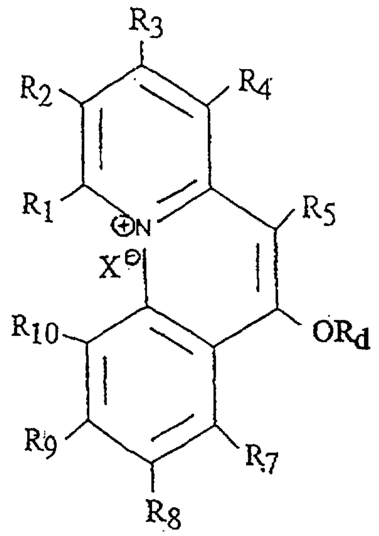

- le cas échéant, hydrogénation des composés de formules (II) ou (III) susmentionnées, notamment par hydrogénation catalytique en présence d'oxyde de platine à pression réduite, ce qui conduit à l'obtention des composés de formules (II) ou (III) suivantes :

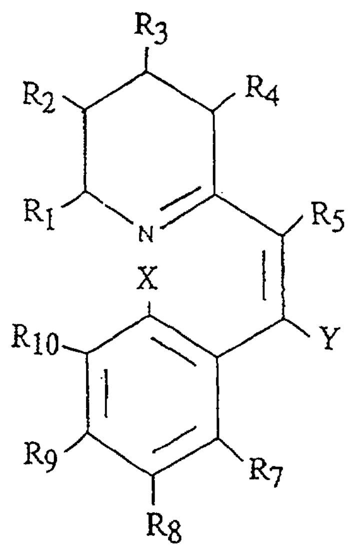

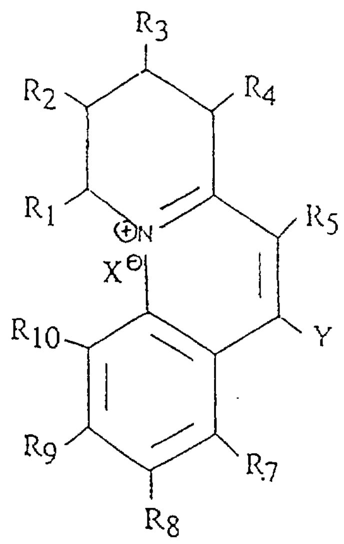

dans lesquelles $R_1$ à $R_5$, $R_7$ à $R_{10}$, X et Y sont tels que définis ci-dessus.

Les composés A à L décrits ci-dessus sont avantageusement obtenus par traitement de l'harmalane par du butyl lithium (Buli, 2 éq) à -40°C, puis addition de 2-chlorobenzonitrile (le cas échéant substitué par un ou plusieurs des groupes $R_7$, $R_8$, $R_9$ et $R_{10}$ tels que définis ci-dessus), ce qui conduit à l'obtention des composés de formule A à F qui, par chauffage à 195°C sous azote, conduisent respectivement aux composés G à L.

L'invention sera davantage illustrée à l'aide de la description détaillée qui suit des procédés de préparation des composés 1 à 24 décrits ci-dessus, ainsi que de l'étude des effets de certains de ces composés sur le CFTR.

**I- Procédés de préparation des composés 1 à 24 :**

a) 1-hydroxy,1-phényl,2-(2-pyridyl)éthylène (composé 1)

Le composé 1 est préparé selon un mode opératoire identique à celui décrit ci-après dans le cadre de la préparation du composé 2, mais avec utilisation de benzonitrile au lieu du 2-chlorobenzonitrile.

b) 1-hydroxy,1-(2-chlorophényl),2-(2-pyridyl)éthylène (composé 2)

Dans un réacteur de 500 ml, muni d'un réfrigérant à reflux avec une garde à chlorure de calcium, et d'une arrivée d'azote, on place 2,22 g (0,022 mole) de diisopropylamine dans 30 ml de THF anhydre. On amène la solution à 0°C puis on ajoute 13,75 ml de BuLi en solution à 1,6 M dans l'hexane (0,022 mole). On agite 30 minutes à 0°C puis on abaisse la température à -40°C, puis on additionne 1,86 g (0,02 mole) de 2-méthylpyridrine. On agite 30 minutes à -40°C, puis on ajoute 2,75 g de 2-chlorobenzonitrile dans 20 ml de THF anhydre. On laisse le milieu réactionnel revenir à la température ambiante et on ajoute 20 ml d'eau, puis on ajuste le pH vers 2 par addition d'$H_2SO_4$ 2N. On chauffe à reflux et sous agitation pendant 1 heure. On extrait par le chloroforme, on sèche sur sulfate de sodium. Le solvant est évaporé et le résidu est dissout dans le minimum d'éther anhydre, puis on ajoute goutte à goutte de l'éthanol saturé d'HCl jusqu'à complète précipitation du chlorhydrate. On obtient ainsi 2,99 g du composé 2 soit un rendement de 56%

- Point de fusion (Pf) = 190 °C
- Analyse élémentaire : $C_{13}$ $H_{11}$ Cl $N_2$ O

| | | | |
|---|---|---|---|
| Calculé % | C : 58,20 | H : 4,10 | N : 5,20 |
| Trouvé % | C : 58,00 | H : 4,10 | N : 5,30 |

c) 1-amino,1-(2-chlorophényl),2-(2-pyridyl)éthylène (composé 3)

Le composé 3 est préparé selon un mode opératoire identique à celui décrit ci-dessus dans le cadre de la préparation du composé 2, mais sans l'étape d'hydrolyse par addition d'$H_2SO_4$.

d) 1-hydroxy,1-(2-bromophényl) 2-(2-pyridyl)éthylène (composé 4)

Le composé 4 est préparé selon un mode opératoire identique à celui décrit ci-dessus dans le cadre de la préparation du composé 2, mais avec utilisation de 2-bromobenzonitrile au lieu du 2-chlorobenzonitrile.

e) 1-hydroxy 1-(2,3-dichlorophényl),2-(2-pyridyl)éthylène (composé 5)

Dans un réacteur de 500 ml, muni d'un réfrigérant à reflux avec une garde à chlorure de calcium, et d'une arrivée d'azote, on place 2,22 g (0,022 mole) de diisopropylamine dans 30 ml de THF anhydre. On amène la solution à 0°C puis on ajoute 13,75 ml de BuLi en solution à 1,6 M dans l'hexane (0,022 mole). On agite 30 minutes à 0°C, puis on abaisse la température à -40°C, puis on additionne 1,86 g (0,02 mole) de 2-méthylpyridrine. On agite 30 minutes à -40°C, puis on ajoute 2,58 g (0,015 mole) de 2,3-dichlorobenzonitrile dans 20 ml de THF anhydre. On laisse le milieu réactionnel revenir à la température ambiante et on ajoute 20 ml d'eau puis on ajuste le pH vers 2 par addition d'$H_2SO_4$ 2N. On chauffe à reflux et sous agitation pendant 2 heures. On sépare la phase organique, on sèche sur sulfate de sodium. Le solvant est évaporé et le résidu est chromatographié sur colonne de silice en éluant au chloroforme pour obtenir 3,19 g (80%) de cétone pure.

- Point de fusion (Pf) = 112°C
- Analyse élémentaire : $C_{13} H_9 N O Cl_2$

| | | | |
|---|---|---|---|
| Calculé % | C : 58,67 | H : 3,41 | N : 5,26 |
| Trouvé % | C : 58,53 | H : 3,63 | N : 5,34 |

- Spectre de $^1H$ RMN ($CDCl_3$) (δ ppm, signal, N protons, attribution) : 8, doublet, H en 6 pyridine ; 7,6,5, multiplet, 6, H aromatiques. 5,55, s, 1 ( 80%) H vinylique ; 4,25, s s2 (20 %) $CH_2$.

La base ainsi obtenue peut-être transformée en chlorhydrate : on dissout la base dans l'éther anhydre et on ajoute goutte à goutte de l'éthanol anhydre saturé d'HCl.

- Analyse élémentaire : $C_{13} H_{10} N O Cl_3$ :

| | | | |
|---|---|---|---|
| Calculé % | C : 51,60 | H : 3,33 | N : 4,63 |
| Trouvé % | C : 51,75 | H : 3,52 | N : 4,58 |

f) 1-hydroxy,1-(2,4-dichlorophényl),2-(2-pyridyl)éthylène (composé 6)

Le composé 6 est préparé selon un mode opératoire identique à celui décrit ci-dessus dans le cadre de la préparation du composé 2, mais avec utilisation de 2-4-dichiorobenzonitrile au lieu du 2-chlorobenzonitrile.

g) 1-hydroxy,1-(2,5-dichlorophényl),2-(2-pyridyl)éthylène (composé 7)

Le composé 7 est préparé selon un mode opératoire identique à celui décrit ci-dessus dans le cadre de la préparation du composé 2, mais avec utilisation de 2-5-dichlorobenzonitrile au lieu du 2-chlorobenzonitrile.

h) 1-hydroxy,1-(2,6-dichlorophényl),2-(2-pyridyl)éthylène (composé 8)

Le composé 8 est préparé selon un mode opératoire identique à celui décrit ci-dessus dans le cadre de la préparation du composé 2, mais avec utilisation de 2-6-dichlorobenzonitrile au lieu du 2-chlorobenzonitrile.

i) 1-hydroxy,1-(2-chlorophényl),2-(2-quinonyl) éthylène (composé 9)

Le composé 9 est préparé selon un mode opératoire identique à celui décrit ci-dessus dans le cadre de la préparation du composé 2, mais avec utilisation du 2-méthylquinoléine au lieu du 2-méthylpyridine.

j) 1-amino,1-(2-chlorophényl),2-(2-quinolyl)éthylène (composé 10)

Le composé 10 est préparé selon un mode opératoire identique à celui décrit ci-dessus dans le cadre de la préparation du composé 9, mais sans l'étape d'hydrolyse par addition d'$H_2SO_4$.

k) Chlorure de 6-aminobenzo[c]quinolizium MPB-26 (composé 11)

Dans un réacteur de 500 ml, muni d'un réfrigérant à reflux avec une garde à chlorure de calcium, et d'une arrivée d'azote, on place 2,22 g (0,022 mole) de diisopropylamine dans 30 ml de THF anhydre. On amène la solution à 0°C, puis on ajoute 13,75 ml de BuLi en solution à 1,6 M dans l'hexane (0,022 mole). On agite 30 minutes à 0°C, puis on abaisse la température à -40°C, puis on additionne 1,86 g (0,02 mole) de 2-méthylpyridrine. On agite 30 minutes à -40°C, puis on ajoute 2,75 g de 2-chlorobenzonitrile (0,02 mole) dans 20 ml de THF anhydre. On laisse le milieu réactionnel revenir à la température ambiante et on ajoute une solution à 10% de chlorure d'ammonium. La phase organique est séparée, on sèche sur $SO_4Na_2$, on évapore et le résidu est porté à 200°C sous un courant d'azote pendant 15 minutes. On observe un dégagement de vapeurs blanches et la prise en une masse brunâtre du résidu. Le produit est purifié par un premier lavage à l'acétone puis dissolution dans l'éthanol et précipité par l'acétate d'éthyle. On peut aussi purifier par chromatographie sur colonne d'alumine neutre et élution par l'acétate d'éthyle On obtient ainsi 1,73 g (35%) d'un produit cristallisant avec une molécule d'eau.

- Point de fusion (Pf) = décomposition vers 280°C
- Analyse élémentaire : $C_{13}$ $H_{13}$ $C_1$ $N_2$ O ($C_{13}$ $H_{11}$ $C_1$ $N_2$, $H_2O$)

| | | | |
|---|---|---|---|
| Calculé % | C : 62,78 | H : 5,27 | N: 11,26 |
| Trouvé % | C : 62,86 | H : 5,03 | N : 11,25 |

- Spectre de masse : m/e 194 ($M^+$ - HCl - $H_2O$).
- Spectre infrarouge (KBr) ($\nu$ $cm^{-1}$, attribution) : 3460, 3360, $NH_2$; 1660, 1640, 1600, C=N, C=C ; 760 benzène orthosubstitué.
- Spectre de $^1$H RMN (DMSOd6) ($\delta$ ppm, signal, n protons, attribution): 3,2 à 4, pic échangeable $D_2O$ ($NH_2$ + $H_2O$); 7,1 singulet, H en 5; 7,4 à 8,15, 2 massifs, 5H aromatiques; 9,00, 2H, aromatiques ; 9,8, doublet J=8 Hz, H en 1.

l) Chlorure de 6-hydroxybenzo[c]quinolizinium MPB-05 (composé 12)

Le 1-hydroxy,1-(2-chlorophényl),2-(2-pyridyl)éthylène (composé 2) est neutralisé par une solution aqueuse de carbonate de sodium, la base est extraite par l'éther, la solution est séchée sur $SO_4Na_2$ puis le solvant évaporé. 1,16 g (0,005 mole) de base sous forme d'huile jaune pâle est chauffée sous azote à 195°C pendant 15 minutes. Le résidu ainsi obtenu, est lavé à l'acétone, puis dissout dans l'éthanol et précipité par addition d'acétate d'éthyle. On obtient 0,75 g (60%) d'un produit cristallisé avec une molécule d'eau de couleur blanc crème.

- Point de fusion (Pf) = 256°C (décomposition)
- Analyse élémentaire : $C_{13}$ $H_{10}$ Cl N O, $H_2O$ soit $C_{13}$ $H_{12}$ Cl N O (M=231,5 + 18=249,5)

| | | | |
|---|---|---|---|
| Calculé % | C : 62,53 | H : 4,85 | N : 5,61 |
| Trouvé % | C : 62,40 | H : 5,00 | N : 5,80 |

- Spectre de masse : m/e 195 ($M^+$ - HCl - $H_2O$), 167 ($M^+$ - HCl - $H_2O$ - CO).
- Spectre infrarouge (KBr) ($\nu$ $cm^{-1}$, attribution) : 3250, OH; 1640, C=N, 770, benzène orthosubstitué.
- Spectre de $^1$H RMN (DMSOd6) ($\delta$ ppm, signal, n protons, attribution): 6,60, pic large échangeable $D_2O$, HO; 7,75, singulet, H en 5 ; 7,9 à 8,6, multiplet, 6H, aromatiques; 9,15, doublet, J = 6,5 Hz, 1H ; 10, doublet, J = 6Hz, H en 1.

m) Bromure de 6-aminobenzo[c]quinolizinium MPB-01 (composé 13) :

Le composé 13 est préparé selon un mode opératoire identique à celui décrit ci-dessus dans le cadre de la préparation du composé 11, mais avec utilisation du 2-bromobenzonitrile au lieu du 2-chlorobenzonitrile.

n) Chlorure de 6-amino,10-chlorobenzo[c]quinolizinium MPB-02 (composé 14) :

Le composé 14 est préparé selon un mode opératoire identique à celui décrit ci-dessus dans le cadre de la préparation du composé 11, mais avec utilisation du 2,3-dichlorobenzonitrile au lieu du 2-chlorobenzonitrile.

o) Chlorure de 6-amino,9-chlorobenzo[c]quinolizinium (composé 15) :

Le composé 15 est préparé selon un mode opératoire identique à celui décrit ci-dessus dans le cadre de la préparation du composé 11, mais avec utilisation du 2,4-dichlorobenzonitrile au lieu du 2-chlorobenzonitrile.

p) Chlorure de 6-amino,7-chlorobenzo[c]quinolizinium (composé 16) :

Le composé 16 est préparé selon un mode opératoire identique à celui décrit ci-dessus dans le cadre de la préparation du composé 11, mais avec utilisation du 2,6-dichlorobenzonitrile au lieu du 2-chlorobenzonitrile.

q) Chlorure de 6-amino,8-chlorobenzo[c]quinolizinium (composé 17) :

Le composé 17 est préparé selon un mode opératoire identique à celui décrit ci-dessus dans le cadre de la préparation du composé 11, mais avec utilisation du 2,5-dichlorobenzonitrile au lieu du 2-chlorobenzonitrile.

r) Bromure de 6-hydroxybenzo[c]quinolizinium MPB-06 (composé 18) :

Le composé 18 est préparé selon un mode opératoire identique à celui décrit ci-après dans le cadre de la préparation du composé 19, mais effectué à partir du composé 4 au lieu du composé 5.

s) Chlorure de 6-hydroxy,10-chlorobenzo[c]quinolizinium MPB-07 (composé 19) :

Le 1-hydroxy,1-(2-bromophényl),2-(2-pyridyl)éthylène (composé 5) 1,40 g (0,0053 mole) est chauffée sous azote à 215°C. Vers 190°C, on note l'apparition de fumées blanches de HCl et on poursuit le chauffage pendant 10 minutes à 220°C. Le produit est lavé au chloroforme puis le résidu (1,82 g) est purifié par chromatographie sur colonne de silice en éluant par l'acétate et l'alcool. On obtient ainsi 0,58 g (42%) de produit.

- Pf = 196 °C (décomposition)
- Analyse élémentaire : $C_{13} H_{10} N O Cl_2$

| | | | |
|---|---|---|---|
| Calculé % | C : 56,75 | H : 3,66 | N : 4,63 |
| Trouvé % | C : 56,25 | H : 3,31 | N : 4,78 |

t) Chlorure de 6-hydroxy,9-chlorobenzo[c]quinolizinium MPB-08 (composé 20) :

Le composé 20 est préparé selon un mode opératoire identique à celui décrit ci-dessus dans le cadre de la préparation du composé 19, mais effectué à partir du composé 6 au lieu du composé 5.

u) Chlorure de 6-hydroxy,7-chlorobenzo[c]quinolizium (composé 21) :

Le composé 21 est préparé selon un mode opératoire identique à celui décrit ci-dessus dans le cadre de la préparation du composé 19, mais effectué à partir du composé 8 au lieu du composé 5.

v) Perchlorate de 8-aminodibenzo[c,f]quinolizinium (composé 22) :

Dans un réacteur de 500 ml, muni d'un réfrigérant à reflux avec une garde à chlorure de calcium, et d'une arrivée d'azote, on place 2,22 g (0,022 mole) de diisopropylamine dans 30 ml de THF anhydre. On amène la solution à 0°C, puis on ajoute 13,75 ml de BuLi en solution à 1,6 M dans l'hexane (0,022 mole). On agite 30 minutes à 0°C, puis on abaisse la température à -40°C, puis on additionne 2,86 g (0,02 mole) de quinaldine. On agite 30 minutes à -40°C, puis on ajoute 2,75 g de 2-chlorobenzonitrile (0,02 mole) dans 20 ml de THF anhydre. On laisse le milieu réactionnel revenir à la température ambiante et on ajoute une solution à 10% de chlorure d'ammonium. La phase organique est séparée, on sèche sur $SO_4Na_2$, on évapore et le résidu est porté à 230°C sous un courant d'azote

pendant 30 minutes. On observe un dégagement de vapeurs blanches d'acide chlorhydrique et la prise en une masse brunâtre du résidu. Le produit est purifié par un premier lavage à l'acétone puis dissolution dans l'éthanol et précipité par l'acétate d'éthyle. Le produit est dissout dans un minimum d'eau et on ajoute une solution d'acide perchlorique jusqu'à fin de précipitation. Le composé est filtré pour recueillir 2,20 g (32%).

- Analyse élémentaire : $C_{17}$ $H_{13}$ Cl $N_2$ $O_4$

| | | | |
|---|---|---|---|
| Calculé % | C : 59,22 | H : 3,80 | N : 8,13 |
| Trouvé % | C : 59,39 | H : 3,95 | N : 8,26 |

- Spectre infrarouge (Kbr) (ν $cm^{-1}$, attribution) : 3400, 3280, $NH_2$; 1650, 1600, 1000, bande large, $ClO_4$.
- Spectre de $^1H$ RMN (DMSOd6) (δ ppm, signal, n protons, attribution) : 7,0, singulet, 1H en 5 ; 7,5 à 8,7, multiplet, 10H aromatiques ; 9,0 singulet, 2H, échangeables $D_2O$.

w) Chlorure de 6-acétamidobenzo[c]quinolizinium (composé 23) :

On dissout 1 g (0,004 mole) de chlorhydrate de 6-aminobenzo[c]quinolizinium dans 10 ml d'acide acétique, puis on ajoute 25 ml d'anhydride acétique. On porte à reflux pendant 24 heures, puis on évapore l'anhydride et l'acide acétique sous pression réduite.

Le produit obtenu de couleur violette est lavé à l'acétate d'éthyle, puis recristallisé dans l'éthanol. On obtient 0,93 g (85%) d'un produit blanc crème.

- Pf = > à 280°C
- Analyse élémentaire : $C_{15}$ $H_{13}$ Cl $N_2$ O

| | | | |
|---|---|---|---|
| Calculé % | C : 66,05 | H : 4,80 | N : 10,27 |
| Trouvé % | C : 65,85 | H : 5,01 | N : 10,31 |

- Spectre infrarouge (KBr) (ν $cm^{-1}$, attribution) : 3450, NH; 1700, C = O.

x) Perchlorate de 1,2,3,4-tétrahydro,6-aminobenzo[c,f]quinolizinium (composé 24) :

Le chlorhydrate de 6-aminobenzo[c]quinolizinium 0,50 g (0,002 mole) est dissous dans 20 ml d'éthanol, et est mis en présence d'oxyde de platine et d'hydrogène à pression atmosphérique. L'hydrogénation est réalisée en quelques minutes puis la solution est filtrée, l'alcool évaporé et le résidu repris par 10 ml d'eau distillée et on ajoute une solution d'acide perchlorique à 25% tant que le précipité se forme. Le produit blanc est recristallisé dans le méthanol pour donner 0,54 g (91%) de perchlorate.

- Pf = 240°C
- Analyse élémentaire : $C_{13}$ $H_{15}$ Cl $N_2$ $O_4$

| | | | |
|---|---|---|---|
| Calculé % | C : 52,27 | H : 5,06 | N : 9,38 |
| Trouvé % | C : 52,30 | H : 5,16 | N : 9,46 |

- Spectre infrarouge (KBr) (ν $cm^{-1}$, attribution) : 3460,3360,$NH_2$; 1650, 1600, 1100, bande large, $CL0_4$.
- Spectre de $^1H$ RMN (DMSOd6) (δ ppm, signal, n protons, attribution) : 2,1, multiplet, $CH_2$ en 2 et 3; 3,20, triplet, $CH_2$ en 4; 4,45, triplet, $CH_2$ en 1,; 6,60, singulet, H en 5; 7,6 à 8,4, multiplet, 4H, aromatiques; 8,6 singulet, 2H, échangeables $D_2O$.

**II- Procédé de préparation des composés A à F :**

a) 1-amino 1-(2,6-dichlorophényl) 2-[1-(3,4-dihydropyrido[3-4-b]indolyl)] éthylène (composé A)

Dans un réacteur muni d'une arrivée d'azote, 1,84 g (0,01 mole) d'harmalane sont mis en solution dans 40 ml de THF et sont amenés à -40°C. On ajoute goutte à goutte 13,75 ml (0,022 mole) de BuLi, une coloration rouge foncée apparaît, la solution est laissée sous agitation pendant 30 min. Le 2,6-dichlorobenzonitrile 1,71 g (0,01 mole)

est mis en solution dans 15 ml de THF puis ajouté goutte à goutte. Après 1 h à -40°C, le mélange est agité 4 h à température du laboratoire. L'évolution de la réaction est suivie sur CCM, la disparition des produits de départ est corrélée à l'apparition d'une tache fluorescente jaune caractéristique de l'imine. L'hydrolyse par 5 ml d'une solution de $NH_4Cl$ à 10 % permet de recueillir la phase THF contenant l'imine. Cette phase est séchée sur $NA_2SO_4$, filtrée puis évaporée à sec. Le produit est purifié par chromatographie colonne sur gel de silice, élution par un mélange $CH_2Cl_2$/ $CH_3COOC_2H_5$ 5 %. On obtient ainsi 2,06 g du composé A, soit un rendement de 58 %.

- Pf = 228°C
- Analyse élémentaire : $C_{19}$ $H_{15}$ $N_3$ $Cl_2$

| | | | |
|---|---|---|---|
| Calculé % | C : 64,06 | H : 4,24 | N : 11,79 |
| Trouvé % | C : 64,21 | H : 4,37 | N : 11,62 |

- Spectre infrarouge (KBr) (ν $cm^{-1}$, attribution) :

  3428, 3255, 3134 $cm^{-1}$, NH; $NH_2$
  3134 $cm^{-1}$, C=C-H
  2932, 2843 $cm^{-1}$, $CH_2$

- Spectre $^1$ H RMN ($CDCl_3$) ( δ ppm, signal, n protons, attibution) :

  8.40 singulet échangeable par $D_2O$, 3 H, NH et $NH_2$
  7.20, multiplet, 7 H, protons aromatiques
  5.00, singulet, 1 H, H vinylique
  3.70, triplet, J=6Hz, 2 H, $CH_2$
  3.00, triplet, J=6Hz, 2 H, $CH_2$

b) 1-amino 1-(o-chlorophényl) 2-[1-(3,4-dihydropyrido [3-4-b]indolyl)] éthylène (composé B)

Le composé B est préparé selon un mode opératoire identique à celui décrit ci-dessus dans le cadre de la préparation du composé A mais avec l'utilisation de l'orthochlorobenzonitrile au lieu du 2,6 dichlorobenzonitrile.

c) 1-amino 1-(2,5-dichlorophényl) 2-[1-(3,4-dihydropyrido [3-4-b]indolyl)] éthylène (composé C)

Le composé C est préparé selon un mode opératoire identique à celui décrit ci-dessus dans le cadre de la préparation du composé A mais avec l'utilisation du 2,5 dichlorobenzonitrile au lieu du 2,6 dichlorobenzonitrile.

d) 1-amino 1-(2,4-dichiorophényl) 2-[1-(3,4-dihydropyrido [3-4-b]indolyl)] éthylène (composé D)

Le composé D est préparé selon un mode opératoire identique à celui décrit ci-dessus dans le cadre de la préparation du composé A mais avec l'utilisation du 2,4 dichlorobenzonitrile au lieu du 2,6 dichlorobenzonitrile.

e) 1-amino 1-(2,3-dichlorophényl) 2-[1-(3,4-dihydropyrido [3-4-b]indolyl)) éthylène (composé E)

Le composé E est préparé selon un mode opératoire identique à celui décrit ci-dessus dans le cadre de la préparation du composé A mais avec l'utilisation du 2,3 dichlorobenzonitrile au lieu du 2,6 dichlorobenzonitrile.

f) 1-amino 1-(4-bromo,2-chlorophényl) 2-[1-(3,4-dihydropyrido [3-4-b]indolyl)] éthylène (composé F)

Le composé F est préparé selon un mode opératoire identique à celui décrit ci-dessus dans le cadre de la préparation du composé A mais avec l'utilisation du 2-chloro-4 bromobenzonitrile au lieu du 2,6 dichlorobenzonitrile.

**III- Procédé de fabrication des composés G à L :**

Cyclisation des composés A à F en quinolizinium G à L.

a) Chlorure de 14-amino 6,7-dihydro 12H-1-chloro benzo-[n] indolo[2,3-a] quinolizinium (composé G).

L'énamine A purifiée est chauffée sous azote ; vers 150°C le produit se liquéfie, puis à 195°C des fumées blanches apparaissent et le produit se prend en masse. Le chauffage est maintenu à cette température pendant 10 min. On observe en CCM la disparition de la tache fluorescente jaune de l'imine d'un Rf de 0,3 sur silice dans $CH_2Cl_2$ et l'apparition d'une tache fluorescente jaune-vert du produit cyclisé d'un Rf de 0,1 sur alumine dans l'alcool. Le produit est purifié par lavage à l'acétone, puis recristallisation dans l'alcool ou par chromatographie : alumine - alcool.
Le produit ainsi obtenu est de couleur marron clair, avec un rendement de 17 %.

- Pf = 228°C.
- Analyse élémentaire : $C_{19}H_{15}N_3Cl_2$, 1/2 $H_2O$

| | | | |
|---|---|---|---|
| Calculé % | C : 62,48 | H : 4,41 | N : 11,50 |
| Trouvé % | C : 62,29 | H : 4,47 | N : 11,43 |

- Spectre $^1H$ RMN ($CF_3COOD$) ( δ ppm, signal, n protons, attibution) :

  8.10 - 6.20, massif, 11 H, protons aromatiques + NH + $NH_2$
  3.90, triplet mal résolu, 2 H, $CH_2$ en 6
  3.00, triplet mal résolu, 2 H, $CH_2$ en 7

- Spectres infrarouge (KBr) (ν $cm^{-1}$, attribution) présentent tous les mêmes absorptions :

  3458, 3371 $cm^{-1}$, NH; $NH_2$
  3073 $cm^{-1}$, C=C-H
  1638 $cm^{-1}$, C=N, C=C

b) Chlorure de 14-amino 6,7-dihydro 12H-benzo-[f] indolo[2,3-a] quinolizinium (composé H).

Le composé H est préparé selon un mode opératoire identique à celui décrit ci-dessus dans le cadre de la préparation du composé G mais avec l'utilisation du composé B au lieu du composé A.
Le produit obtenu est de couleur jaune moutarde avec un rendement de 56 %.

- Pf supérieur à 260°C.
- Analyse élémentaire : $C_{19}H_{16}N_3Cl$

| | | | |
|---|---|---|---|
| Calculé % | C : 70,91 | H : 5,01 | N : 13,06 |
| Trouvé % | C : 70,33 | H : 5,06 | N : 12,71 |

c) Chlorure de 14-amino 6,7-dihydro 12H-2-chloro benzo-[1] indolo[2,3-a] quinolizinium (composé I).

Le composé I est préparé selon un mode opératoire identique à celui décrit ci-dessus dans le cadre de la préparation du composé G mais avec l'utilisation du composé C au lieu du composé A.
Le produit obtenu est de couleur marron clair avec un rendement de 7 %.

- Pf supérieur à 260°C.
- Analyse élémentaire : $C_{19}H_{15}N_3Cl_2$, $H_2O$

| | | | |
|---|---|---|---|
| Calculé % | C : 60,97 | H : 4,38 | N : 11,22 |
| Trouvé % | C : 61,32 | H : 5,03 | N : 10,52 |

d) Chlorure de 14-amino 6,7-dihydro 12H-3-chloro benzo-[1] indolo[2,3-a] quinolizinium (composé J).

Le composé J est préparé selon un mode opératoire identique à celui décrit ci-dessus dans le cadre de la préparation du composé G mais avec l'utilisation du composé D au lieu du composé A.
Le produit obtenu est de couleur marron clair avec un rendement de 52 %.

- Pf supérieur à 260°C.
- Analyse élémentaire : $C_{19} H_{15} N_3 Cl_2$

| | | | |
|---|---|---|---|
| Calculé % | C : 64,06 | H : 4,24 | N : 11,79 |
| Trouvé % | C : 63,89 | H : 4,48 | N : 11,58 |

e) Chlorure de 14-amino 6,7-dihydro 12H-4-chloro benzo-[1] indolo[2,3-a] quinolizinium (composé K).

Le composé K est préparé selon un mode opératoire identique à celui décrit ci-dessus dans le cadre de la préparation du composé G mais avec l'utilisation du composé E au lieu du composé A.
Le produit obtenu est de couleur marron clair avec un rendement de 6 %.

- Pf supérieur à 260°C.
- Analyse élémentaire : $C_{19}H_{15} N_3 Cl_2$, $H_2O$

| | | | |
|---|---|---|---|
| Calculé % | C : 60,97 | H : 4,58 | N : 11,22 |
| Trouvé % | C : 60,56 | H : 4,66 | N : 10,84 |

f) Chlorure de 14-amino 6,7-dihydro 12H-3-bromo benzo-[1] indolo[2,3-a] quinolizinium (composé L).

Le composé L est préparé selon un mode opératoire identique à celui décrit ci-dessus dans le cadre de la préparation du composé G mais avec l'utilisation du composé F au lieu du composé A.
Le produit obtenu est de couleur jaune moutarde avec un rendement de 12 %.

- Pf supérieur à 260°C.
- Analyse élémentaire : $C_{19} H_{15} N_3$ Cl, Br, $2H_2O$

| | | | |
|---|---|---|---|
| Calculé % | C : 52,25 | H : 4,38 | N : 9,62 |
| Trouvé % | C : 52,28 | H : 4,36 | N : 9,66 |

**IV- Etude des effets des composés de l'invention sur le CFTR :**

A) Méthodologie

a) Culture des cellules épithéliales humaines et des cellules recombinantes CHO :

Plusieurs types cellulaires sont utilisés pour cette étude : les lignées intestinales T84, Caco-2 et HT 29. Des cellules Chinese-Hamster-Ovary (CHO-K1) ont été transfectées à l'aide du vecteur pNUT (Tabcharani et al., 1991) incorporant ou non (cellules contrôles) l'ADNc CFTR normal ou muté (Chang et al., 1993). Les cellules sont maintenues dans ce milieu spécifique puis ensemencées à faible densité sur lamelles de verres et cultivées à 37°C (5% $CO_2$) avant les expériences de patch-clamp. Le milieu de survie des cellules est composé de αMEM avec du sérum de boeuf foetal (7%) et des antibiotiques : antibiotiques : 50 IU/ml pénicilline et 50 μg/ml streptomycine et méthotrexate (100 μM à 200 μM).

b) Principes de la technique d'électrophysiologie moléculaire ou patch-clamp :

L'activité électrique des cellules est contrôlée par la présence et le fonctionnement de pores transmembranaires, les canaux ioniques. Les canaux ioniques sont des protéines dont l'état conformationnel peut être modifié en réponse à différents facteurs : le champ électrique transmembranaire, la fixation de ligands ou des réactions biochimiques post-transcriptionnelles. Le courant traversant un canal ionique est de l'ordre du milliardième d'Ampère (pico Ampère, 1 pA = $10^{-12}$ A). Il peut être mesuré par les techniques d'électrophysiologie moléculaire plus communément nommées patch-clamp.

Avec les techniques classiques de mesure des courants transmembranaires par des microélectrodes intracellulaires, le bruit de fond thermodynamique est au moins cent fois supérieur au courant traversant un seul canal ionique. Dans de telles conditions, le flux dans un canal est masqué par ce bruit dont la variance croît avec le courant moyen. Erwin Neher et Bert Sackman (voir Hamill et al., 1981) du Max Planck Institut de Gôttingen ont montré que l'on pouvait par l'analyse du bruit estimer le courant passant dans un canal ionique. Le bruit de fond thermodynamique est proportionnel à la surface de la membrane. Ainsi, en limitant celle-ci, le bruit de fond devient inférieur au courant traversant le canal.

La technique du patch-clamp découle de l'observation faite par ces deux chercheurs et leurs collaborateurs : une micropipette de verre appliquée sur une surface membranaire y adhère de telle sorte que la résistance électrique établie entre la pipette et la membrane atteint la valeur du gigaohm (1 Gohm = $10_9$ ohm). La loi d'ohm donne la résistance électrique (R) par rapport à l'intensité du courant I (en Ampère) et le potentiel U (en Volt) imposé (équation 1) et permet de déterminer la conductance (unité : le picoSiemens, ps) unitaire du canal g (équation 2).

(équation 1) $$U = R.I$$

(équation 2) $$g = 1/R$$

Les forces d'interaction entre le verre de la pipette et les phospholipides de la membrane permettent de réduire les courants de fuite, étape indispensable pour la mesure du courant traversant un canal ionique. La configuration obtenue ainsi est désignée par le terme de "cell-attached" ou cellule attachée. En retirant la pipette à partir de la configuration cellule attachée, on arrache un fragment (patch) de membrane qui reste fixée à l'extrémité de la pipette. La configuration ainsi obtenue est dite "inside-out" car la face intracellulaire de la membrane se retrouve dans le bain. La face extracellulaire de la membrane est en contact avec la solution contenue dans la pipette alors que la partie intracellulaire est en contact avec le milieu de perfusion de la cuve expérimentale. Un montage électronique permet d'imposer (to clamp) une différence de potentiel (Vref-Vp) entre l'électrode de référence du bain (Vref) et la pipette (Vp) et de mesurer le courant I résultant. Si la composition ionique est la même de part et d'autre de la membrane (milieux symétriques), l'intensité du courant I est alors directement proportionnelle à la différence de potentiel imposée à la membrane. Les points I(V) se répartissent généralement sur une droite dont la pente et la position définissent la conductance unitaire (g) du canal et le potentiel d'inversion du courant, Erev, Au potentiel d'inversion, le flux de charges à travers la membrane est nul. Le potentiel d'inversion sera défini par l'équation de Nernst (équation 3) ou E représente le potentiel de Nernst pour l'ion considéré et C la concentration de l'ion dans les compartiments extracellulaire (Co) et intracellulaire (Ci).

(équation 3) $$E = RT/_{z}F \text{ Log } Co/Ci$$

En configuration cell-attached, le potentiel de l'électrode s'additionne à celui de la membrane qui est d'environ -60 mV. Dans toutes les cellules, les concentrations en ions potassium ($K^+$) et chlore ($Cl^-$) sont respectivement voisines de 150 mM et 10 mM. Avec une pipette contenant 150 mM KCl, les potentiels de Nernst pour les ions $K^+$ ($E_K$) et $Cl^-$ ($E_{Cl}$) seront respectivement proches de zéro et -50 mV. L'inversion du courant chlore sera donc obtenue au voisinage du potentiel de repos, c'est-à-dire sans appliquer de potentiel à la membrane (Vp = O mV). Par contre, l'inversion d'un courant potassium sera obtenu en annulant le potentiel de la membrane, donc en la dépolarisant de 50 à 60 mV. Cet exemple illustre les stratégies utilisées pour apprécier la nature ionique d'un canal. Les informations collectées par cette technique sont représentées par la fluctuation d'un courant électrique (de l'ordre de la milliseconde, ms) qui traduit les transitions entre les différents états de conductance du canal.

c) Patch-clamp appliqué à l'étude des cellules épithéliales en culture.

Les expériences de patch-clamp sont effectuées sur des cellules confluantes. Un fragment de la lamelle de verre (support des cellules) est placé dans une cuve d'expérimentation (volume 600 µl ou 1 ml) sur la platine d'un microscope inversé, équipé avec un éclairage en contraste de phase (Olympus IMT2). Les configurations cell-attached et inside-out sont utilisées (Hamill et al., 1981). Les expériences sont réalisées à température ambiante (20-22°C). Les courants sont amplifiés avec un amplificateur LIST EPC 7 (Darmstadt, Germany) (Filtre de 3 kHz) avec un filtre

passe-bas de 2-5 kHz (filtre Bessel à 6 pôles) et enregistrés avec un DAT (Digital Audio Tape) après digitalisation (16 bits) à 44 kHz. Les données sont ensuite transférées sur un ordinateur Olivetti M28PC. La fabrication des pipettes s'effectue à partir de tubes de verre de 1 mm de diamètre (Clark Electromedical Instrument) en deux ou trois étapes avec une étireuse horizontale (type Flaming/Brown, modèle P-87. Sutter Inst. Co. USA). Les pipettes remplies d'une solution de 150 mM NaCl ont une résistance comprise entre 4 et 12MΩ. Les potentiels sont exprimés comme la différence entre le potentiel de l'électrode de patch et celui du bain. En configuration cell-attached, ils représentent le changement de potentiel par rapport au potentiel de repos de la cellule. Les potentiels de jonction sont évalués par le potentiel de l'électrode correspondant à un courant nul (lorsque les canaux sont fermés). Ils sont minimisés en utilisant un pont d'agar établissant la connexion entre le bain et l'électrode de référence (terre) et contenant la même solution que celle de la pipette. Le potentiel d'inversion du courant et la conductance unitaire des canaux sont obtenus à partir de la relation courant-voltage (I/V) par régression linéaire. Pour déterminer la relation courant-voltage, les amplitudes courants ioniques sont mesurées à partir d'histogrammes d'amplitude. Les histogrammes d'amplitude se présentent comme la somme de deux ou plusieurs distributions Gaussiennes dont les pics correspondent aux états ouverts et fermés des canaux présents dans l'électrode. A partir de ces histogrammes on peut déterminer : N, le nombre total de canaux présents dans le patch ; n, le nombre de canaux simultanément ouverts (n=O, 1 2...N);Po, la probabilité d'ouverture d'un canal; et I, l'intensité moyenne du courant dans un canal. Lorsque les canaux en présence sont de même type et supposés s'ouvrir et se fermer indépendamment les uns des autres, la probabilité d'avoir n canaux ouverts simultanément est donnée par la distribution binomiale (équation 4) d'où l'on déduit la probabilité individuelle Po (équation 5).

$$\text{(équation 4)} \qquad P(n) = (N!/n!(N-n)Po^{n}.(1-Po)^{N-n}$$

$$\text{(équation 5)} \qquad Po = P(n)/N$$

L'étude présente ne concernant que des canaux $Cl^-$, un courant sortant devra être interprété comme un mouvement d'ions $Cl^-$ sortant de la pipette vers le milieu intracellulaire des cellules ou vers le milieu de perfusion. La perméabilité relative $P_X/P_{Cl}$ d'un anion $X^-$ par rapport aux ions $Cl^-$ a été utilisée pour évaluer la sélectivité ionique des canaux en configuration inside-out. L'équation de Goldman-Hodgkin-Katz (équation 6) permet de relier le rapport des perméabilités en fonction du potentiel d'inversion (Erev) obtenu expérimentalement et des concentrations respectives des anions en présence.

$$\text{(équation 6)} \qquad Erev = -RT/F \ln ([Cl^-]_e + P_X/P_{Cl} [X^-]_e/[Cl^-]_i + P_X/P_{Cl} [X^-]_i)$$

i et e : concentration ionique intracellulaire et extracellulaire, R, T et F ont leur signification habituelle.

Pour le remplissage des électrodes de patch, la composition des solutions salines est (en mM) ; 150 NaCl, 2 $MgCl_2$, 10 TES (pH 7,4). Le bain de perfusion des cellules contient (en mM) : 145 NaCl, 4 KCl, 2 $MgCl_2$, 0,5 $CaCl_2$, 10 TES (pH 7,4).

d) Mesure des courants de court-circuit en chambre de Ussing.

L'intérêt de la culture des épithéliums dans des chambres à fond perméable et notamment des épithéliums digestifs (lignée HT 29 et ses différents clones, lignée T84 ou Caco2) a été largement démontré lors d'études de biologie cellulaire. Dans cette technique, les cellules sont mises en culture à l'intérieur d'une cupule où le fond est constitué d'une membrane de polystyrène percée de trous dont le diamètre (entre 0,45 et 3 µm) et la répartition sont soigneusement mesurés. Elle permet l'attachement des cellules sans ajouter de matrice supplémentaire. Elle est transparente dans un milieu dont l'indice de réfraction est voisin de celui de l'eau et permet l'examen optique de la couche cellulaire. Cependant des limitations existent. Bien que la faible épaisseur de la membrane limite la rétention des fluides, on ne peut exclure qu'elle puisse constituer un piège pour des macromolécules ou des complexes comme les gélosomes. Cette cupule de 5 $cm^2$ de surface est placée dans une plaque à 6 puits. L'attachement et la culture des cellules y sont conduits de manière traditionnelle. Une quinzaine de jours après l'ensemencement les cellules forment une monocouche étanche. Cette étanchéité est avérée par l'apparition d'une résistance électrique ou par la non diffusion de macromolécules entre les deux compartiments muqueux et séreux. La culture est stable une dizaine de jours en maintenant un milieu de culture identique dans les deux compartiments. Cette culture des épithéliums dans des chambres à fond perméable est tout à fait utile pour étudier la nature et la régulation des sécrétions et des passages de molécules chargées ou non chargées à travers l'épithélium. Le transport transépithélial va dépendre de la nature

des perméases présentes dans les deux domaines apicaux ou basolatéraux, de part et d'autre de la jonction serrée. Les propriétés de l'épithélium qui se traduisent par un passage de molécules chargées, peuvent être facilement déduites de la mesure du potentiel transépithélial ou du courant de court-circuit. Quand les molécules sont neutres on utilise des molécules marquées pour suivre leurs mouvements.

e) Principes de la méthode de mesure.

Schématiquement le transport transépithélial est le bilan du transport cellulaire et de la diffusion sélective à travers la jonction. Le transport cellulaire résulte de l'activité de couples de perméases spécifiques situées respectivement sur le pôle apical et sur le pôle basal de la cellule (canal $Na^+$ et $Na^+/K^+$ ATPase, canal $Cl^-$ et cotransporteur $Na^+/K^+/Cl^-$, cotransporteur Na/glucose et transporteur diffusionnel du glucose ...). Quand l'épithélium transporteur est étanche la jonction serrée isole deux parties de la membrane qui ont un potentiel différent par rapport au milieu. On peut utiliser une analogie électrique simple et les considérer comme des éléments de circuit ayant respectivement les potentiels Vm (muqueux) et Vs (séreux). Ces deux membranes en série ont donc un potentiel Vt qui est la somme algébrique Vm + Vs. Dans le tissu utilisé Vt peut atteindre -5 mV dans les conditions standards. Pour déterminer les caractéristiques de l'épithélium on peut utiliser trois types de mesure.

<u>Mesure en circuit ouvert.</u>

On mesure la différence de potentiel existant d'une part et d'autre de la couche cellulaire. Puis à temps fixe, on envoie dans le circuit un courant paramétrable i (µA) qui provoque une variation de différence de potentiel $\Delta V$ proportionnelle à la résistance du circuit.

<u>Mesure en courant de court-circuit.</u>

On introduit dans le circuit un courant i, ajustable, qui utilise la résistance du tissu pour créer une différence de potentiel qui va s'ajouter algébriquement à celle existante. Quand le courant a la valeur Isc (courant de court-circuit) Vt est nul ; Isc x Rt - Et = 0 ou Isc = Et/Rt. Dans ces conditions Vm-Vs=0 et Vm=Vs, les deux membranes muqueuse et séreuse sont au même potentiel. Pour déterminer la résistance on impose au circuit une différence de potentiel choisie et on mesure la résistance en appréciant la déviation du courant Isc qui en résulte.

<u>Mesure en voltage imposé.</u>

C'est un cas particulier de la mesure en courant de court-circuit où l'on impose à l'épithélium d'avoir un potentiel nul. Dans ces conditions on fixe le potentiel transépithélial sans connaître le potentiel de chacune des membranes. Pour cela on surimpose une différence de potentiel en prolongeant l'ajout algébrique de courant qui sert à la mesure de la résistance. On choisit un temps court pour le courant de court-circuit et un temps long pour le surajout. Il est clair que la différence de potentiel va dépendre, pour une résistance donnée de la capacité de l'appareil à délivrer un courant maximum (100 µA ; pour 500 ohms la différence de potentiel est de 50 mV) mais aussi de pouvoir mesurer la différence de potentiel résultante (1 V).

Pour réaliser ces mesures les cupules sont montées dans une chambre de Ussing modifiée. Nous utilisons une unité de contrôle "courant-voltage clamp" (WPI), couplée à un générateur d'impulsions permettant la production de courbe intensité, voltage. Le signal recueilli est digitalisé (MacLab) et traité en utilisant le logiciel Chart sur un ordinateur Macintosh Apple.

f) Propriétés de l'épithélium testé.

Dans un premier temps on utilisera l'épithélium formé par les cellules HT29. L'ajout de glucose au milieu de base (milieu salin symétrique, absence de stimulateur) dans le compartiment muqueux provoque une élévation du potentiel transépithélial et du courant de court-circuit sans affecter la résistance. L'épithélium fonctionne comme un absorbeur de glucose qui utilise du côté muqueux un transporteur de glucose Na dépendant, et sans doute un transporteur diffusionnel du côté séreux. Le transport de sodium associé au glucose provoque une différence de potentiel qui peut être inhibée par la phlorizine tandis que le flux net de glucose est mesuré avec des analogues de glucose radiomarqués placés dans les compartiments muqueux ou séreux.

L'ajout dans le milieu d'agents qui provoquent une augmentation du taux d'AMPc induit un accroissement de Vt et de Isc qui est indépendant du glucose. Il paraît lié à la mise en place d'un transport transépithélial de $Cl^-$ (séreux vers muqueux) et implique un transporteur basolatéral de chlorure du côté séreux et un canal chlorure du côté muqueux. Ce transport de $Cl^-$ est associé à un transport d'eau de même sens. L'application, après une élévation du taux d'AMPc,

d'agents qui provoquent une élévation du taux de $Ca^{2+}$ intracellulaire entraîne une nouvelle augmentation de Vt et de Isc qui associe, sans doute un passage de $Ca^{2+}$ muqueux vers séreux et un nouveau passage de $Cl^-$ séreux vers muqueux.

g) Mesure des flux de traceurs radioactifs appliqué à l'étude des cellules épithéliales en culture.

Cette technique permet de suivre la cinétique de sortie de l'iodure. Les cellules sont mises en culture dans des plaques 12 puits avec une dilution au 1/10 après passage. Au jour 3, les drogues à tester sont mises en solution dans du milieu B (37°C) en fonction de la concentration voulue. Les puits sont lavés 2 fois avec 1 ml de milieu B NaOH, 0,1 % glucose, qui est ensuite remplacé par 1 ml de solution de charge pendant 30 min.

La cinétique de sortie de l'iodure est réalisée après avoir éliminé la solution de charge et lavé 3 fois les puits par 1,5 ml de milieu B. Pour cela 1 ml de milieu B est laissé 1 min dans le puits et récupéré dans un tube à hémolyse pour être remplacé par 1 ml de milieu B neuf. La première minute sert de contrôle, le produit à tester est rajouter à partir de la deuxième minute. L'opération est répétée sur 10 min puis, les celles sont décrochées avec 1 ml de NaOH 0,1N SDS 0,1%. Le contenu de chaque puits est récupéré dans un tube à hémolyse après 25 mn d'agitation et les tubes sont comptés 2 min dans un compteur gamma.

B) Résultats

L'étude concerne des molécules de la famille des benzoquinoliziniums, décrites ci-dessus. Elles sont testées pour leur capacité à activer le canal CFTR. Le criblage des molécules en tant qu'ouvreurs du canal CFTR a été réalisé en mesurant leur effet sur l'efflux d'iodure radioactif et sur les courants de chlorure transmembranaires (Becq et al., 1993a). Ces données ont été complémentées par la mesure du taux d'AMP cyclique (AMPc) intracellulaire et de ses variations dans diverses situations expérimentales

. Trois modèles cellulaires ont été utilisés pour évaluer l'effet d'activation du CFTR par les benzoquinoliziniums : l'ovocyte de Xénope injecté avec de l'ARN codant pour le CFTR (cet ARN étant désigné par la suite ARNc-CFTR); la cellule recombinante CHO exprimant de manière stable la protéine CFTR, et la cellule colonique humaine de lignée HT29 exprimant constitutivement la protéine CFTR. Le canal CFTR étant principalement régulé par des protéines kinases A stimulés par le taux d'AMPc intracellulaire, les expériences contrôles ont fait appel à des dérivés de l'AMPc capables de passer la membrane cellulaire, et à la forskoline activateur de l'enzyme adénylate cyclase conduisant à la synthèse d'AMPc dans une cellule. La figure 1 montre une telle activation obtenue avec 500µM de cpt-AMPc (8-(4-chlorphénylthio)-adénosine-3',5'-monophosphate, cyclique), un analogue de l'AMPc traversant la membrane des cellules. L'activation du canal CFTR, mesurée par l'efflux de iodure, induit une augmentation de l'amplitude de l'efflux d'iodure (exprimé en % du contenu cellulaire au temps t=0) et de la vitesse de sortie de l'iodure (pente des courbes à l'origine).

Les expériences contrôle permettant d'évaluer l'efficacité des molécules testées sur l'activité du canal CFTR sont les suivantes :

1) ovocyte de Xénope : efflux de iodure radioactif et courant chlorure transmembranaire dans :

- ovocytes injectés avec l'ARNc-CFTR (notés CFTR sur la figure 1 B);
- les mêmes ovocytes en présence d'activateurs de la voie AMPc (cpt-AMPc, forskoline);
- ovocytes injectés avec de l'eau (notés "eau" sur la figure 1 B) aux lieu et place de l'ARNc-CFTR;
- les mêmes ovocytes mis en présence d'activateurs, ci-dessus mentionnés, de la voie AMPc.

2) cellule CHO: efflux de iodure radioactif dans

- cellules CHO non transfectées avec la protéine CFTR (notées, CHO-CFTR(-) sur la figure 2 B) en présence ou non d'activateurs, ci-dessus mentionnés;
- cellules CHO transfectées avec le gène CFTR (notées, CHO-CFTR(+) sur la figure 2 B) en présence ou non des activateurs, ci-dessus mentionnés.

3) cellule HT29 : efflux d'iodure radioactif dans

- cellules HT29 en l'absence d'activateur (notées basal sur la figure 3);
- cellules en présence d'activateurs (notées AMPc sur la figure 3), ci-dessus mentionnés.

La présence d'un canal chlorure activé par l'augmentation du niveau du calcium intracellulaire a été testé en

présence de l'ionophore calcium A23187. Les effets des activateurs de l'AMPc, de l'A23187 et des benzoquinoliziniums ont été évalués dans ces diverses conditions expérimentales (considérées *mutatis mutandis* comme contrôle, notés basal) par leur capacité à favoriser l'efflux de iodure radioactif et à augmenter le courant chlorure transmembranaire.

Les figures 2 A et 2 B montrent l'activation du CFTR par 500µM de cpt-AMPc dans la cellule recombinante CHO exprimant CFTR. Dans la cellule CHO (CFTR-) témoin le cpt-AMPc est sans effet. Dans la cellule HT29 le canal CFTR est stimulable par l'AMPc comme le montre l'augmentation de l'amplitude du flux de iodure en présence de 500µM de cpt-AMPc (figure 3 A et B).

Effet des benzoquinoliziniums sur l'activation du canal CFTR dans la cellule CHO

La figure 4 montre l'effet du dérivé MPB-07 (500µM) sur l'efflux d'iodure dans la cellule CHO (CFTR+) et CHO (CFTR-). L'activation de l'efflux d'iodure dans la cellule CHO (CFTR+) est comparable, en intensité et vitesse, à celle induite par la forskoline (5µM), activateur de l'AMPc, dans les cellules CHO (CFTR+) (figure 5). Les résultats concernant les effets du dérivé MPB-07 sur l'efflux d'iodure dans les cellules CHO (CFTR-) et CHO (CFTR+) sont résumés dans les histogrammes des figures 6 et 7, respectivement. MPB-07 (500µM) stimule aussi efficacement l'efflux d'iodure que le fait la forskoline (5µM) sur les cellules CHO (CFTR+) (figure 7). Sur ces mêmes cellules, A23187 (10µM) est sans effet. Dans les cellules CHO (CFTR-), forskoline (5µM) A23187 (10µM), soit séparément soit ajoutés conjointement, ainsi que MPB-07 (500µM) ne modifient pas significativement le niveau basal de l'efflux d'iodure (figure 6).

Effet des benzoquinoliziniums sur l'activation du canal CFTR dans la cellule HT29

L'effet du MPB-07 sur l'efflux de iodure dans la cellule recombinante CHO est reproduit dans la cellule épithéliale HT29. L'application de 500µM de cpt-AMPc (figure 8 A) ou de 500µM de MPB-07 (figure 8 B) déclenche, avec une vitesse et une amplitude similaire un efflux d'iodure accru par rapport au niveau basal (sans activateur) (figure 9).

Effet des benzoquinoliziniums sur l'activation du canal CFTR exprimé dans l'ovocyte de Xénope

MPB-07 (500µM) stimule l'efflux d'iodure dans l'ovocyte de Xénope. Cette activation (figure 10 A) est comparable à celle obtenue par l'application de 500 µM de cpt-AMPc (figure 10 B) et significativement différente (figure 11) de l'efflux mesuré dans l'ovocyte non-injecté stimulé (AMPc, et MPB-07) et non stimulé (basal) (figure 11, eau) et dans l'ovocyte non stimulé mais exprimant CFTR (figure 10 A, efflux noté CFTR basal).

Etude structure-fonction des benzoquinoliziniums et corrélation avec l'ouverture du CFTR

L'étude a porté sur 16 dérivés de noyau benzoquinolizinium.

Les tableaux 1 et 2 présentent la structure chimique des composés de la famille des benzoquinoliziniums testés ici en tant qu'activateur du canal CFTR.

Les tableaux 1 et 2 présentent les résultats relatifs aux efflux mesurés en présence des différents composés testés sur la cellule CHO (CFTR+) recombinante. Le composé de base, le phenanthrène (tableau 2) n'active pas le canal CFTR. Deux séries de molécules ont été testées : série $NH_2$ (tableau 1, MPB-26, MPB-01 à 04; tableau 2: MPB-24) et série OH (tableau 1, MPB-05 à 08, MPB-27, 29, 30 et 32; tableau 2: MPB-25). Les pourcentages d'activation du canal CFTR sont donnés dans les tableaux correspondants. Ils montrent que la série OH active CFTR avec des pourcentages compris entre 15 et 110 %. La série $NH_2$ est moins active (10 à 30 %).

Dans la série OH, l'efficacité est la suivante :

$$\text{MPB-05, 08, 25, 32} < 30 < 29 < 06 < 27, 07$$

De l'ensemble des composés étudiés, il apparaît que la présence du groupement OH en position 6 est déterminante quant à la capacité d'activer le canal CFTR :

. 9 composés avec OH en position 6 : 45 % d'activation du canal CFTR
. 6 composés avec $NH_2$ en position 6 : 13 % d'activation du canal CFTR.

Effet des benzoquinoliziniums sur l'AMPc intracellulaire

La figure 12 présente les taux en AMPc cellulaire dans la cellule CHO recombinante mesurés après 5min en présence de 5µM de forskoline (activateur de l'enzyme de synthèse de l'AMPc ; adénylate cyclase), de 10µM de rolipram (un inhibiteur de l'enzyme de dégradation de l'AMPc : la phosphodiesterase de type IV) et de 500µM de MPB-07. Le même niveau d'AMPc est atteint en présence des composés MPB-07 et du rolipram mais seul MPB-07 déclenche l'activation du canal CFTR. L'effet forskoline sur l'AMPc est multiplié par un facteur d'environ 4 suggérant que cet effet est purement dépendant de l'AMPc. Le rolipram n'a aucun effet activateur de CFTR mesuré en flux de iodure et en patch-clamp. Ces résultats montrent que le composé MPB-07 stimule le canal CFTR par une voie indépendante de la voie de l'AMPc cellulaire.

C) Conclusion

Ces résultats montrent que le composé MPB-07 et certains membres de la famille des benzoquinoliziniums stimulent l'ouverture du canal CFTR par une voie indépendante de l'AMPc ou du calcium intracellulaire. Ces molécules représentent donc une nouvelle famille d'activateurs du canal CFTR.

Légendes des figures

- Figure 1 : effet du cpt-AMPc sur l'efflux d'iodure radioactif, dans l'ovocyte de Xénope ;

  . figure 1 A : courbes de l'efflux de $^{125}I$ (% en ordonnée) en fonction de temps (mn en abscisse) ; la courbe passant par des points représentés par des carrés noirs correspond à l'efflux de $^{125}I$ mesuré en fonction de temps dans des ovocytes injectés avec l'ARNc-CFTR (courbe désignée CFTR basal) ; la courbe passant par des points représentés par des carrés blancs correspond à l'efflux de $^{125}I$ mesuré en fonction de temps dans des ovocytes injectés avec l'ARNc-CFTR et activés par cpt-AMPc (courbe désignée CFTR+AMPc) ;
  . figure 1 B : histogrammes de l'efflux de $^{125}I$ dans les ovocytes non activés par cpt-AMPc (représentés en noir) et dans les ovocytes activés par cpt-AMPc (représentés en blanc) ; à gauche sont représentés les ovocytes activés ou non par cpt-AMPc et injectées avec de l'eau (notés "eau") ; à droite sont représentés les ovocytes activés ou non par cpt-AMPc et injectés avec l'ARNc-CFTR (notés "CFTR") ; n représente le nombre d'expériences.

- Figure 2 : effet du cpt-AMPc sur l'efflux d'iodure radioactif dans les cellules CHO ;

  . figure 2 A : courbes de l'efflux de $^{125}I$ (% en ordonnée) en fonction de temps (mn en abscisse) ; la courbe passant par des points représentés par des triangles noirs correspond à l'efflux de $^{125}I$ mesuré en fonction de temps dans des cellules CHO non activées par cpt-AMPc (courbe désignée basal) ; la courbe passant par des points représentés par des triangles blancs correspond à l'efflux de $^{125}I$ mesuré en fonction de temps dans des cellules CHO activées par cpt-AMPc (courbe désignée AMPc) ;
  . figure 2 B : histogrammes de l'efflux de $^{125}I$ dans les cellules CHO non activées par cpt-AMPc (représentées en blanc) ; à gauche sont représentées les cellules CHO activées ou non par cpt-AMPc et non transfectées avec le gène CFTR (notées CHO (CFTR-)) ; à droite sont représentées les cellules CHO activées ou non par cpt-AMPc et transfectées avec le gène CFTR (notées CHO (CFTR+)) ; n représente le nombre d'expériences.

- Figure 3 : effet du cpt-AMPc sur l'efflux d'iodure radioactif dans les cellules HT29 ;

  . figure 3 A : courbes de l'efflux de $^{125}I$ (% en ordonnée) en fonction de temps (mn en abscisse) ; la courbe passant par des points représentés par des carrés noirs correspond à l'efflux de $^{125}I$ mesuré en fonction de temps dans des cellules HT29 non activées par cpt-AMPc (courbe désignée basal) ; la courbe passant par des points représentés par des carrés blancs correspond à l'efflux de $^{125}I$ mesuré en fonction de temps dans les cellules HT29 activées par cpt-AMPc (courbe désignée AMPc) ;
  . figure 3 B : histogrammes de l'efflux de $^{125}I$ dans les cellules HT29 non activées par cpt-AMPc (en noir), et dans les cellules HT29 activées par cpt-AMPc (en blanc).

- Figure 4 : effet du dérivé MPB-07 (500µM) sur l'efflux de $^{125}I$ (% en ordonnée) en fonction de temps (mn en abscisse) dans la cellule CHO ; la courbe passant par des points représentés par des cercles blancs correspond à la mesure de l'efflux de $^{125}I$ en fonction de temps dans les cellules CHO activées par MPB-07 et transfectées avec le gène CFTR (courbe désignée MPB-07 (CFTR+); la courbe passant par des points représentés par des cercles noirs correspond à la mesure de l'efflux 125I en fonction de temps dans les cellules CHO activées par MPB-07 mais pas transfectées avec le gène CFTR (courbe désignée MPB-07 (CFTR-)).

- Figure 5 : effet de la "forskoline" (500 µM) sur l'efflux de $^{125}I$ (% en ordonnée) en fonction de temps (mn en abscisse) dans la cellule CHO ; la courbe passant par des points représentés par des triangles blancs correspond à la mesure de l'efflux de $^{125}I$ en fonction de temps dans les cellules CHO activées par la forskoline et transfectées avec le gène CFTR (courbe désignée forskoline CHO (CFTR+)) ; la courbe passant par des points représentés par des triangles noirs correspond à la mesure de l'efflux $^{125}I$ en fonction de temps dans les cellules CHO non activées par la forskoline et transfectées avec le gène CFTR (courbe désignée basal CHO (CFTR+)).

- Figure 6 : histogrammes de l'efflux de $^{125}I$ dans les cellules CHO non transfectées avec le gène CFTR (désignées CHO (CFTR-)) et :

  . non activées (basal)
  . activées par la forskoline (forskoline)
  . activées par A23187 (A23187)
  . activées par A23187 et la forskoline (A23187+fsk)
  . activées par MPB-07 (MPB-07)
  n représente le nombre d'expériences.

- Figure 7 : histogrammes de l'efflux de $^{125}I$ dans les cellules CHO transfectées avec le gène CFTR (désignées CHO (CFTR+)) et :

  . non activées (basal)
  . activées par la forskoline (forskoline)
  . activées par MPB-07
  . activées par A23187 (A23187)
  n représente le nombre d'expériences.

- Figure 8 : effets comparés du cpt-AMPc (500 µM) et du MPB-07 (500 µ M) sur l'efflux de $^{125}I$ (% en ordonnée) en fonction du temps (mn en abscisse) dans les cellules HT29 ;

  . figure 8 A : la courbe passant par des points représentés par des carrés noirs correspond à la mesure de l'efflux de $^{125}I$ en fonction du temps dans les cellules HT29 non activées (courbe désignée basal) ; la courbe passant par des points représentés par des carrés blancs correspond à la mesure de l'efflux de $^{125}I$ en fonction du temps dans les cellules HT29 activées par cpt-AMPc (courbe désignée AMPc) ;
  . figure 8 B : la courbe passant par des points représentés par des carrés blancs correspond à la mesure de $^{125}I$ en fonction du temps dans les cellules HT29 non activées (courbe désignée basal) ; la courbe passant par des points représentés par des carrés noirs correspond à la mesure de l'efflux de $^{125}I$ en fonction du temps dans les cellules HT29 activées par MPB-07 (courbe désignée MPB-07).

- Figure 9 : histogramme de l'efflux de $^{125}I$ dans les cellules HT29 non activées (basal), activées par cpt-AMPc (AMPc) et activées par MPB-07 (MPB-07) ; n représente le nombre d'expériences.

- Figure 10 : effets comparés du cpt-AMPc (500µM) sur l'efflux de $^{125}I$ (% en ordonnée) en fonction du temps (non en abscisse) dans les ovocytes de Xénope :

  . figure 10 A : la courbe passant par des points représentés par des cercles blancs correspond à la mesure de l'efflux de $^{125}I$ en fonction du temps dans les ovocytes injectés avec de l'eau (courbe désignée eau basal) ; la courbe passant par des points représentés par des carrés noirs correspond à la mesure de l'efflux de $^{125}I$ en fonction du temps dans les ovocytes injectés avec l'ARNc-CFTR (courbe désignée CFTR basal) ; la courbe passant par des points représentés par des carrés blancs correspond à la mesure de l'efflux de $^{125}I$ en fonction du temps dans les ovocytes injectés avec l'ARNc-CFTR et activés avec cpt-AMPc (courbe désignée CFTR + AMPc) ;
  . figure 10 B : la courbe passant par des points représentés par des carrés blancs correspond à la mesure de l'efflux de $^{125}I$ en fonction du temps dans les ovocytes injectés avec l'ARNc-CFTR et activés par MPB-07 (courbe désignée MPB-07).

- Figure 11 : histogrammes de l'efflux de $^{125}I$ dans les ovocytes de Xénope non activés (basal), ou activés par cpt-AMPc (AMPc), ou activés par MPB-07 (MPB-07), ces ovocytes étant soit injectés avec de l'eau (notés "eau" sur la gauche), soit injectés avec de l'ARNc-CFTR (notés "CFTR" sur la droite); n représente le nombre d'expériences.

- Figure 12 : histogrammes représentant les taux en AMPc (mesurés en nmole par mg de protéine) dans les cellules CHO transfectées avec le gène CFTR et non activées (basal), ou activées par la forskoline (forskoline), ou activées par le rolipran (rolipran), ou activées par MPB-07 (MPB-07).

Tableau 1

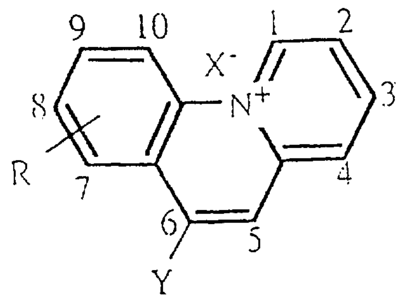

| Composé | X | Y | R | Activation en % par rapport au témoin |
|---|---|---|---|---|
| MPB-26 | Cl | $NH_2$ | H | 30 |
| MPB-03 | Cl | $NH_2$ | 9-Cl | 18 |
| MPB-04 | Cl | $NH_2$ | 7-Cl | 11 |
| MPB-06 | Br | OH | H | 53 |
| MPB-05 | Cl | OH | H | 20 |
| MPB-07 | Cl | OH | 10-Cl | 110 |
| MPB-08 | Cl | OH | 9-Cl | 22 |
| MPB-27 | Cl | OH | 7-Cl | 95 |
| MPB-30 | Cl | OH | 8-Cl | 28 |
| MPB-29 | Cl | OH | 9-F | 42 |
| MPB-32 | Cl | OH | 8-Br | 13 |
| témoin (basal) | | | | 0 |
| forskoline | | | | 109 |

N.B. : forskoline : 5$\mu$M, MPB et phénanthrène : 500$\mu$M.

Tableau 2

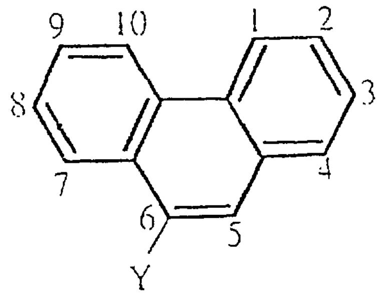

| Composé | X | Y | R | Activation en % par rapport au témoin |
|---|---|---|---|---|
| phénanthrène | H | H | H | <5 |
| MPB-24 | H | $NH_2$ | H | 18 |
| MPB-25 | H | OH | H | 25 |

Bibliographie

Becq F. et al.(1993a). FEBS Lett. 327 : 337-342

Becq F. et al. (1993b). Pflügers Arch 425 : 1-8

Becq F. et al. (1994). PNAS 91, 9160-9164

Chang X-B et al. (1993). J. Biol Chem. 268 : 11304-11311

Cutting G.R. et al. (1990). Nature 346 : 366-369

Dalemans W. et al. (1991). Nature 354 : 526-528

Dils F. (1979), J. Int. Med. Res 7 : 302-304

Drumm M.L. et al. (1991). Science 254 : 1797-1999

Gregory R.J. et al. (1994). Mol. Cell. Biol. 11 : 3886-3893

Grem J.L. (1990). Cancer cells. 2 : 131-137

Gribkoff V.K. et al. (1994). J. Biol. Chem. 269 : 10983-10986

Hamill O.P. et al. (1981). Pflügers Arch 391 : 85-100

Kerem B-S. et al. (1989). Science 245 : 1073-1080

Olesen S.P. et al. (1994), Europ. J. Pharm. 251 : 53-59

Riordan J.R. et al. (1989). Science 245 : 1066-1072

Sheppard D.N. et al. (1993). Nature 362 : 160-164

Tabcharani J.A. et al. (1991). Nature 352 : 628-631

Tsui L-C & Buchwald M. (1991). Advances in human genetic 20 : 153-266

Van Eygen M. et al. (1976). The Lancet 1 : 382-385

## Revendications

**1.** Utilisation de dérivés des benzo[c] quinoliziniums de formule générale (III) suivante :

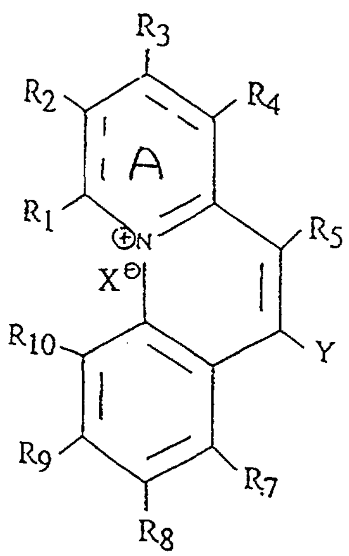

(III)

dans laquelle :

- l'hétérocycle A est aromatique ou non, étant entendu que dans ce dernier cas l'atome d'azote de cet hétérocycle est lié par une double liaison au carbone en position 4a,
- $R_1$, $R_2$, $R_3$, $R_4$, $R_5$, $R_7$, $R_8$, $R_9$ et $R_{10}$, représentent, indépendamment les uns des autres:
  - . un atome d'hydrogène, ou de brome, ou de fluor, ou
  - . un atome d'halogène, notamment un atome de chlore, ou
  - . un groupe alkyle, alkoxy, carbonyle, ou oxycarbonyle, linéaire ou ramifié, de 1 à 10 atomes de carbone, ces groupes étant le cas échéant substitués, notamment par un halogène, et/ou par un hydroxyle, et/ou par une amine (primaire, secondaire ou tertiaire), et/ou par un cycle aromatique et/ou aliphatique, de 5 à 10 atomes de carbone dans le cycle, ces cycles étant eux-mêmes, le cas échéant, substitués notamment par un halogène, et/ou par un hydroxyle, et/ou par une amine (primaire, secondaire ou tertiaire), et/ou par un groupe alkyle, alkoxy, carbonyle, ou oxycarbonyle, ces groupes étant tels que définis ci-dessus, ou

. un cycle aromatique ou aliphatique, de 5 à 10 atomes de carbone dans le cycle, ce cycle étant lui-même, le cas échéant, substitué notamment par un halogène, et/ou par un hydroxyle, et/ou par une amine (primaire, secondaire ou tertiaire), et/ou par un groupe alkyle, alkoxy, carbonyle, ou oxycarbonyle, ces groupes étant tels que définis ci-dessus, ou

. un groupe $-OR_a$, $R_a$ représentant un atome d'hydrogène, ou un groupe alkyle, carbonyle, ou oxycarbonyle, linéaire ou ramifié, ces groupes étant tels que définis ci-dessus, ou un cycle aromatique ou aliphatique, ces cycles étant tels que définis ci-dessus, ou

. un groupe $-NR_bR_c$, $R_b$ et $R_c$, indépendamment l'un de l'autre, représentant un groupe alkyle, alkoxy, carbonyle, ou oxycarbonyle, linéaire ou ramifié, ces groupes étant tels que définis ci-dessus, ou un cycle aromatique ou aliphatique, ces cycles étant tels que définis ci-dessus, ou

. lorsque $R_1$ et $R_2$, ou $R_3$ et $R_4$, et/ou $R_4$ et $R_5$, et/ou $R_7$ et $R_8$, et/ou $R_8$ et $R_9$, et/ou $R_9$ et $R_{10}$, ne représentent pas les différents atomes ou groupes ou cycles mentionnés ci-dessus, alors $R_1$ en association avec $R_2$, ou $R_2$ en association avec $R_3$, et/ou $R_3$ en association avec $R_4$, et/ou $R_4$ en association avec $R_5$, et/ou $R_7$ en association avec $R_8$, et/ou $R_8$ en association avec $R_9$, et/ou $R_9$ en association avec $R_{10}$, forment respectivement avec $C_1$ et $C_2$, ou avec $C_2$ et $C_3$, ou avec $C_3$ et $C_4$, ou avec $C_4$, $C_{4a}$ et $C_5$, ou avec $C_7$ et $C_8$, ou avec $C_8$ et $C_9$, ou avec $C_9$ et $C_{10}$, un cycle aromatique ou aliphatique de 5 à 10 atomes de carbone, ce cycle étant le cas échéant substitué, notamment par un halogène, et/ou par un groupe alkyle, alkoxy, carbonyle, ou oxycarbonyle, et/ou par un cycle aromatique ou aliphatique, ces groupes ou cycles étant tels que définis ci-dessus, ou

. lorsque $R_3$ et $R_4$ ne représentent pas les différents atomes ou groupes ou cycles mentionnés ci-dessus, alors $R_3$ en association avec $R_4$ forment un groupe indole de formule

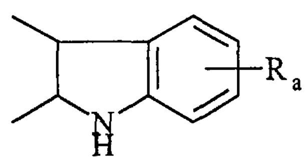

dans laquelle $R_a$ est tel que défini ci-dessus,

- Y représente :

. un groupe $-OR_d$, $R_d$ représentant un atome d'hydrogène, ou un groupe alkyle, carbonyle, ou oxycarbonyle, linéaire ou ramifié, ces groupes étant tels que définis ci-dessus, ou un cycle aromatique ou aliphatique, ces cycles étant tels que définis ci-dessus, ou

. un groupe $-NR_eR_f$ $R_e$ et $R_f$ indépendamment l'un de l'autre, représentant un groupe alkyle, alkoxy, carbonyle, ou oxycarbonyle, linéaire ou ramifié, ces groupes étant tels que définis ci-dessus, ou un cycle aromatique ou aliphatique, ces cycles étant tels que définis ci-dessus,

. étant entendu que lorsque $R_d$, ou l'un au moins de $R_e$ ou de $R_f$, ne représentent pas l'un des différents atomes ou groupes ou cycles mentionnés ci-dessus, alors $R_d$, ou l'un au moins de $R_e$ ou de $R_f$, en association avec $R_5$, ou en association avec $R_7$, forment respectivement avec $C_5$ et $C_6$, ou avec $C_6$, $C_{6a}$ et $C_7$, un hétérocycle aromatique ou aliphatique de 5 à 10 atomes de carbone, le cas échéant substitué, notamment par un halogène, et/ou par un groupe alkyle, alkoxy, carbonyle, ou oxycarbonyle, et/ou par un cycle aromatique ou aliphatique, ces groupes ou cycles étant tels que définis ci-dessus,

- X représente un atome sous forme anionique, tel qu'un atome d'halogène, notamment un atome de brome ou de chlore, ou un groupe d'atomes sous forme anionique, tel qu'un perchlorate, et l'azote de l'hétérocycle A de la formule (I) est sous forme quaternaire et est lié d'une part par liaison covalente au carbone en position 11, et, d'autre part, par liaison ionique à X défini ci-dessus,

* étant entendu que lorsque $R_1$ et $R_{10}$ ne représentent pas l'un des différents atomes ou groupes ou cycles mentionnés ci-dessus, alors $R_1$ en association avec $R_{10}$ forment avec $C_1$, l'azote de l'hétérocycle A de la formule (I), $C_{11}$, et $C_{10}$, un hétérocycle aromatique ou aliphatique de 5 à 10 atomes de carbone, le cas échéant substitué, notamment par un halogène, et/ou par un groupe alkyle, alkoxy, carbonyle, ou oxycarbonyle, et/ou par un cycle aromatique ou aliphatique, ces groupes ou cycles étant tels que définis ci-dessus,

pour la préparation de médicaments destinés au traitement de pathologies, notamment pulmonaires, digestives ou cardiaques, liées à des troubles des flux ioniques transmembranaires, notamment de chlore et, le cas échéant, de bicarbonate, dans l'organisme (humain ou animal), notamment pour la préparation de médicaments destinés au traitement de la mucoviscidose, ou à la prévention du rejet de drogues cytotoxiques (notamment antitumorales), ou au traitement des obstructions des voies bronchiques ou des voies digestives (notamment pancréatique ou intestinale)

**2.** Utilisation selon la revendication 1 de dérivés de formule générale (IIIa) suivante :

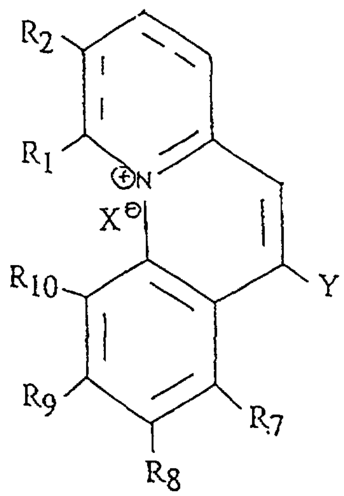

(IIIa)

dans laquelle :

- $R_1$ et $R_2$ représentent un atome d'hydrogène, ou forment en association avec $C_1$ et $C_2$ un cycle aromatique à 6 atomes de carbone,
- Y représente un groupe $-OH$ ou $-NH_2$, ou $-NHCOCH_3$,
- $R_7$, $R_8$, $R_9$ et $R_{10}$ représentent un atome d'hydrogène, ou l'un de $R_7$, $R_8$, $R_9$ ou $R_{10}$, représente un atome d'halogène, notamment un atome de chlore, de brome ou de fluor,
- X représente un atome d'halogène sous forme anionique, notamment un atome de brome $Br^-$, ou de chlore $Cl^-$, ou un groupe d'atomes sous forme anionique, notamment un perchlorate $ClO_4^-$.

**3.** Utilisation selon la revendication 1 ou la revendication 2, de composés de formule (IIIa) dans laquelle :

- $R_1$ et $R_2$ représentent un atome d'hydrogène,
- Y représente un groupe $-OH$,
- X représente un atome d'halogène sous forme anionique, notamment un atome de brome $Br^-$, ou de chlore $Cl^-$, ou un groupe d'atomes sous forme anionique, notamment un perchlorate $ClO_4^-$,
- $R_7$, $R_8$, $R_9$ et $R_{10}$ représentent indépendamment les uns des autres un atome d'hydrogène ou un atome d'halogène, notamment un atome de chlore, de brome ou de fluor.

**4.** Utilisation selon l'une des revendications 1 à 3, de dérivés des benzo[c]quinoliziniums de formule générale (IIIa) choisis parmi les suivants :

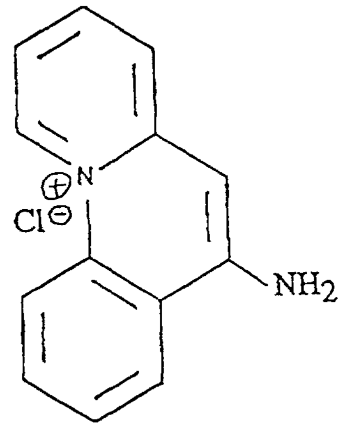

(composé 11 ou MPB-26)

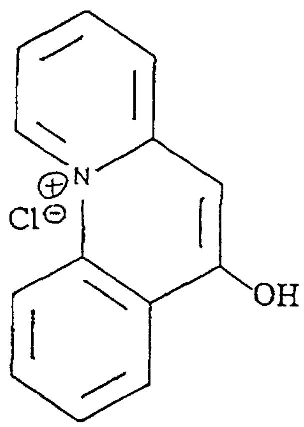

(composé 12 ou MPB-05)

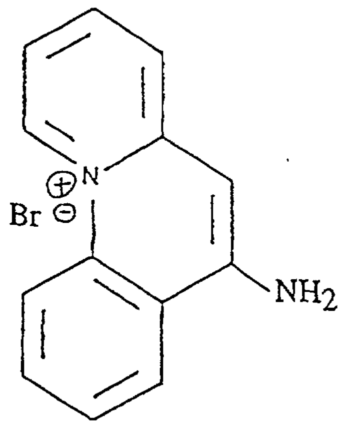

(composé 13 ou MPB-01)

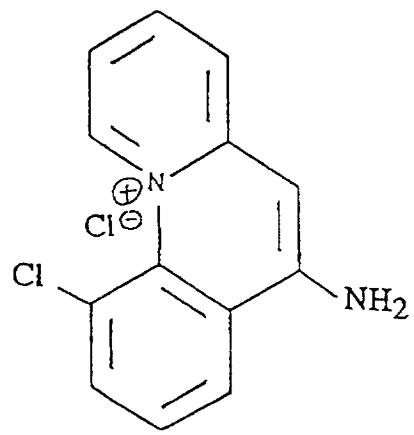

(composé 14 ou MPB-02)

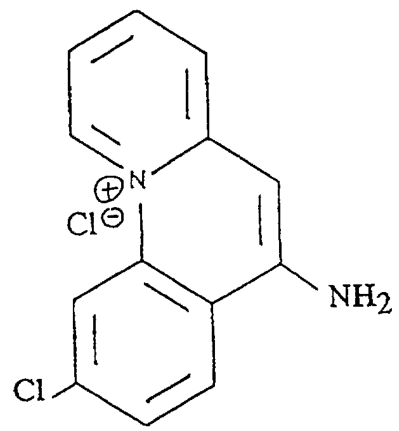

(composé 15 ou MPB-03)

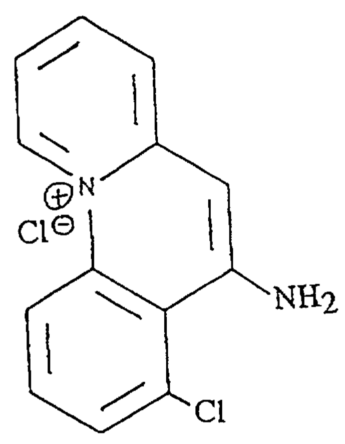

(composé 16)

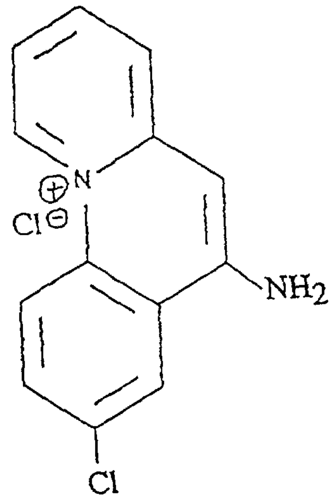

(composé 17)

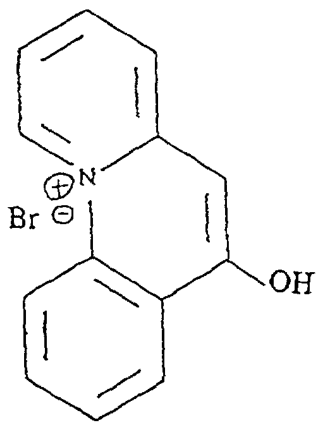

(composé 18 ou MPB-06)

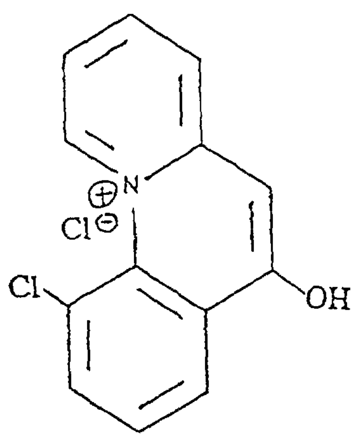

(composé 19 ou MPB-07)

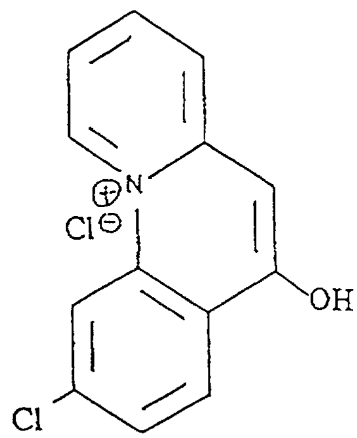

(composé 20 ou MPB-08)

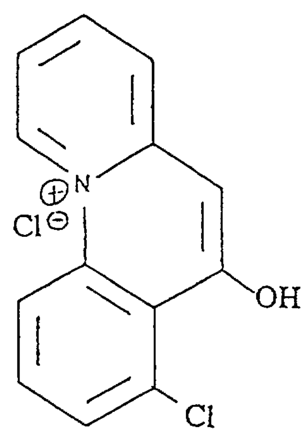

(composé 21 ou MPB-27)

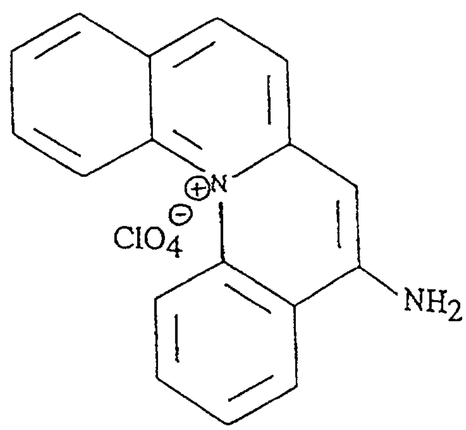

(composé 22)

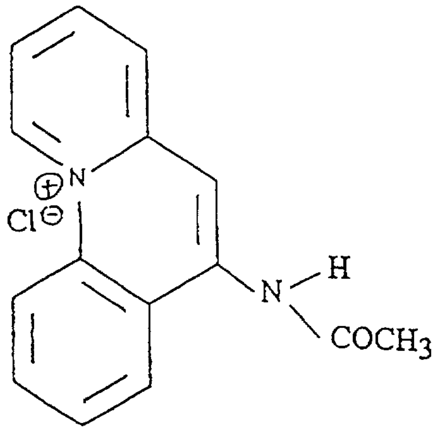

(composé 23)

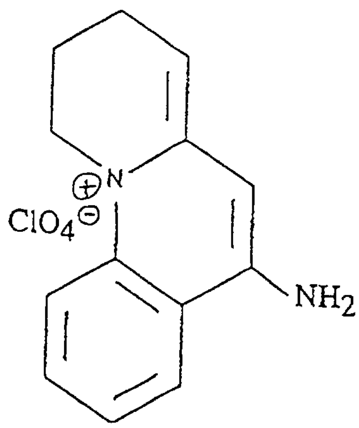

(composé 24)

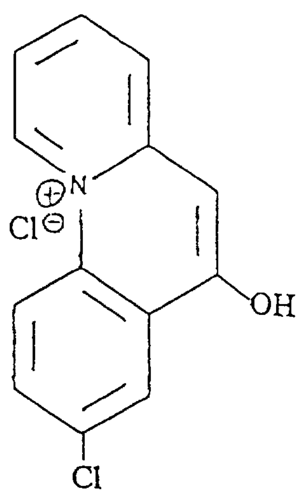

(composé 25 ou MPB-30)

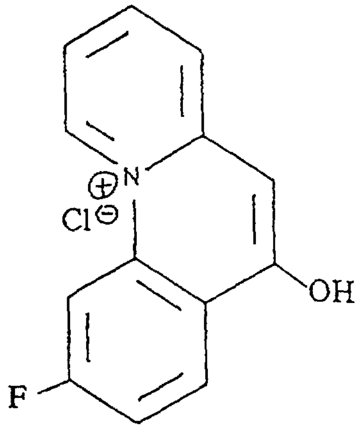

(composé 26 ou MPB-29)

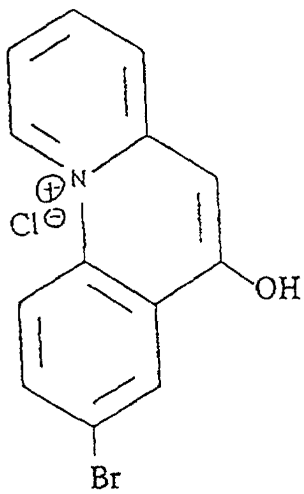

(composé 27 ou MPB-32)

**5.** Utilisation selon la revendication 1, de dérivés de formule générale (IIIb) suivante :

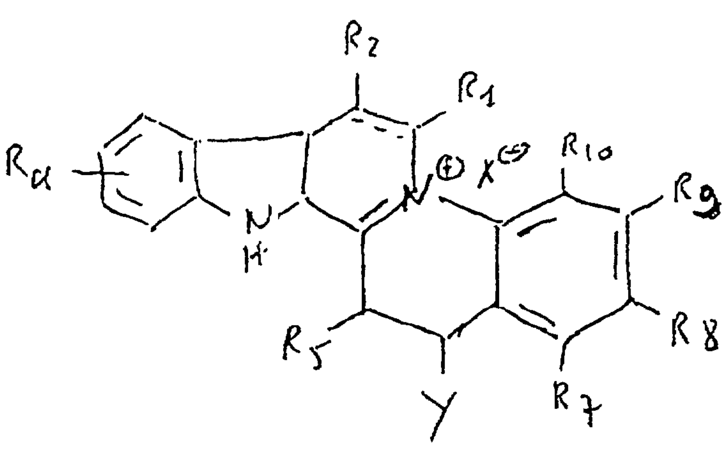

(IIIb)

dans laquelle :

- $R_a$ représente un atome d'hydrogène,
- $R_1$ et $R_2$ représentent un atome d'hydrogène, et il n'y a pas de double liaison entre les deux carbones portant $R_1$ et $R_2$,
- $R_5$ représente un atome d'hydrogène,
- $R_7$, $R_8$, $R_9$ et $R_{10}$ représentent un atome d'hydrogène, ou l'un de $R_7$, $R_8$, $R_9$ ou $R_{10}$ représente un atome d'halogène, notamment un atome de chlore, de brome ou de fluor,
- Y représente $-NH_2$,
- X représente un atome d'halogène sous forme anionique, notamment un atome de brome $Br^-$, ou de chlore $Cl^-$, ou un groupe d'atomes sous forme anionique, notamment un perchlorate $ClO_4^-$.

**6.** Utilisation selon la revendication 1, de dérivés de formule générale (IIIb) choisis parmi les suivants :

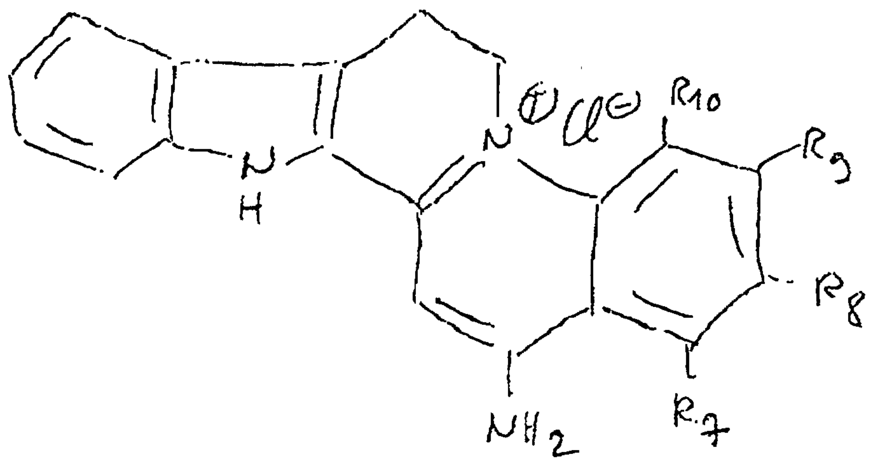

- composé G : $R_7$ = Cl, $R_8$ = $R_9$ = $R_{10}$ = H,
- composé H : $R_7$ = $R_8$ = $R_9$ = $R_{10}$ = H,
- composé I : $R_8$ = Cl, $R_7$ = $R_9$ = $R_{10}$ = H,
- composé J : $R_9$ = Cl, $R_7$ = $R_8$ = $R_{10}$ = H,
- composé K : $R_{10}$ = Cl, $R_7$ = $R_8$ = $R_9$ = H,
- composé L : $R_9$ = Br, $R_7$ = $R_8$ = $R_{10}$ = H.

**7.** Compositions pharmaceutiques **caractérisées en ce qu'**elles comprennent, à titre de principe(s) actif(s), au moins un des dérivés de formule générale (III) telle que définis dans l'une des revendications 1 à 6, en association avec un véhicule physiologiquement acceptable.

**8.** Compositions pharmaceutiques selon la revendication 7, **caractérisées en ce qu'**elles se présentent sous une forme administrable par voie orale, notamment sous forme de comprimés, ou de gélules, ou sous une forme administrable par voie parentérale, notamment sous forme de préparations injectables par voie intraveineuse, intramusculaire, ou sous-cutanée, ou encore par voie aérienne, notamment par voie pulmonaire sous forme d'aérosols.

**9.** Compositions pharmaceutiques selon la revendication 7 ou la revendication 8, **caractérisées en ce que** les quantités de principe(s) actif(s) sont telles que la posologie journalière en principe(s) actif(s) est d'environ 0,1 mg/kg à 5 mg/kg, notamment d'environ 3 mg/kg, en une ou plusieurs prises.

**10.** Dérivés des benzo[c]quinoliziniums de formule (III) suivante :

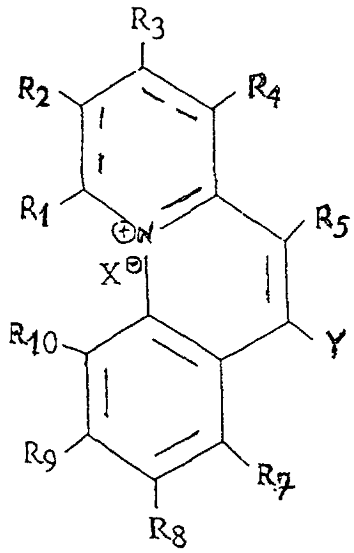

(III)

dans laquelle $R_1$, $R_2$, $R_3$, $R_4$, $R_5$, $R_7$, $R_8$, $R_9$, $R_{10}$, X et Y sont tels que définis dans la revendication 1, les composés de formule suivante étant exclus :

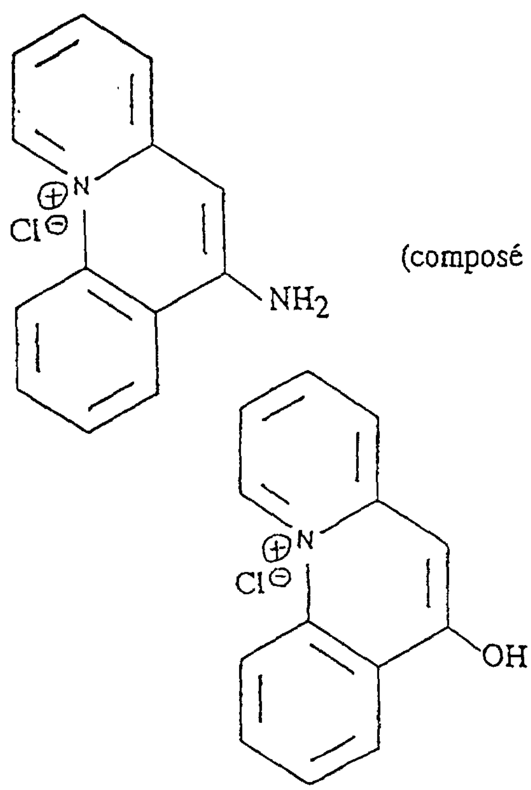

(composé 11 ou MPB-26)

(composé 12 ou MPB-05)

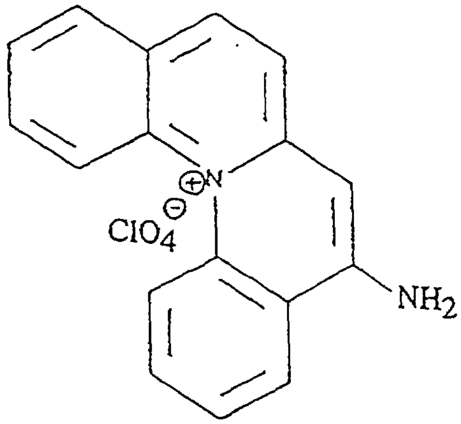

(composé 22)

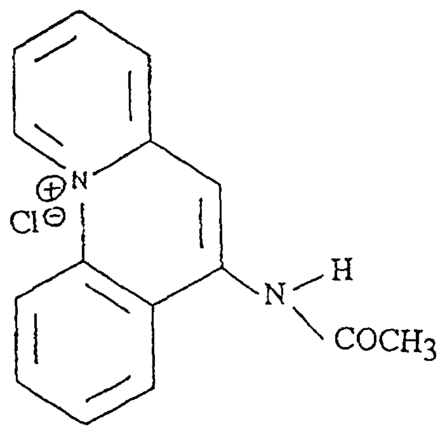

(composé 23)

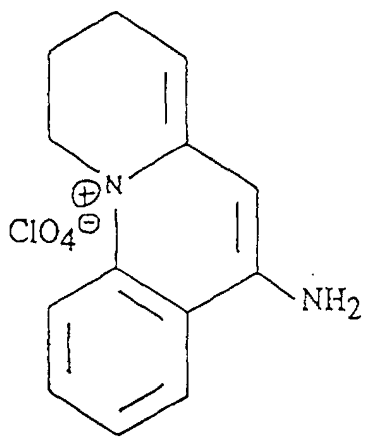

(composé 24)

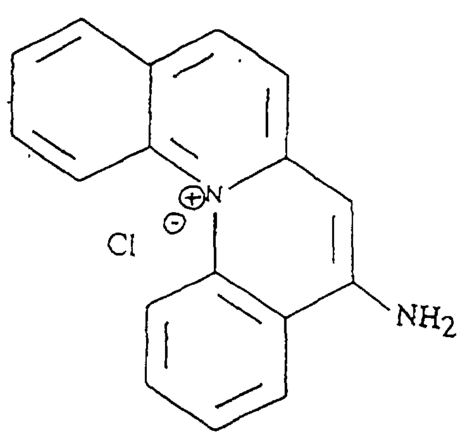

**11.** Dérivés selon la revendication 10, de formule générale (IIIa) suivante :

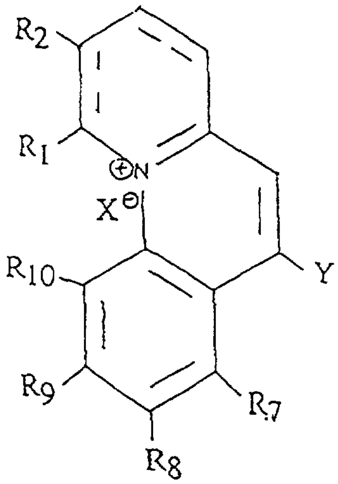

(IIIa)

dans laquelle :

- $R_1$ et $R_2$ représentent un atome d'hydrogène, ou forment en association avec $C_1$ et $C_2$ un cycle aromatique à 6 atomes de carbone,
- Y représente un groupe -OH ou $-NH_2$, ou $-NHCOCH_3$,
- $R_7$, $R_8$, $R_9$ et $R_{10}$ représentent un atome d'hydrogène, ou l'un de $R_7$, $R_8$, $R_9$ ou $R_{10}$, représente un atome d'halogène, notamment un atome de chlore. de brome ou de fluor,
- X représente un atome d'halogène sous forme anionique, notamment un atome de brome Br-, ou de chlore $Cl^-$, ou un groupe d'atomes sous forme anionique, notamment un perchlorate $ClO_4^-$.

**12.** Dérivés selon la revendication 11, de formule (IIIa) dans laquelle :

- $R_1$ et $R_2$ représentent un atome d'hydrogène,
- Y représente un groupe -OH,
- X représente un atome d'halogène sous forme anionique, notamment un atome de brome $Br^-$, ou de chlore $Cl^-$, ou un groupe d'atomes sous forme anionique, notamment un perchlorate $ClO_4^-$,
- $R_7$, $R_8$, $R_9$ et $R_{10}$ représentent indépendamment les uns des autres un atome d'hydrogène ou un atome d'halogène, notamment un atome de chlore, de brome ou de fluor.

**13.** Dérivés des benzo[c]quinoliziniums selon l'une des revendications 10 à 12, de formule générale (IIIa) choisis parmi les suivants :

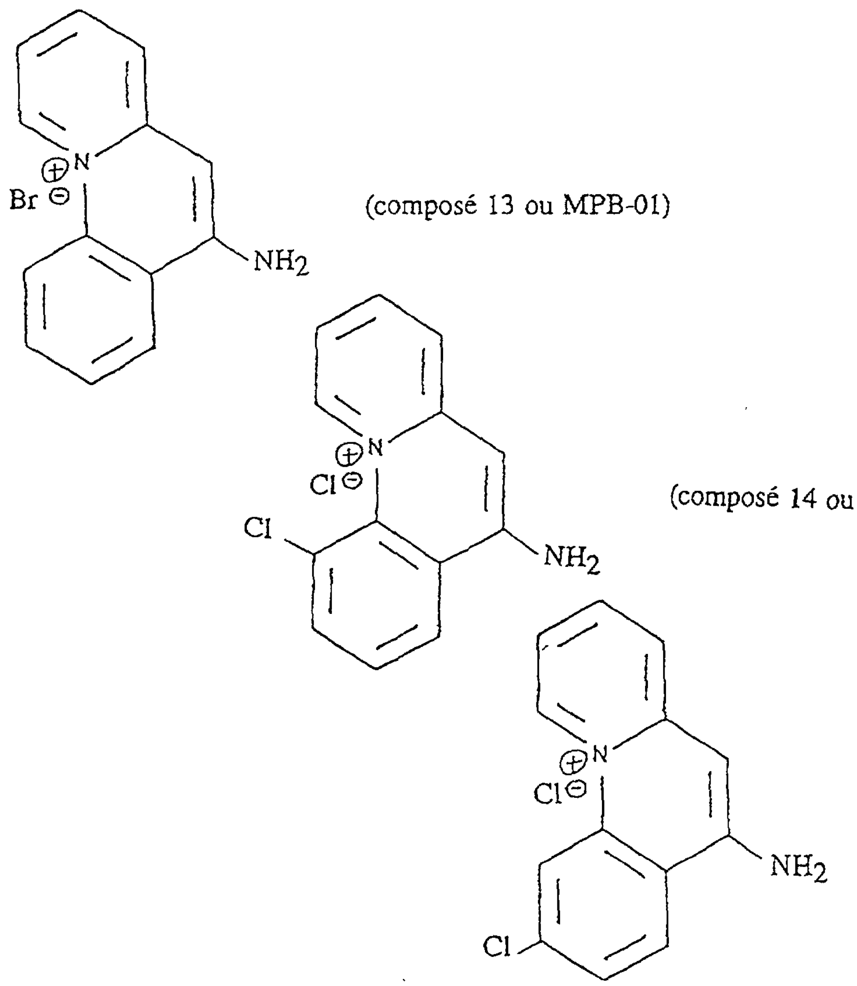

(composé 13 ou MPB-01)

(composé 14 ou MPB-02)

(composé 15 ou MPB-03)

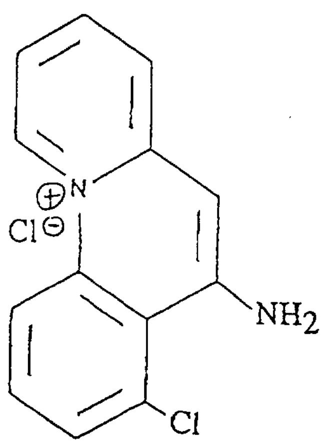

(composé 16)

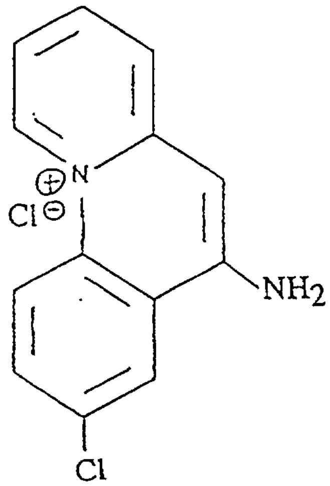

(composé 17)

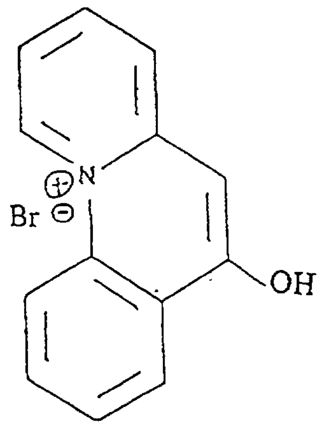

(composé 18 ou MPB-06)

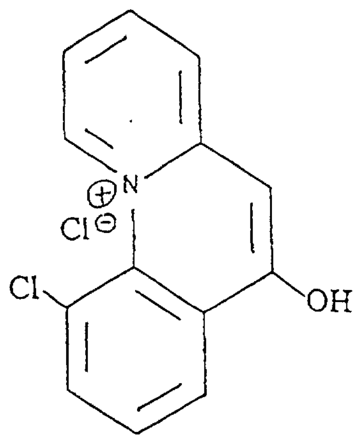

(composé 19 ou MPB-07)

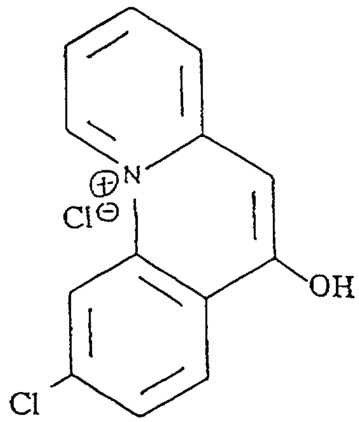

(composé 20 ou MPB-08)

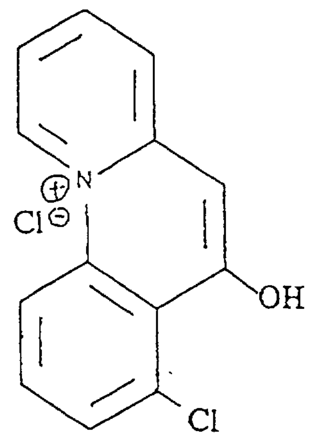

(composé 21 ou MPB-27)

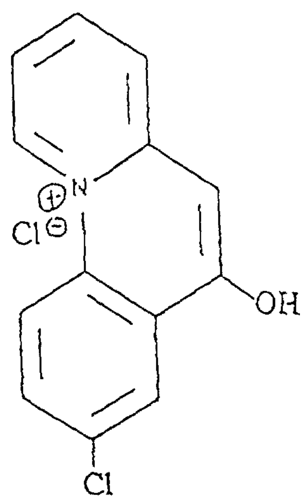

(composé 25 ou MPB-30)

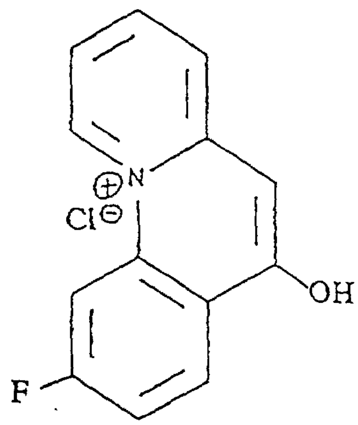

(composé 26 ou MPB-29)

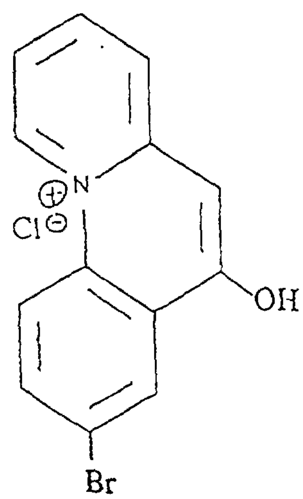

(composé 27 ou MPB-32)

**14.** Dérivés selon la revendication 10, de formule générale (IIIb) suivante :

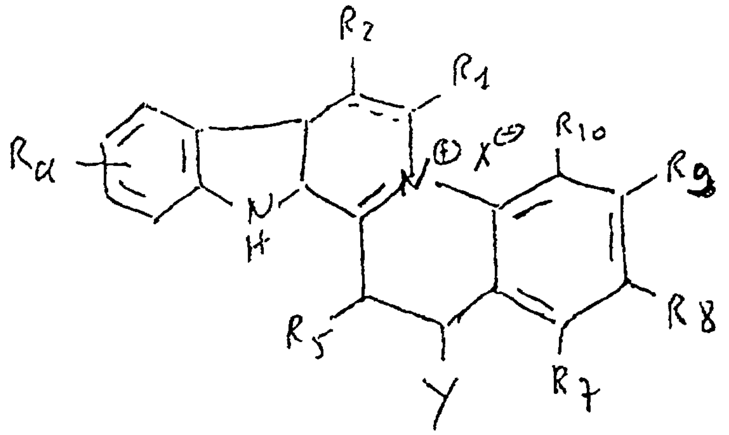

(IIIb)

dans laquelle $R_a$, $R_1$, $R_2$, $R_5$, $R_7$, $R_8$, $R_9$, $R_{10}$, X et Y sont tels que définis dans la revendication 1, et notamment

les composés de formule (IIIb) dans laquelle :

- $R_a$ représente un atome d'hydrogène,
- $R_1$ et $R_2$ représentent un atome d'hydrogène, et il n'y a pas de double liaison entre les deux carbones portant $R_1$ et $R_2$,
- $R_5$ représente un atome d'hydrogène,
- $R_7$, $R_8$, $R_9$ et $R_{10}$ représentent un atome d'hydrogène, ou l'un de $R_7$, $R_8$, $R_9$ ou $R_{10}$ représente un atome d'halogène, notamment un atome de chlore, de brome ou de fluor,
- Y représente $-NH_2$,
- X représente un atome d'halogène sous forme anionique, notamment un atome de brome $Br^-$, ou de chlore $Cl^-$, ou un groupe d'atomes sous forme anionique, notamment un perchlorate $ClO_4^-$.

**15.** Dérivés selon la revendication 14, de formule générale (IIIb) choisis parmi les suivants :

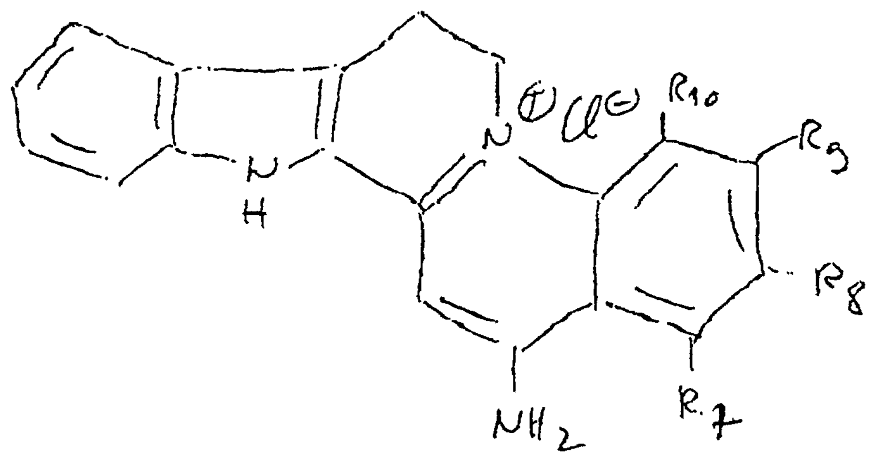

- composé G : $R_7$ = Cl, $R_8$ = $R_9$ = $R_{10}$ = H,
- composé H : $R_7$ = $R_8$ = $R_9$ = $R_{10}$ = H,
- composé I : $R_8$ = Cl, $R_7$ = $R_9$ = $R_{10}$ = H,
- composé J : $R_9$ = Cl, $R_7$ = $R_8$ = $R_{10}$ = H,
- composé K : $R_{10}$ = Cl, $R_7$ = $R_8$ = $R_9$ = H,
- composé L : $R_9$ = Br, $R_7$ = $R_8$ = $R_{10}$ = H.

**16.** Procédé de préparation de dérivés tels que définis dans la revendication 1, **caractérisé en ce qu'**il comprend les étapes suivantes :

- traitement du dérivé de formule (A) dans laquelle $R_1$, $R_2$, $R_3$, $R_4$, et $R_5$, sont tels que définis dans la revendication 1, par le phényllithium ou le diisopropyl amidure de lithium, avantageusement dans l'éther ou le THF, ce qui conduit à l'obtention de dérivés de formule (B) dans laquelle $R_1$, $R_2$, $R_3$, $R_4$, et $R_5$, sont tels que définis dans la revendication 1, selon le schéma réactionnel suivant:

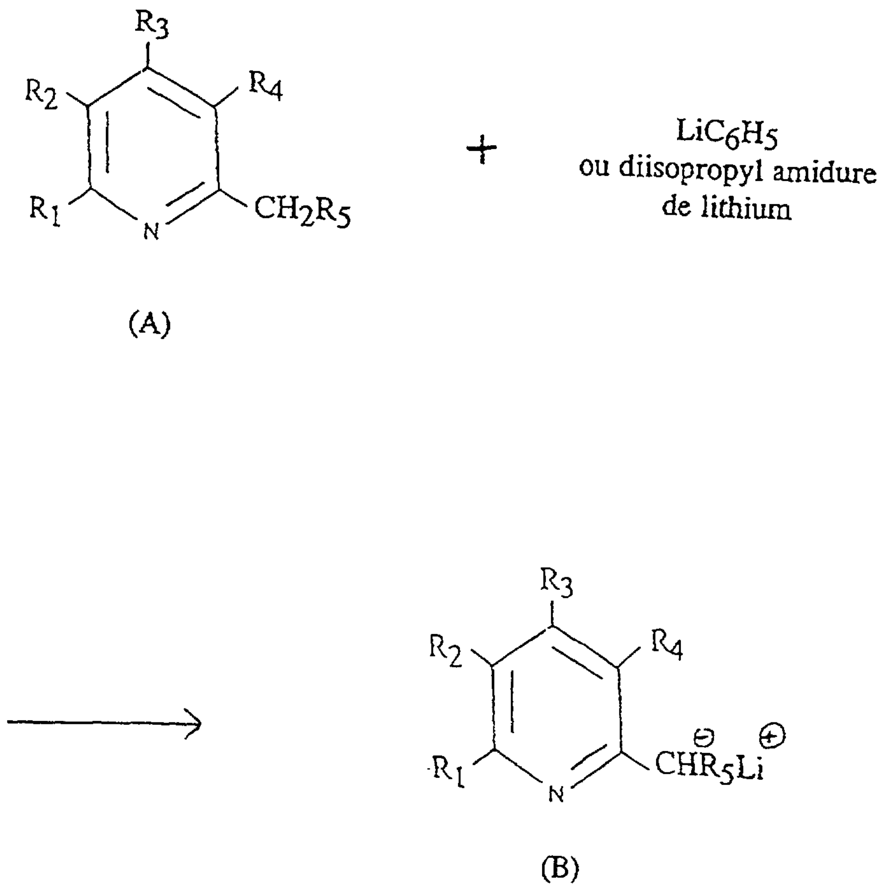


(A)

(B)

- condensation du dérivé de formule (B) obtenu à l'étape précédente avec le dérivé de formule (C) dans laquelle $R_7$, $R_8$, $R_9$, $R_{10}$, et X sont tels que définis dans la formule (I), ce qui conduit à l'obtention de dérivés de formule (D) dans laquelle $R_1$, $R_2$, $R_3$, $R_4$, $R_5$, $R_7$, $R_8$, $R_9$, $R_{10}$, et X sont tels que définis dans la revendication 1, selon le schéma réactionnel suivant :

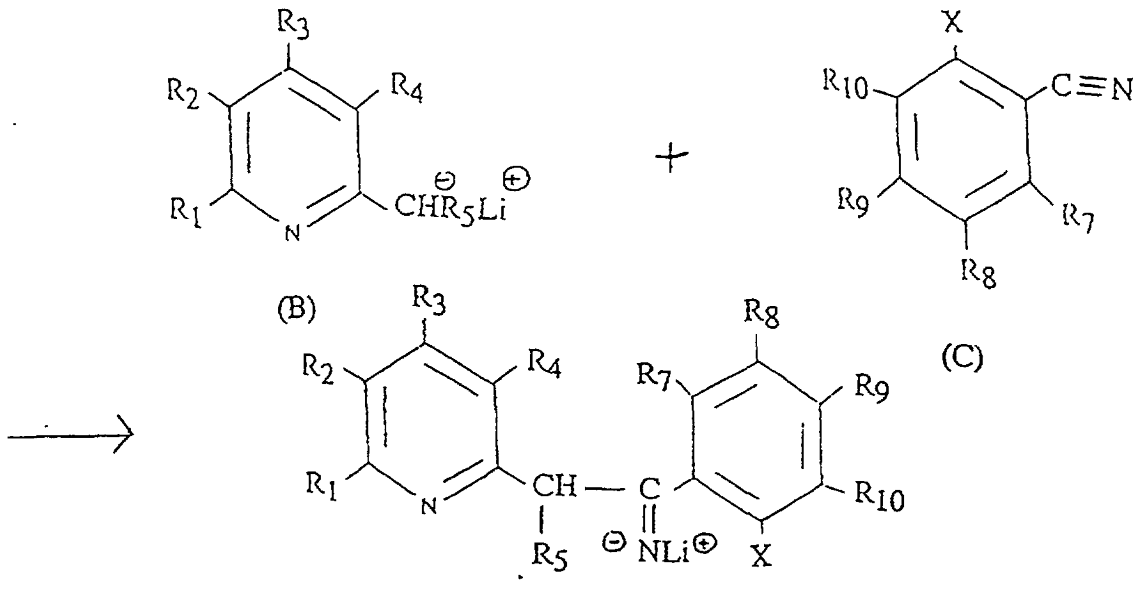

(B)

(C)

(D)

- traitement du composé de formule (D) par addition d'$H_2O$, ce qui conduit à l'obtention du dérivé de formule (II) suivante, dans laquelle $R_1$ à $R_5$, $R_7$ à $R_{10}$, et X sont tels que définis dans la revendication 1, et Y représente $-NH_2$,

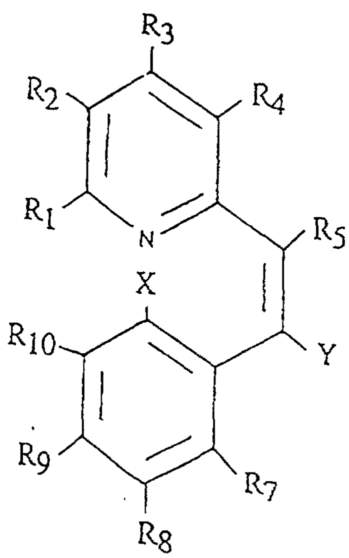

- le cas échéant, traitement du composé de formule (II) susmentionnée, par un dérivé comportant les groupes $R_e$ et $R_f$ tels que définis dans la revendication 1, ce dérivé étant susceptible de réagir avec l'atome d'azote lié au carbone en position 6 du composé de formule (II) susmentionnée, notamment par un halogénure de $R_e$ et/ou de $R_f$ tout en ayant, si nécessaire, pris soin de protéger au préalable celles des autres fonctions présentes sur le composé de formule (II) susmentionnée et susceptibles de réagir avec le dérivé comportant les groupes $R_e$ et $R_f$ susmentionnés, ce qui conduit à l'obtention du composé de formule (II) suivante dans laquelle $R_1$ à $R_5$, $R_7$ à $R_{10}$, et X sont tels que définis ci-dessus, et Y représente un groupe $-NR_eR_f$ tel que défini dans la revendication 1,

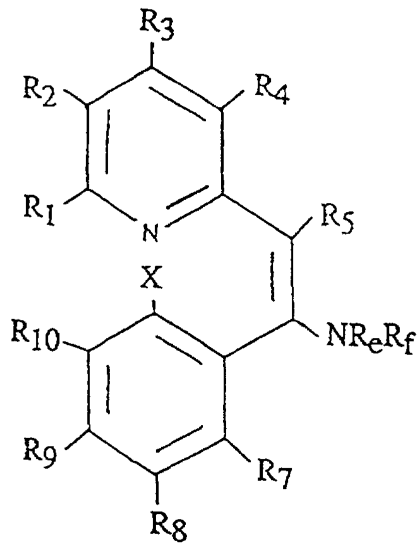

- le cas échéant, hydrolyse, notamment par action de l'acide sulfurique (pH3) à 40°C, du composé de formule (II) susmentionnée dans laquelle Y représente $NH_2$, ce qui conduit à l'obtention du composé de formule (II) suivante, dans laquelle $R_1$ à $R_5$, $R_7$ à $R_{10}$, et X sont tels que définis dans 11a revendication 1, et Y représente un groupe -OH,

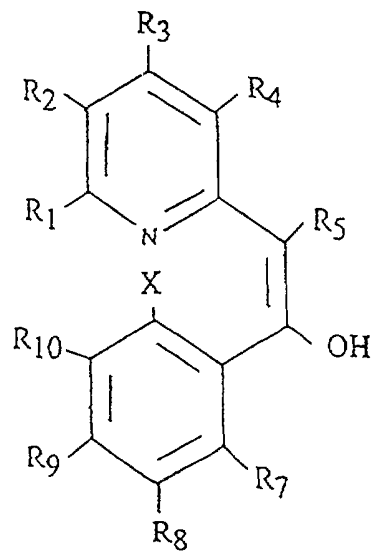

- le cas échéant, traitement du composé de formule (II) susmentionnée, dans laquelle Y représente un groupe -OH, par un dérivé comportant le groupe $R_d$, tel que défini dans la revendication 1, ce dérivé étant susceptible de réagir avec l'atome d'oxygène lié au carbone en position 7 du composé de formule (II) susmentionnée, notamment par un halogénure de $R_d$, tout en ayant, si nécessaire, pris soin de protéger au préalable celles des autres fonctions présentes sur le composé de formule (II) susmentionnée et susceptibles de réagir avec le dérivé comportant le groupe $R_d$ susmentionné, ce qui conduit à l'obtention du composé de formule (II) suivante dans laquelle $R_1$ à $R_5$, $R_7$ à $R_{10}$, et X sont tels que définis ci-dessus, et Y représente un groupe $-OR_d$ tel que défini dans la revendication 1,

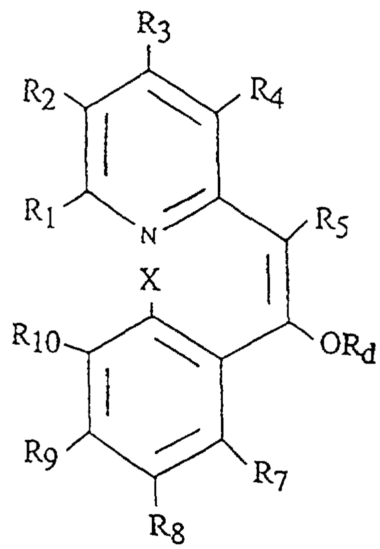

- chauffage, avantageusement à 200°C, des composés de formule (II) susmentionnée dans laquelle Y représente $-NH_2$ ou -OH, ce qui conduit respectivement aux composés de formules (III) suivantes, dans laquelle $R_1$ à $R_5$, $R_7$ à $R_{10}$, et X sont tels que définis dans la revendication 1, et Y représente un groupe $-NH_2$ ou -OH,

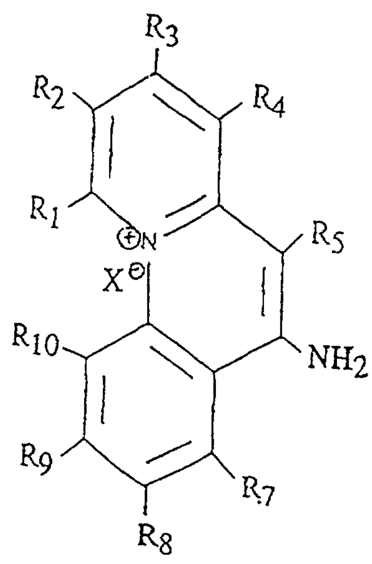

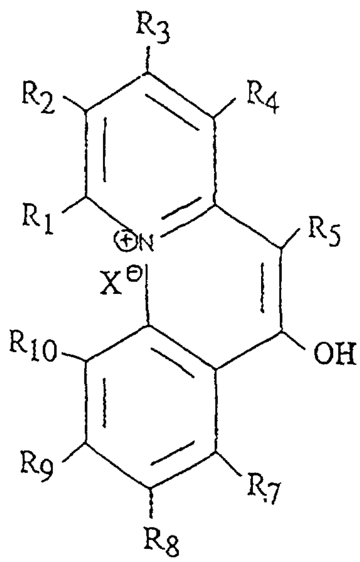

- le cas échéant, traitement du composé de formule (III) susmentionnée, dans laquelle Y représente un groupe $-NH_2$, par un dérivé comportant les groupes $R_e$ et $R_f$ tels que définis dans la revendication 1, ce dérivé étant susceptible de réagir avec l'atome d'azote lié au carbone en position 6 du composé de formule (III) susmentionnée, notamment par un halogénure de $R_e$ et/ou de $R_f$, tout en ayant, si nécessaire, pris soin de protéger au préalable celles des autres fonctions présentes sur le composé de formule (III) susmentionnée et susceptibles de réagir avec le dérivé comportant les groupes $R_e$ et $R_f$ susmentionnés, ce qui conduit à l'obtention du composé de formule (III) suivante dans laquelle $R_1$ à $R_5$, $R_7$ à $R_{10}$, et X sont tels que définis ci-dessus, et Y représente un groupe $-NR_eR_f$ tel que défini dans la revendication 1,

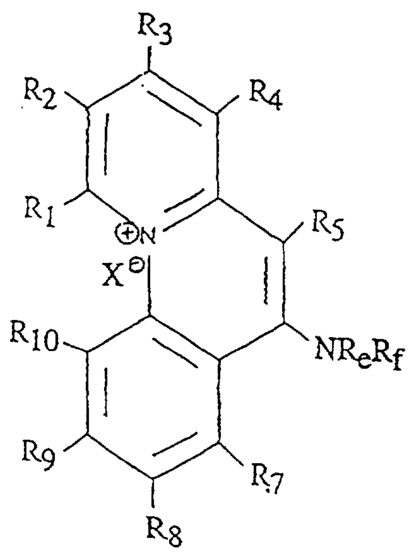

- le cas échéant, traitement du composé de formule (III) susmentionnée, dans laquelle Y représente un groupe -OH, par un dérivé comportant le groupe $R_d$, tel que défini dans la revendication 1, ce dérivé étant susceptible de réagir avec l'atome d'oxygène lié au carbone en position 7 du composé de formule (III) susmentionnée, notamment par un halogénure de $R_d$, tout en ayant, si nécessaire, pris soin de protéger au préalable celles des autres fonctions présentes sur le composé de formule (III) susmentionnée et susceptibles de réagir avec le dérivé comportant le groupe $R_d$ susmentionné, ce qui conduit à l'obtention du composé de formule (III) suivante dans laquelle $R_1$ à $R_5$, $R_7$ à $R_{10}$, et X sont tels que définis ci-dessus, et Y représente un groupe $-OR_d$ tel que défini dans la revendication 1,

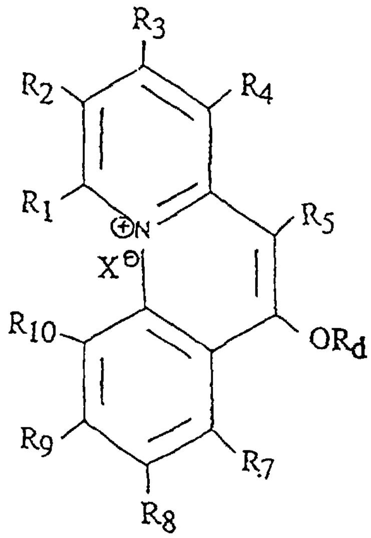

- le cas échéant, hydrogénation des composés de formules (II) ou (III) susmentionnées, notamment par hydrogénation catalytique en présence d'oxyde de platine à pression réduite, ce qui conduit à l'obtention des composés de formules (II) ou (III) suivantes :

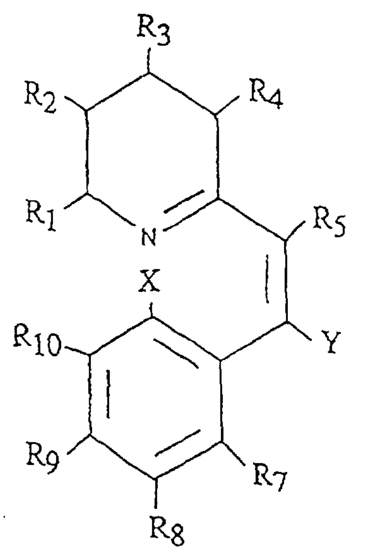

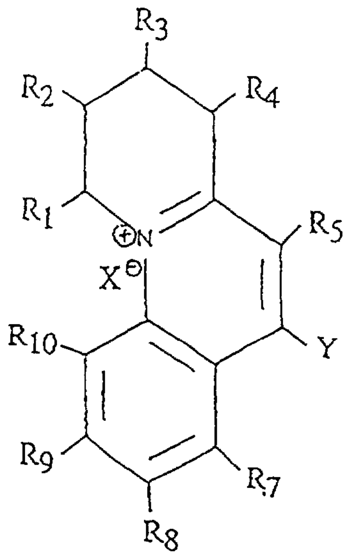

dans lesquelles $R_1$ à $R_5$, $R_7$ à $R_{10}$, X et Y sont tels que définis ci-dessus.

## Claims

**1.** Use of benzo[c]quinolizinium derivatives of general formula (III) which follows:

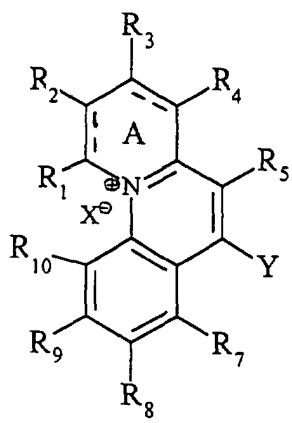

**(III)**

in which:

- heterocycle A is aromatic or non-aromatic, it being understood that in the latter case the nitrogen atom of this heterocycle is linked by a double bond to the carbon in position 4a,
- $R_1$, $R_2$, $R_3$, $R_4$, $R_5$, $R_7$, $R_8$, $R_9$ and $R_{10}$, represent, independently of each other:

  . a hydrogen, or bromine or fluorine atom, or
  . a halogen atom, in particular a chlorine atom, or
  . an alkyl, alkoxy, carbonyl or oxycarbonyl group, linear or branched, with 1 to 10 carbon atoms, if appropriate these groups being substituted, in particular by a halogen, and/or by a hydroxyl, and/or by an amine (primary, secondary or tertiary), and/or by an aromatic and/or aliphatic cycle, with 5 to 10 carbon atoms in the cycle, these cycles being themselves, if appropriate, substituted in particular by a halogen, and/or by a hydroxyl, and/or by an amine (primary, secondary or tertiary), and/or by an alkyl, alkoxy, carbonyl or oxycarbonyl group, these groups being as defined above, or
  . an aromatic or aliphatic cycle, with 5 to 10 carbon atoms in the cycle, this cycle being itself, if appropriate, substituted in particular by a halogen, and/or by a hydroxyl, and/or by an amine (primary, secondary or

tertiary), and/or by an alkyl, alkoxy, carbonyl or oxycarbonyl group, these groups being as defined above, or

. an $-OR_a$ group, $R_a$ representing a hydrogen atom or an alkyl, carbonyl or oxycarbonyl group, linear or branched, these groups being as defined above, or an aromatic or aliphatic cycle, these cycles being as defined above, or

. when $R_1$ and $R_2$, or $R_3$ and $R_4$, and/or $R_4$ and $R_5$, and/or $R_7$ and $R_8$, and/or $R_8$ and $R_9$, and/or $R_9$ and $R_{10}$, do not represent the different atoms or groups or cycles mentioned above, then $R_1$ in combination with $R_2$, or $R_2$ in combination with $R_3$, and/or $R_3$ in combination with $R_4$, and/or $R_4$ in combination with $R_5$, and/or $R_7$ in combination with $R_8$, and/or $R_8$ in combination with $R_9$, and/or $R_9$ in combination with $R_{10}$, form respectively with $C_1$ and $C_2$, or with $C_2$ and $C_3$, or with $C_3$ and $C_4$, or with $C_4$, $C_{4a}$ and $C_5$, or with $C_7$ and $C_8$, or with $C_8$ and $C_9$, or with $C_9$ and $C_{10}$, an aromatic or aliphatic cycle, with 5 to 10 carbon atoms, if appropriate this cycle being substituted, in particular by a halogen, and/or by an alkyl, alkoxy, carbonyl or oxycarbonyl group, and/or an aromatic or aliphatic cycle, these groups or cycles being as defined above, or

. when $R_3$ and $R_4$ do not represent the different atoms or groups or cycles mentioned above, then $R_3$ in combination with $R_4$ form an indole group of formula

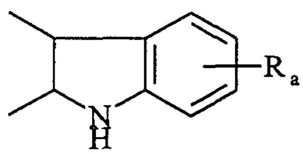

in which $R_a$ is as defined above,

- Y represents:

. an $-OR_d$ group, $R_d$ representing a hydrogen atom or an alkyl, carbonyl or oxycarbonyl group, linear or branched, these groups being as defined above, or an aromatic or aliphatic cycle, these cycles being as defined above, or

. an $NR_eR_f$ group, $R_e$ and $R_f$ independently of each other, representing an alkyl, alkoxy, carbonyl or oxycarbonyl group, linear or branched, these groups being as defined above, or an aromatic or aliphatic cycle, these cycles being as defined above,

. it being understood that when $R_d$, or at least one of $R_e$ and $R_f$ do not represent one of the different atoms or groups or cycles mentioned above, then $R_d$, or at least one of $R_e$ and $R_f$, in combination with $R_5$, or in combination with $R_7$, form respectively with $C_5$ or $C_6$, or with $C_6$, $C_{6a}$ or $C_7$, an aromatic or aliphatic heterocycle with 5 to 10 carbon atoms, if appropriate substituted, in particular by a halogen, and/or an alkyl, alkoxy, carbonyl or oxycarbonyl group, and/or an aromatic or aliphatic cycle, these groups or cycles being as defined above,

- X represents an atom in anionic form, such as a halogen atom, in particular a bromine or chlorine atom, or a group of atoms in anionic form, such as a perchlorate, and the nitrogen of heterocycle A of formula (I) is in quaternary form and is linked on the one hand by a covalent bond to the carbon in position 11, and, on the other hand, by an ionic bond to X defined above,

* it being understood that when $R_1$ and $R_{10}$ do not represent one of the different atoms or groups or cycles mentioned above, then $R_1$ in combination with $R_{10}$ form with $C_1$, the nitrogen of heterocycle A of formula (I), $C_{11}$, and $C_{10}$, an aromatic or aliphatic heterocycle with 5 to 10 carbon atoms, if appropriate substituted, in particular by a halogen, and/or an alkyl, alkoxy, carbonyl or oxycarbonyl group, and/or by an aromatic or aliphatic cycle, these groups or cycles being as defined above,

for the preparation of medicaments intended for the treatment of pathologies, in particular pulmonary, digestive or cardiac, linked to transmembrane ionic flux disorders, in particular chlorine and, if appropriate, bicarbonate, in the organism (human or animal), in particular for the preparation of medicaments intended for the treatment of mucoviscidosis, or for the prevention of rejection of cytotoxic drugs (in particular antitumoral), for the treatment of obstructions to the bronchial routes or digestive tracts (in particular pancreatic or intestinal).

2. Use according to claim 1 of derivatives of general formula (IIIa) which follows:

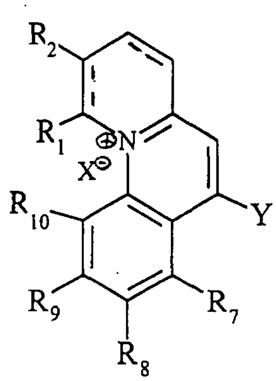

(IIIa)

in which:

- $R_1$ and $R_2$ represent a hydrogen atom, or form in combination with $C_1$ and $C_2$ an aromatic cycle with 6 carbon atoms,
- Y represents an -OH or -$NH_2$ or -$NHCOCH_3$ group,
- $R_7$, $R_8$, $R_9$ and $R_{10}$, represent a hydrogen atom or one of $R_7$, $R_8$, $R_9$ and $R_{10}$ represents a halogen atom, in particular a chlorine, bromine or fluorine atom,
- X represents a halogen atom in anionic form, in particular a bromine atom $Br^-$, or a chlorine atom $Cl^-$, or a group of atoms in anionic form, in particular a perchlorate $ClO_4^-$.

**3.** Use according to claim 1 or to claim 2, of compounds of formula (IIIa) in which:

- $R_1$ and $R_2$ represent a hydrogen atom,
- Y represents an -OH group,
- X represents a halogen atom in anionic form, in particular a bromine atom Br, or a chlorine atom $Cl^-$, or a group of atoms in anionic form, in particular a perchlorate $ClO_4^-$,
- $R_7$, $R_8$, $R_9$ and $R_{10}$, represent independently of each other a hydrogen atom or a halogen atom, in particular a chlorine, bromine or fluorine atom.

**4.** Use according to one of claims 1 to 3, of benzo[c]quinolizinium derivatives of general formula (IIIa) chosen from the following:

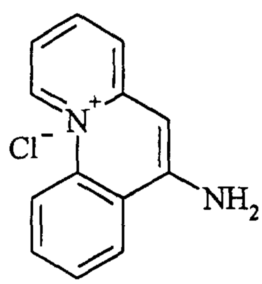

(compound 11 or MPB-26)

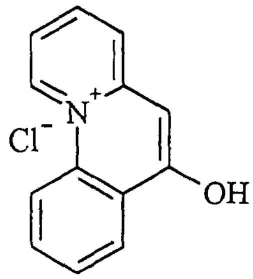

(compound 12 or MPB-05)

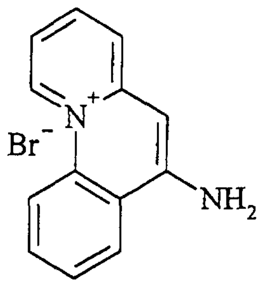

(compound 13 or MPB-01)

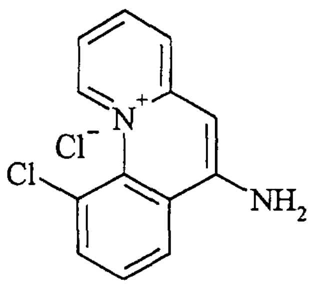

(compound 14 or MPB-02)

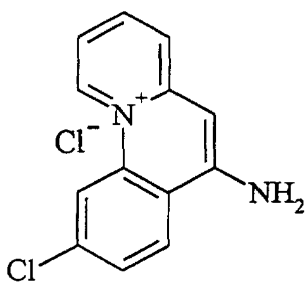

(compound 15 or MPB-03)

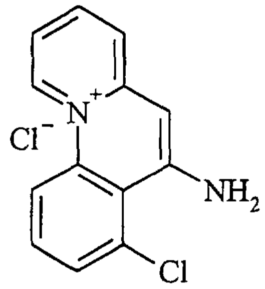

(compound 16)

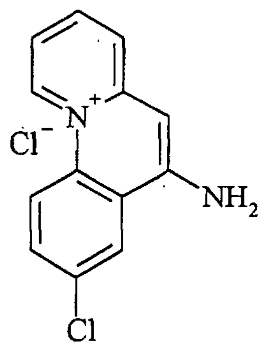

(compound 17)

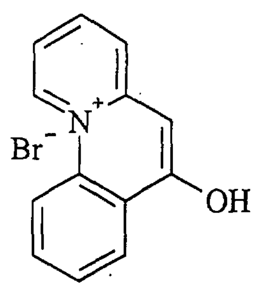

(compound 18 or MPB-06)

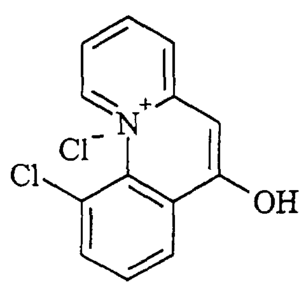

(compound 19 or MPB-07)

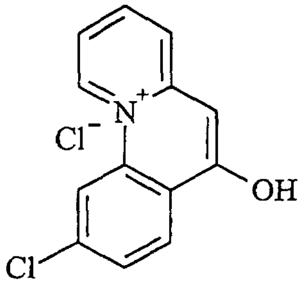

(compound 20 or MPB-08)

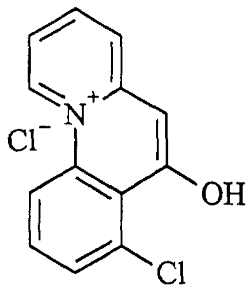

(compound 21 or MPB-27)

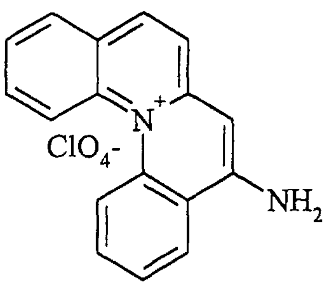

(compound 22)

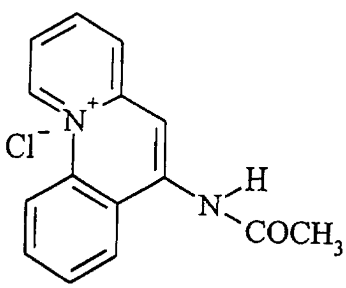

(compound 23)

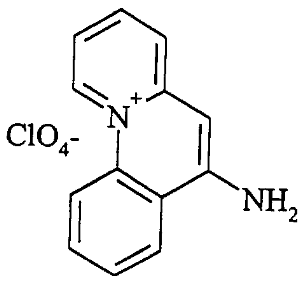

(compound 24)

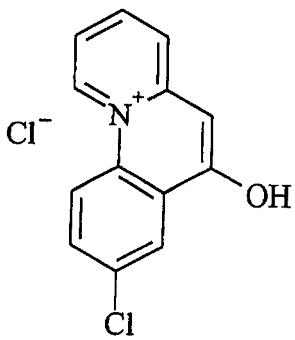

(compound 25 or MPB-30)

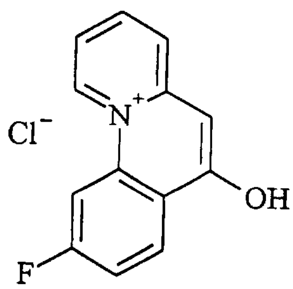

(compound 26 or MPB-29)

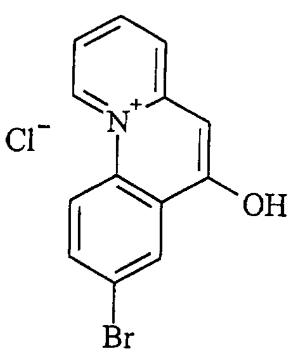

(compound 27 or MPB-32)

**5.** Use according to claim 1 of derivatives of general formula (IIIb) which follows:

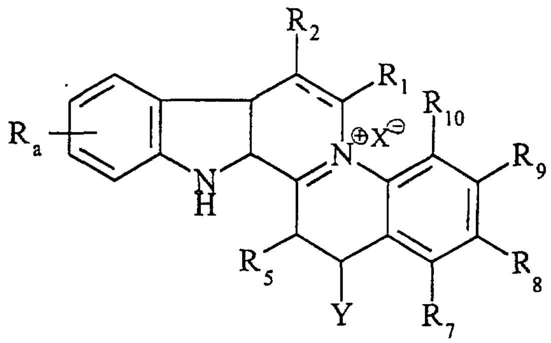

(IIIb)

in which:

- $R_a$ represents a hydrogen atom,
- $R_1$ and $R_2$ represent a hydrogen atom, and there is no double bond between the two carbons carrying $R_1$ and $R_{2,}$
- $R_5$ represents a hydrogen atom,
- $R_7$, $R_8$, $R_9$, and $R_{10}$ represent a hydrogen atom, or one of $R_7$, $R_8$, $R_9$, and $R_{10}$ represents a halogen atom, in particular a chlorine, bromine or fluorine atom,
- Y represents $-NH_2$,
- X represents a halogen atom in anionic form, in particular a bromine atom $Br^-$, or a chlorine atom $Cl^-$, or a group of atoms in anionic form, in particular a perchlorate $ClO_4^-$.

**6.** Use according to claim 1, of derivatives of general formula (IIIb) chosen from the following:

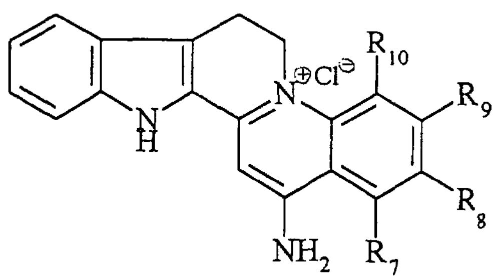

- compound G: $R_7$ = Cl, $R_8 = R_9 = R_{10}$ = H,
- compound H: $R_7 = R_8 = R_9 = R_{10}$ = H,
- compound I: $R_8$ = Cl, $R_7 = R_9 = R_{10}$ = H,
- compound J: $R_9$ = Cl, $R_7 = R_8 = K_{10}$= H,
- compound K: $R_{10}$ = Cl, $R_7 = R_8 = R_9$ = H,
- compound L: $R_9$ = Br, $R_7 = R_8 = R_{10}$ = H.

**7.** Pharmaceutical compositions **characterized in that** they contain, as active ingredient(s), at least one of the derivatives of general formula (III) as defined in one of claims 1 to 6, in association with a physiologically acceptable vehicle.

**8.** Pharmaceutical compositions according to claim 7, **characterized in that** they are presented in a form which can be administered by oral route, in particular in the form of tablets or capsules, or in a form which can be administered by parenteral route, in particular in the form of preparations which are injectable by intravenous, intramuscular or sub-cutaneous route, or also via the airways, in particular by pulmonary route in the form of aerosols.

**9.** Pharmaceutical compositions according to claim 7 or to claim 8, **characterized in that** the quantities of active ingredient(s) are such that the daily dose of active ingredient(s) is approximately 0.1 mg/kg to 5 mg/kg, in particular approximately 3 mg/kg, in one or more doses.

**10.** Benzo[c]quinolizinium derivatives of formula (III) which follows:

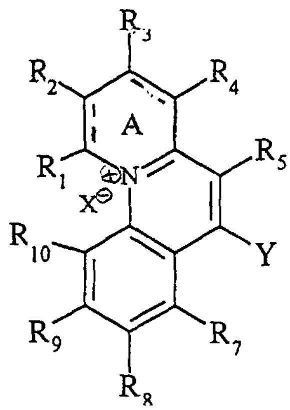

(III)

in which $R_1$, $R_2$, $R_3$, $R_4$ $R_5$, $R_7$, $R_8$, $R_9$, $R_{10}$, X and Y are as defined in claim 1, the compounds of the following formula being excluded:

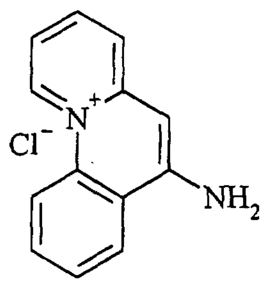

(compound 11 or MPB-26)

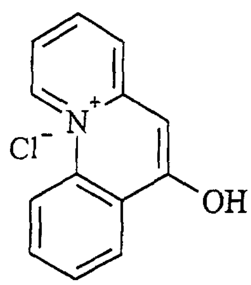

(compound 12 or MPB-05)

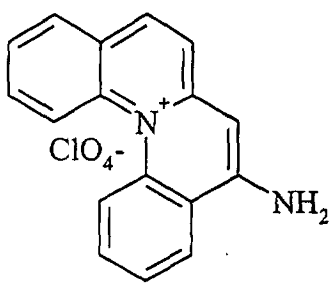

(compound 22 )

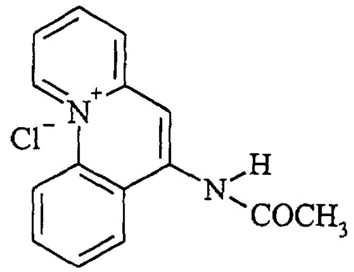

(compound 23 )

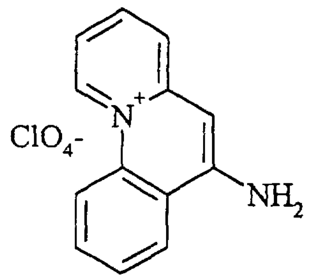

(compound 24)

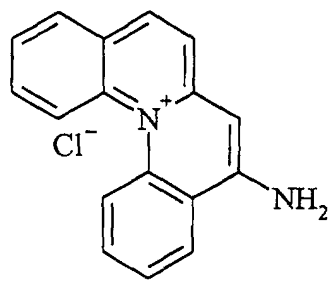

**11.** Derivatives according to claim 10, of general formula (IIIa) which follows:

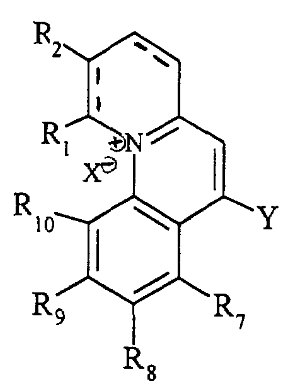

**(IIIa)**

in which:

- $R_1$ and $R_2$ represent a hydrogen atom, or form in combination with $C_1$ and $C_2$ an aromatic cycle with 6 carbon atoms,
- Y represents an -OH or $-NH_2$ or $NHCOCH_3$ group,
- $R_7$, $R_8$, $R_9$ and $R_{10}$, represent a hydrogen atom or one of $R_7$, $R_8$, $R_9$ or $R_{10}$ represents a halogen atom, in particular a chlorine, bromine or fluorine atom,
- X represents a halogen atom in anionic form, in particular a bromine atom $Br^-$, or a chlorine atom $Cl^-$, or a group of atoms in anionic form, in particular a perchlorate $ClO_4^-$.

**12.** Derivatives according to claim 11, of formula (IIIa) in which:

- $R_1$ and $R_2$ represent a hydrogen atom,
- Y represents an -OH group,
- X represents a halogen atom in anionic form, in particular a bromine atom $Br^-$, or a chlorine atom $Cl^-$, or a group of atoms in anionic form, in particular a perchlorate $ClO_4^-$,
- $R_7$, $R_8$, $R_9$ and $R_{10}$, represent independently of each other a hydrogen atom or a halogen atom, in particular a chlorine, bromine or fluorine atom.

**13.** Benzo[c]quinolizinium derivatives according to one of claims 10 to 12, of general formula (IIIa) chosen from the following:

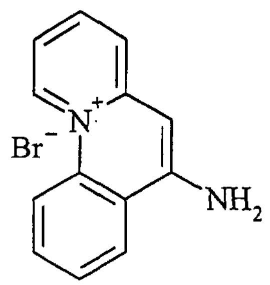

(compound 13 or MPB-01)

7

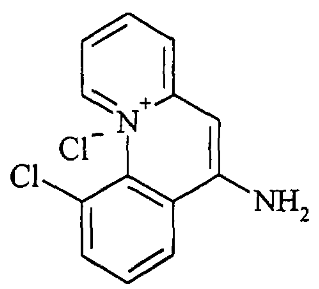

(compound 14 or MPB-02)

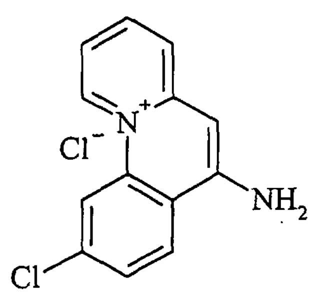

(compound 15 or MPB-03)

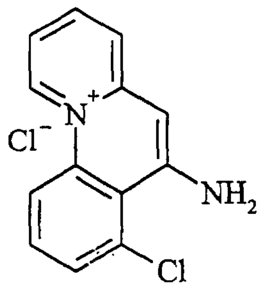

(compound 16)

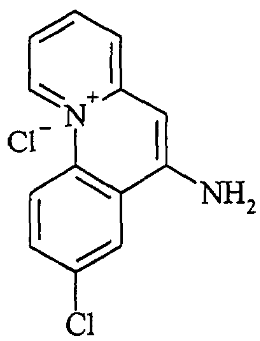

(compound 17)

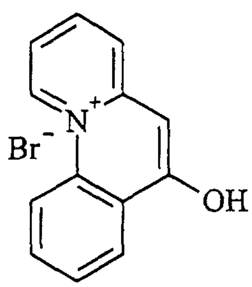

(compound 18 or MPB-06)

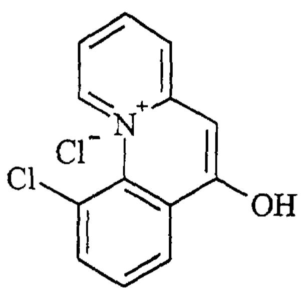

(compound 19 or MPB-07)

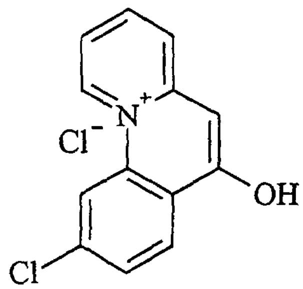

(compound 20 or MPB-08)

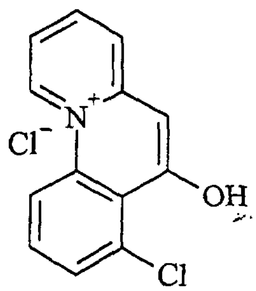

(compound 21 or MPB-27)

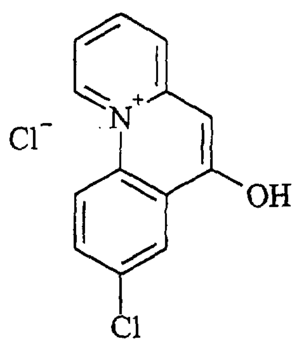

(compound 25 or MPB-30)

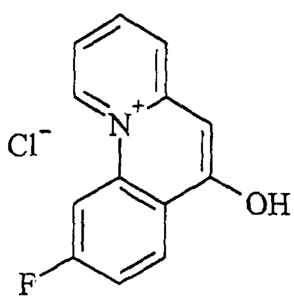

(compound 26 or MPB-29)

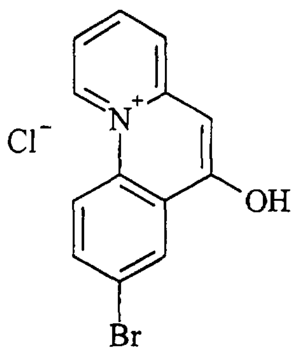

(compound 27 or MPB-32)

**14.** Derivatives according to claim 10, of general formula (IIIb) which follows:

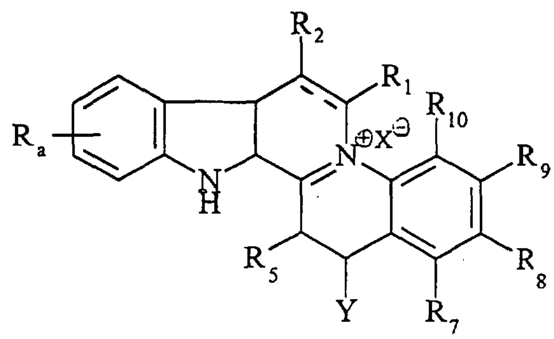

**(IIIb)**

in which $R_a$, $R_1$, $R_2$, $R_5$, $R_7$, $R_8$, $R_9$, $R_{10}$, X and Y are as defined in claim 1, and in particular the compounds of formula (IIIb) in which:

- $R_a$ represents a hydrogen atom,
- $R_1$ and $R_2$ represent a hydrogen atom, and there is no double bond between the two carbons carrying $R_1$ and $R_2$,
- $R_5$ represents a hydrogen atom,
- $R_7$, $R_8$, $R_9$, and $R_{10}$ represent a hydrogen atom, or one of $R_7$, $R_8$, $R_9$, or $R_{10}$ represents a halogen atom, in particular a chlorine, bromine or fluorine atom,
- Y represents $-NH_2$,
- X represents a halogen atom in anionic form, in particular a bromine atom $Br^-$, or a chlorine atom $Cl^-$, or a group of atoms in anionic form, in particular a perchlorate $ClO_4^-$.

**15.** Derivatives according to claim 14, of general formula (IIIb) chosen from the following:

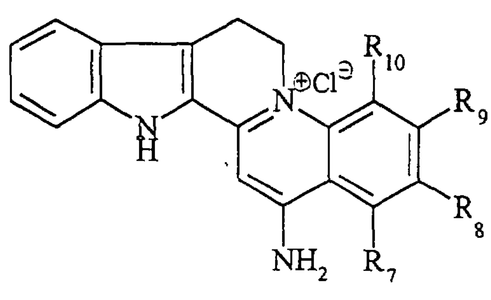

- compound G: $R_7$ = Cl, $R_8 = R_9 = R_{10}$ = H,
- compound H: $R_7 = R_8 = R_9 = R_{10}$ = H,
- compound I: $R_8$ = Cl, $R_7 = R_9 = R_{10}$ = H,
- compound J: $R_9$ = Cl, $R_7 = R_8 = R_{10}$ = H,

- compound K: $R_{10}$ = Cl, $R_7 = R_8 = R_9$ = H,
- compound L: $R_9$ = Br, $R_7 = R_8 = R_{10}$ = H.

**16.** Preparation process for derivatives as defined in claim 1, **characterized in that** it includes the following steps:

- treatment of the derivative of formula (A) in which $R_1$, $R_2$, $R_3$, $R_4$ and $R_5$ are as defined in claim 1, with phenyllithium or lithium diisopropyl amide, advantageously in ether or THF, which leads to the obtaining of derivatives of formula (B) in which $R_1$, $R_2$, $R_3$, $R_4$ and $R_5$ are as defined in claim 1, according to the following reaction diagram:

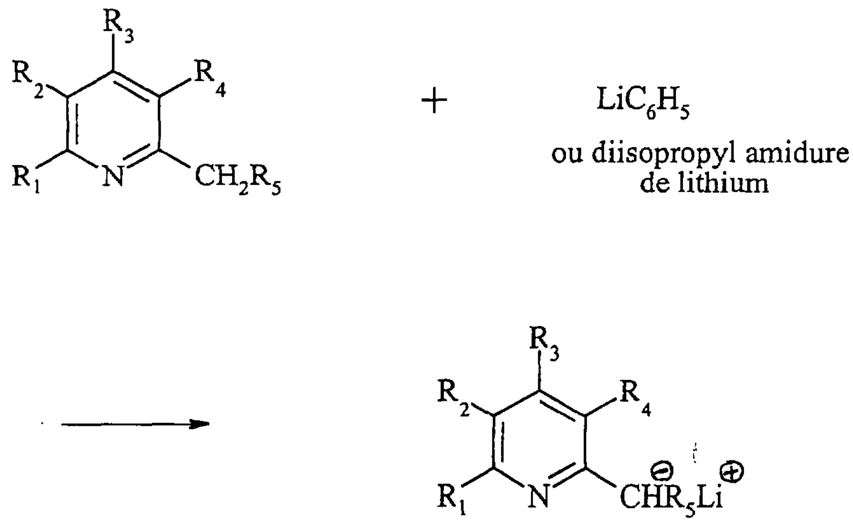


(B)

- condensation of the derivative of formula (B) obtained in the previous stage with the derivative of formula (C) in which $R_7$, $R_8$, $R_9$, $R_{10}$ and X are as defined in formula (I), which leads to the obtaining of derivatives of formula (D) in which $R_1$, $R_2$, $R_3$, $R_4$, $R_5$, $R_7$, $R_8$, $R_9$, $R_{10}$ and X are as defined in claim 1, according to the following reaction diagram:

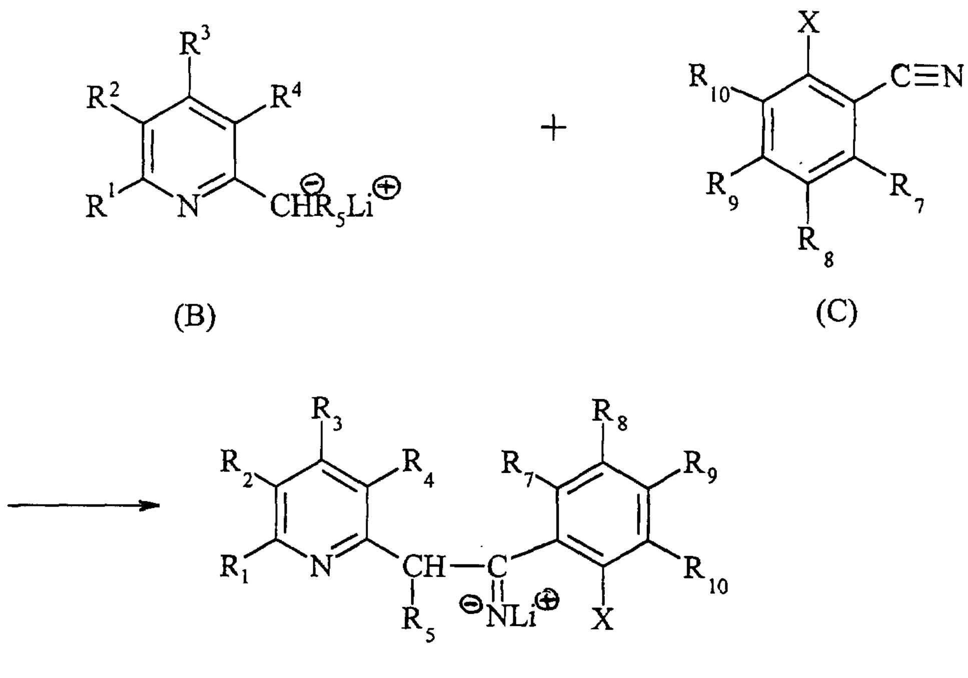

(B) (C)

(D)

- treatment of the compound of formula (D) by the addition of $H_2O$, which leads to the obtaining of the following derivative of formula (II), in which $R_1$ to $R_5$, $R_7$ to $R_{10}$, and X are as defined in claim 1, and Y represents $-NH_2$,

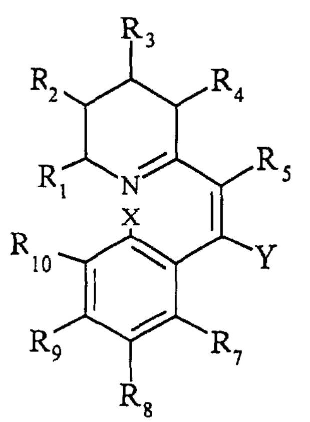

- if appropriate, treatment of the above-mentioned compound of formula (II), with a derivative containing the $R_e$ and $R_f$ groups as defined in claim 1, this derivative being capable of reacting with the nitrogen atom linked to the carbon in position 6 of the above-mentioned compound of formula (II), in particular by a halide of $R_e$ and/or $R_f$ while having, if necessary, taken care to protect beforehand those other functions present on the above-mentioned compound of formula (II) and capable of reacting with the derivative containing the above-mentioned $R_e$ and $R_f$ groups, which leads to the obtaining of the following compound of formula (II) in which R, to $R_5$, $R_7$ to $R_{10}$ and X are as defined above and Y represents an $-NR_eR_f$ group as defined in claim 1,

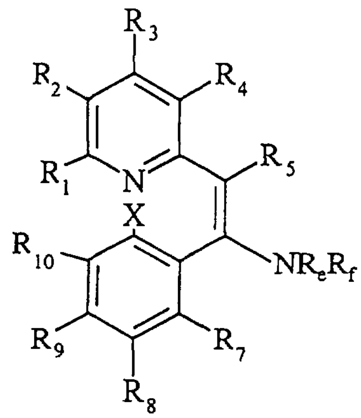

- if appropriate, hydrolysis, in particular by the action of sulphuric acid (pH3) at 40°C, of the above-mentioned compound of formula (II) in which Y represents $NH_2$, which leads to the obtaining of the following compound of formula (II), in which $R_1$ to $R_5$, $R_7$ to $R_{10}$ and X are as defined in claim 1, and Y represents an -OH group,

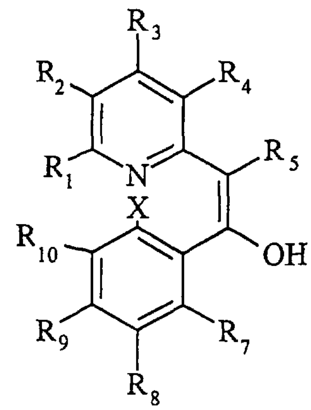

- if appropriate, treatment of the above-mentioned compound of formula (II), in which Y represents an -OH group, with a derivative containing the $R_d$ group, as defined in claim 1, this derivative being capable of reacting with the oxygen atom linked to the carbon in position 7 of the above-mentioned compound of formula (II), in particular by a halide of $R_d$, while having, if necessary, taken care to protect beforehand those other functions present on the above-mentioned compound of formula (II) and capable of reacting with the derivative containing the above-mentioned $R_d$ group, which leads to the obtaining of the following compound of formula (II) in which $R_1$ to $R_5$, $R_7$ to $R_{10}$ and X are as defined above and Y represents an $-OR_d$ group as defined in claim 1,

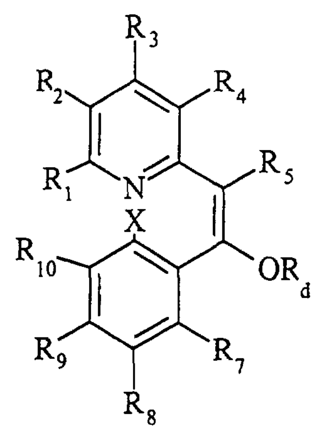

- heating, advantageously at 200°C, the above-mentioned compounds of formula (II) in which Y represents

$-NH_2$ or -OH, which leads respectively to the following compounds of formula (III), in which $R_1$ to $R_5$, $R_7$ to $R_{10}$ and X are as defined in claim 1 and Y represents an $-NH_2$ or -OH group,

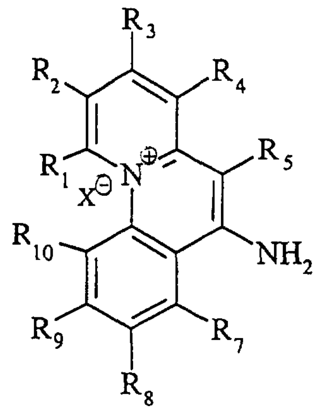

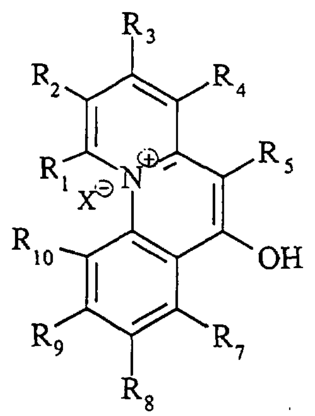

- if appropriate, treatment of the above-mentioned compound of formula (III), in which Y represents an $-NH_2$ group, with a derivative containing the $R_e$ and $R_f$ groups as defined in claim 1, this derivative being capable of reacting with the nitrogen atom linked to the carbon in position 6 of the above-mentioned compound of formula (III), in particular by a halide of $R_e$ and/or $R_f$ while having, if necessary, taken care to protect beforehand those other functions present on the above-mentioned compound of formula (III) and capable of reacting with the derivative containing the above-mentioned $R_e$ and $R_f$ groups, which leads to the obtaining of the following compound of formula (III) ii which $R_1$ to $R_5$, $R_7$ to $R_{10}$ and X are as defined above and Y represents an $-NR_eR_f$ group as defined in claim 1,

- if appropriate, treatment of the above-mentioned compound of formula (III), in which Y represents an -OH group, with a derivative containing the $R_d$ group, as defined in claim 1, this derivative being capable of reacting with the oxygen atom linked to the carbon in position 7 of the above-mentioned compound of formula (III), in particular by a halide of $R_d$, while having, if necessary, taken care to protect beforehand those other functions present on the above-mentioned compound of formula (III) and capable of reacting with the derivative containing the above-mentioned $R_d$ group, which leads to the obtaining of the following compound of formula (III) in which $R_1$ to $R_5$, $R_7$ to $R_{10}$ and X are as defined above and Y represents an $-OR_d$ group as defined in claim 1,

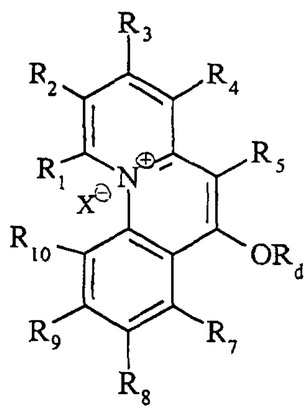

- if appropriate, hydrogenation of the above-mentioned compounds of formulae (II) or (III), in particular by catalytic hydrogenation in the presence of platinum oxide at reduced pressure, which leads to the obtaining of the following compounds of formulae (II) or (III):

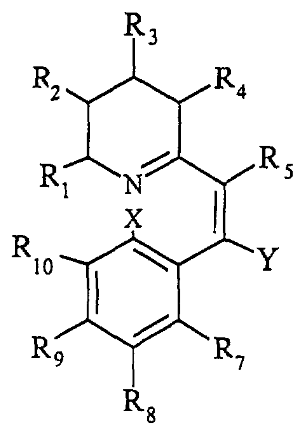

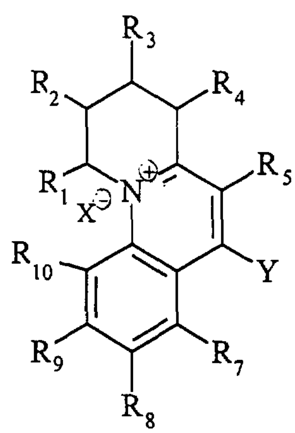

in which $R_1$ to $R_5$, $R_7$ to $R_{10}$, X and Y are as defined above.

**Patentansprüche**

1. Verwendung der Derivate der Benzo[c]chinoliziniums der folgenden allgemeinen Formel (III):

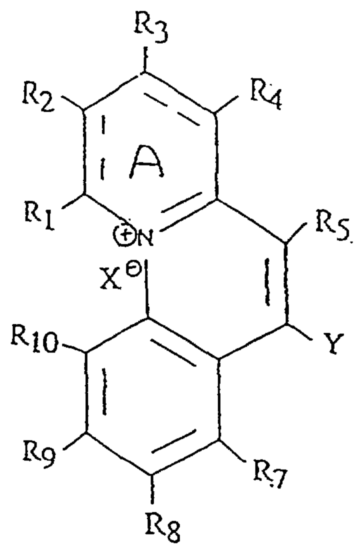

(III)

in welcher:

- der Heterocyclus A aromatisch oder nicht aromatisch ist, wobei im letzteren Fall das Stickstoffatom des Heterocyclus durch eine Doppelbindung an das Kohlenstoffatom der Position 4a gebunden ist,
- $R_1$, $R_2$, $R_3$, $R_4$, $R_5$, $R_7$, $R_8$, $R_9$ und $R_{10}$ unabhängig voneinander Folgendes darstellen:

    - ein Wasserstoff-, Brom- oder Fluoratom, oder
    - ein Halogenatom, insbesondere ein Chloratom, oder
    - eine geradkettige oder verzweigte Alkyl-, Alkoxy-, Carbonyl- oder Oxycarbonylgruppe mit 1 bis 10 Kohlenstoffatomen, wobei die Gruppen gegebenenfalls substituiert sind, insbesondere durch ein Halogen, und/oder durch eine Hydroxylgruppe, und/oder durch eine Amingruppe (primär, sekundär oder tertiär), und/oder durch eine cyclische aromatische und/oder aliphatische Gruppe mit 5 bis 10 Kohlenstoffatomen im Cyclus, wobei die cyclischen Gruppen gegebenenfalls selbst substituiert sind, insbesondere durch ein Halogen, und/oder durch eine Hydroxylgruppe, und/oder durch ein Amin (primär, sekundär oder tertiär), und/oder durch eine Alkyl-, Alkoxy-, Carbonyl- oder Oxycarbonylgruppe, wobei die Gruppen so wie vorstehend definiert sind, oder

- eine cyclische aromatische oder aliphatische Gruppe mit 5 bis 10 Kohlenstoffatomen im Cyclus, wobei der Cyclus selbst gegebenenfalls substituiert ist, insbesondere durch ein Halogen, und/oder durch eine Hydroxylgruppe, und/oder durch ein Amin (primär, sekundär oder tertiär), und/oder durch eine Alkyl-, Alkoxy-, Carbonyl- oder Oxycarbonylgruppe, wobei die Gruppen wie vorstehend definiert sind, oder
- eine -$OR_a$-Gruppe, wobei $R_a$ ein Wasserstoffatom oder eine geradkettige oder verzweigte Alkyl-, Carbonyl- oder Oxycarbonylgruppe darstellt, wobei die Gruppen wie vorstehend definiert sind oder eine cyclische aromatische oder aliphatische Gruppe, wobei die Gruppen wie vorstehend definiert sind, oder
- eine Gruppe -$NR_bR_c$, wobei $R_b$ und $R_c$ unabhängig voneinander eine geradkettige oder verzweigte Alkyl-, Alkoxy-, Carbonyl- oder Oxycarbonylgruppe darstellen,
  wobei die Gruppen wie vorstehend definiert sind, oder eine cyclische aromatische oder aliphatische Gruppe, wobei die Gruppen wie vorstehend definiert sind, oder
- wenn $R_1$ und $R_2$, oder $R_3$ und $R_4$, und/oder $R_4$ und $R_5$, und/oder $R_7$ und $R_8$, und/oder $R_8$ und $R_9$, und/oder $R_9$ und $R_{10}$ nicht die verschiedenen Atome oder Gruppen oder cyclischen Gruppen darstellen, die vorstehend erwähnt wurden, dann bildet $R_1$ in Verbindung mit $R_2$, oder $R_2$ in Verbindung mit $R_3$, und/oder $R_3$ in Verbindung mit $R_4$, und/oder $R_4$ in Verbindung mit $R_5$, und/oder $R_7$ in Verbindung mit $R_8$, und/oder $R_8$ in Verbindung mit $R_9$, und/oder $R_9$ in Verbindung mit $R_{10}$ jeweils mit $C_1$ und $C_2$, oder mit $C_2$ und $C_3$, oder mit $C_3$ und $C_4$, oder mit $C_4$, $C_{4a}$ und $C_5$, oder mit $C_7$ und $C_8$, oder mit $C_8$ und $C_9$, oder mit $C_9$ und $C_{10}$ eine aromatische oder aliphatische cyclische Gruppe mit 5 bis 10 Kohlenstoffatomen, wobei der Cyclus gegebenenfalls substituiert ist, insbesondere durch ein Halogen, und/oder durch eine Alkyl-, Alkoxy-, Carbonyl- oder Oxycarbonylgruppe, und/oder durch eine aromatische oder aliphatische Gruppe, wobei die Gruppen oder Cyclen wie vorstehend definiert sind, oder
- wenn $R_3$ und $R_4$ nicht die verschiedenen Atome oder Gruppen oder Cyclen, die vorstehend erwähnt wurden,

darstellen, dann bildet $R_3$ in Verbindung mit $R_4$ eine Indolgruppe der Formel

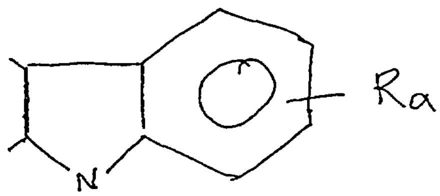

in welcher $R_a$ wie vorstehend definiert ist,

- Y darstellt:

  - eine Gruppe $-OR_d$, wobei $R_d$ ein Wasserstoffatom oder eine geradkettige oder verzweigte Alkyl-, Carbonyl- oder Oxycarbonylgruppe darstellt, wobei die Gruppen wie vorstehend definiert sind, oder eine cyclische aromatische oder aliphatische Gruppe, wobei die cyclischen Gruppen wie vorstehend definiert sind, oder
  - eine Gruppe $-NR_eR_f$, wobei $R_e$ und $R_f$ unabhängig voneinander eine geradkettige oder verzweigte Alkyl-, Alkoxy-, Carbonyl- oder Oxycarbonylgruppe darstellen,

wobei die Gruppen wie vorstehend definiert sind, oder eine cyclische aromatische oder aliphatische Gruppe sind,
wobei die cyclischen Gruppen wie vorstehend definiert sind,

- wobei wenn $R_d$, oder wenigstens eine von $R_e$ oder $R_f$ nicht eine der verschiedenen Atome oder Gruppen oder Cyclen darstellen, die vorstehend erwähnt wurden, dann $R_d$ oder wenigstens eine von $R_e$ oder $R_f$ in Verbindung mit $R_5$ oder in Verbindung mit $R_7$ jeweils mit $C_5$ und $C_6$ oder mit $C_6$, $C_{6a}$ und $C_7$ einen aromatischen oder aliphatischen Heterocyclus mit 5 bis 10 Kohlenstoffatomen bilden, der gegebenenfalls substituiert ist, insbesondere durch ein Halogen, und/oder durch eine Alkyl-, Alkoxy-, Carbonyl- oder Oxycarbonylgruppe, und/oder durch eine cyclische aromatische oder aliphatische Gruppe, wobei die Gruppen oder Cyclen wie vorstehend definiert sind,

  - X ein Atom in anionischer Form darstellt, wie etwa ein Halogenatom, insbesondere ein Bromatom oder Chloratom, oder eine Gruppe von Atomen in anionischer Form, wie etwa Perchlorat, und der Stickstoff des Heterocyclus A der Formel (I) in quartärer Form ist und einerseits durch kovalente Bindung an den Kohlenstoff in Position 11 gebunden, und andererseits durch ionische Bindung an das vorstehend definierte X gebunden ist,

  * wobei wenn $R_1$ und $R_{10}$ nicht eines der verschiedenen Atome oder Gruppen oder Cyclen, die vorstehend definiert wurden, darstellen, dann $R_1$ in Verbindung mit $R_{10}$ mit $C_1$, dem Stickstoff des Heterocyclus A der Formel (I), $C_{11}$, und $C_{10}$ einen aromatischen oder aliphatischen Heterocyclus mit 5 bis 10 Kohlenstoffatomen bildet, der gegebenenfalls substituiert ist, insbesondere durch ein Halogen und/oder durch eine Alkyl-, Alkoxy-, Carbonyl- oder Oxycarbonylgruppe, und/oder durch eine cyclische aromatische oder aliphatische Gruppe, wobei die Gruppen oder Cyclen wie vorstehend definiert sind, zur Herstellung von Medikamenten, die auf die Behandlung von Krankheiten im (menschlichen oder tierischen) Organismus gerichtet sind, insbesondere Lungen-, Verdauungs- oder Herzerkrankungen, die mit Störungen des ionischen Transmembranflusses, insbesondere von Chlor und gegebenenfalls von Bicarbonat, verbunden sind, insbesondere zur Herstellung von Medikamenten, die auf die Behandlung von Mukoviszidose gerichtet sind, oder auf die Verhinderung der Abstoßung von cytotoxischen Medikamenten (insbesondere gegen Tumore), oder auf die Behandlung von Verschlüssen der Bronchienwege oder der Verdauungswege (insbesondere Pankreas oder Darm) gerichtet sind.

**2.** Verwendung gemäß Anspruch 1 der Derivate der folgenden allgemeinen Formel (IIIa):

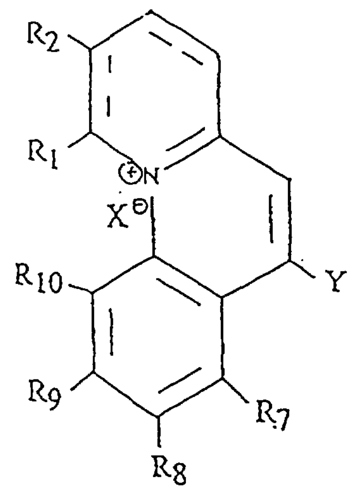

(IIIa)

in welcher:

- $R_1$ und $R_2$ ein Wasserstoffatom darstellen, oder in Verbindung mit $C_1$ und $C_2$ einen aromatischen Cyclus mit 6 Kohlenstoffatomen bilden,
- Y eine Gruppe -OH oder $-NH_2$ oder $-NHCOCH_3$ darstellt,
- $R_7$, $R_8$, $R_9$ und $R_{10}$ ein Wasserstoffatom darstellen, oder eines von $R_7$, $R_8$, $R_9$ oder $R_{10}$ ein Halogenatom darstellt, insbesondere ein Chloratom, Bromatom oder Fluoratom,
- X ein Halogenatom in anionischer Form darstellt, insbesondere ein Bromatom Br-, oder Chloratom Cl oder eine Gruppe von Atomen in anionischer Form, insbesondere ein Perchlorat $ClO_4^-$.

**3.** Verwendung gemäß Anspruch 1 oder Anspruch 2, von Verbindungen der Formel (IIIa), in welcher:

- $R_1$ und $R_2$ ein Wasserstoffatom darstellen,
- Y eine Gruppe -OH darstellt,
- X ein Halogenatom in anionischer Form darstellt, insbesondere ein Bromatom $Br^-$ oder Chloratom $Cl^-$, oder eine Gruppe von Atomen in anionischer Form, insbesondere ein Perchlorat $ClO_4^-$,
- $R_7$, $R_8$, $R_9$ und $R_{10}$ unabhängig voneinander ein Wasserstoffatom oder ein Halogenatom darstellen, insbesondere ein Chloratom, Bromatom oder Fluoratom.

**4.** Verwendung gemäß Ansprüchen 1 bis 3, der Derivate der Benzo[c]chinoliziniums der allgemeinen Formel (IIIa), die aus den folgenden ausgewählt sind:

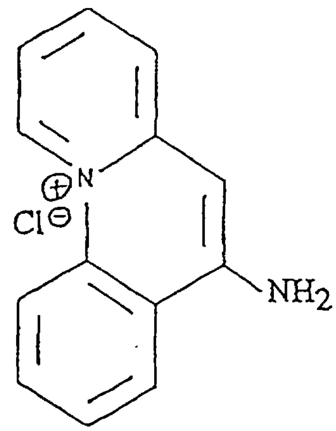

(Verbindung 11 oder MPB-26)

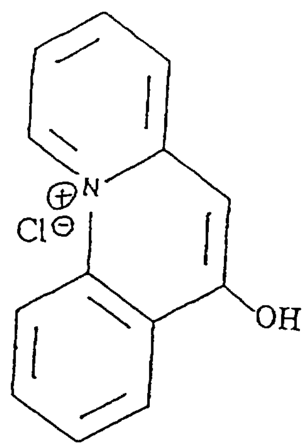

(Verbindung 12 oder MPB-05)

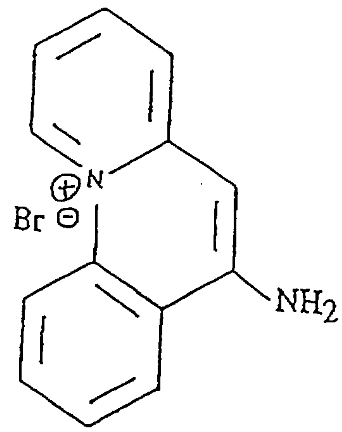

(Verbindung 13 oder MPB-01)

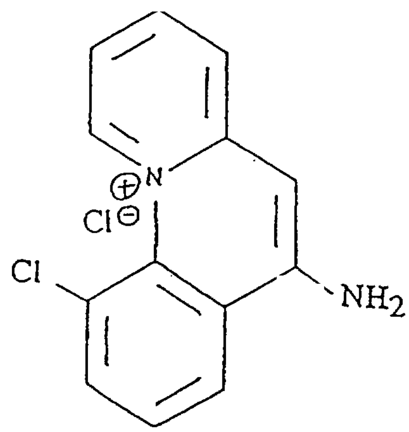

(Verbindung 14 oder MPB-02)

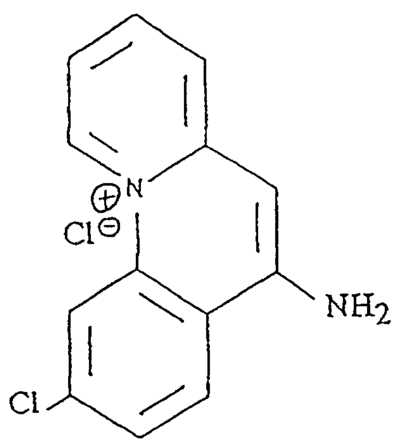

(Verbindung 15 oder MPB-03)

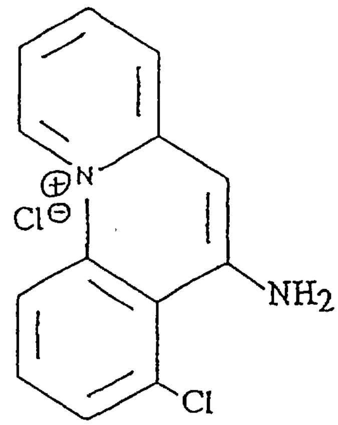

(Verbindung 16)

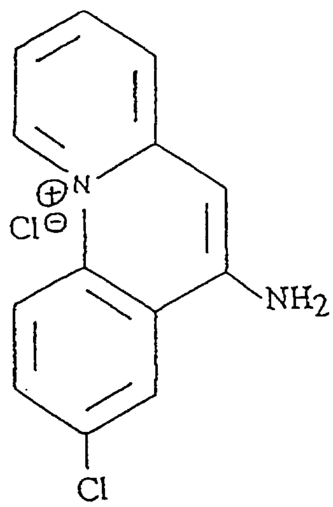

(Verbindung 17)

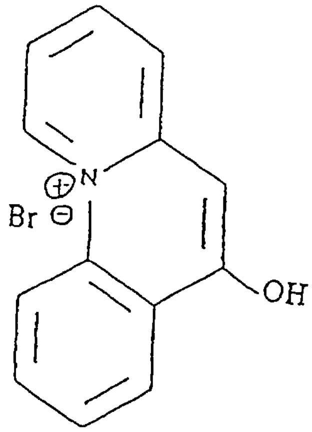

(Verbindung 18 oder MPB-06)

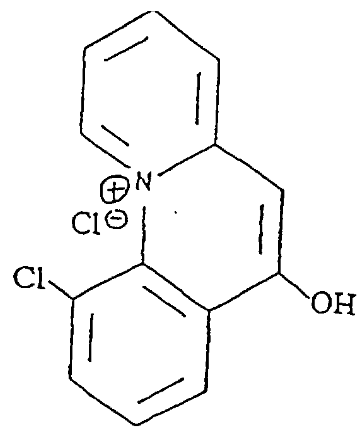

(Verbindung 19 oder MPB-07)

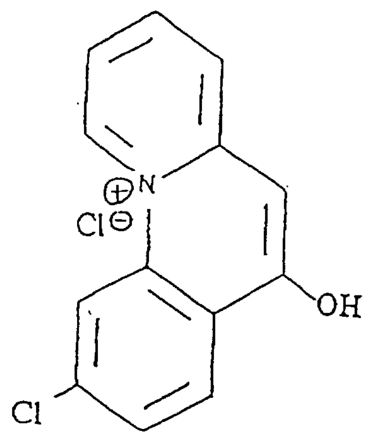

(Verbindung 20 oder MPB-08)

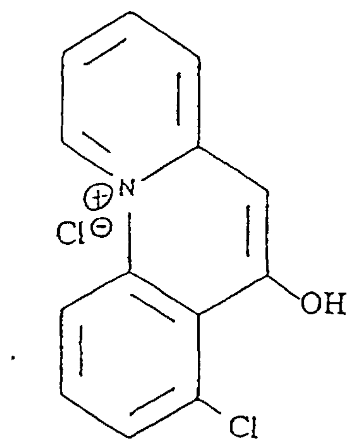

(Verbindung 21 oder MPB-27)

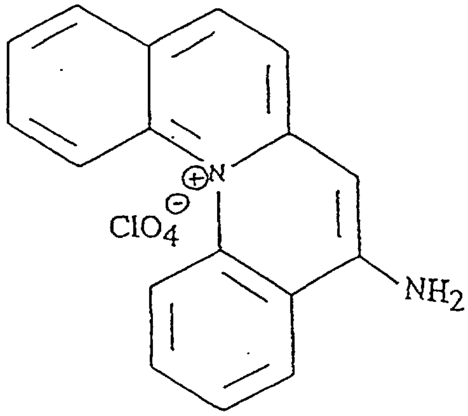

(Verbindung 22)

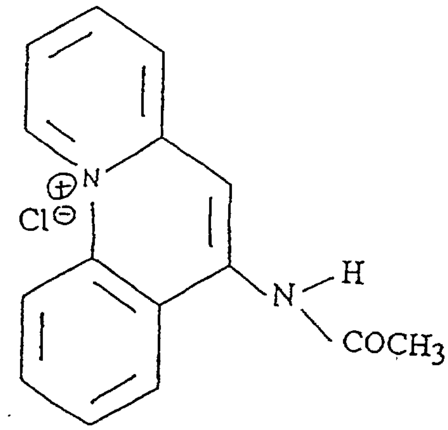

(Verbindung 23)

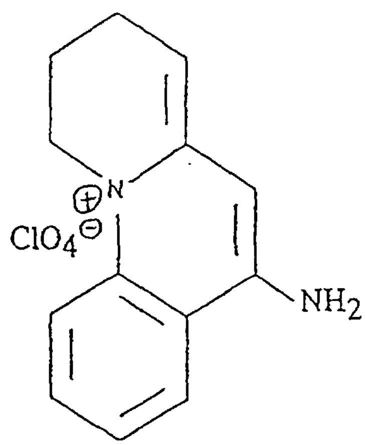

(Verbindung 24)

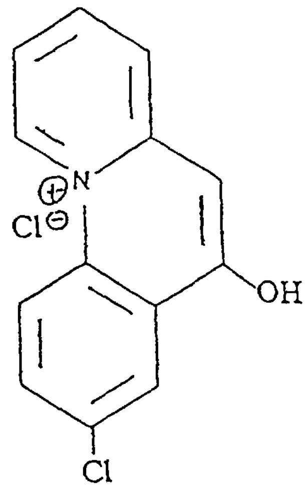

(Verbindung 25 oder MPB-30)

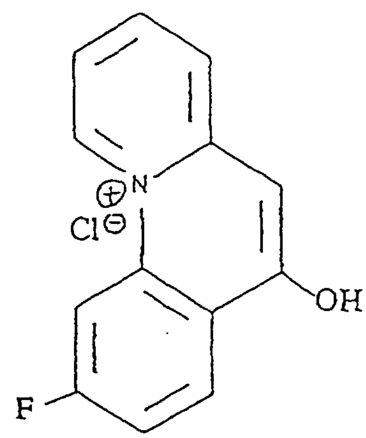

(Verbindung 26 oder MPB-29)

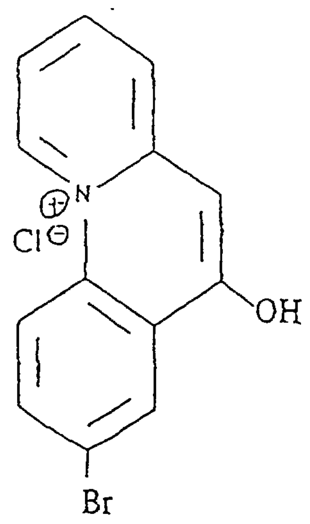

(Verbindung 27 oder MPB-32)

**5.** Verwendung gemäß Anspruch 1, der Derivate der folgenden allgemeinen Formel (IIIb):

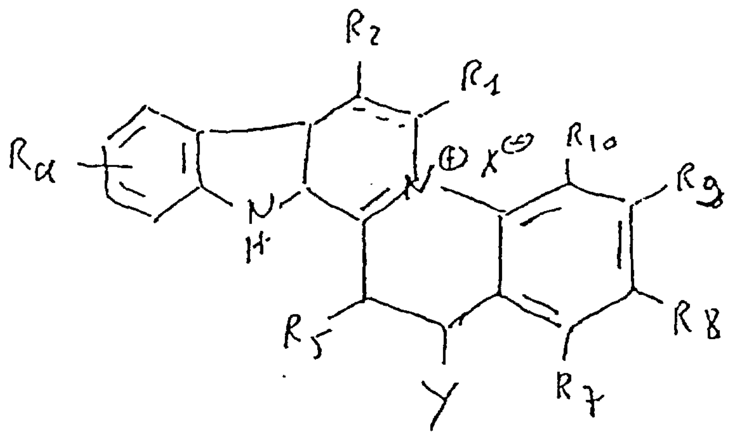

(IIIb)

in welcher:

- $R_a$ ein Wasserstoffatom darstellt,
- $R_1$ und $R_2$ ein Wasserstoffatom darstellen, und es keine Doppelbindung zwischen zwei Kohlenstoffen, die $R_1$ und $R_2$ tragen, gibt,
- $R_5$ ein Wasserstoffatom darstellt,
- $R_7$, $R_8$, $R_9$ und $R_{10}$ ein Wasserstoffatom darstellen, oder das eine von $R_7$, $R_8$, $R_9$ oder $R_{10}$ ein Halogenatom darstellt, insbesondere ein Chloratom, Bromatom oder Fluoratom,
- Y $-NH_2$ darstellt,
- X ein Halogenatom in anionischer Form darstellt, insbesondere ein Bromatom $Br^-$, oder ein Chloratom $Cl^-$, oder eine Gruppe von Atomen in anionischer Form, insbesondere ein Perchlorat $ClO_4^-$ darstellt.

**6.** Verwendung gemäß Anspruch 1 der Derivate der allgemeinen Formel (IIIb), die aus den folgenden ausgewählt sind:

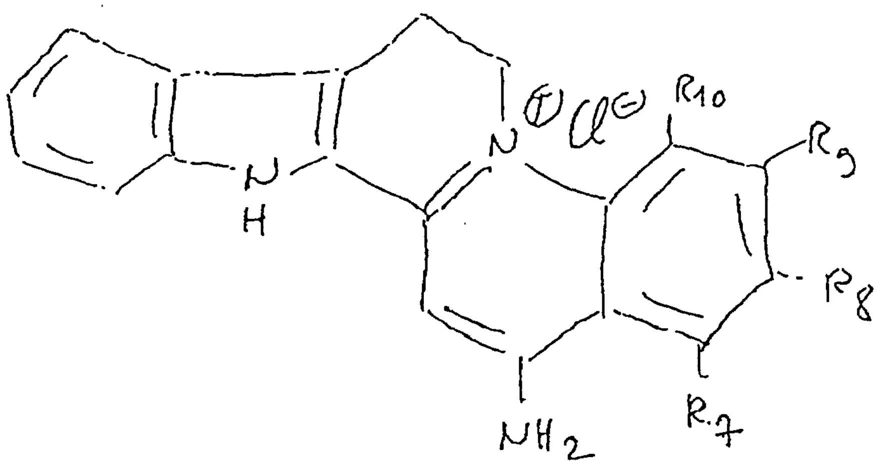

- Verbindung G: $R_7$ = Cl, $R_8$ = $R_9$ = $R_{10}$ = H,
- Verbindung H: $R_7$ = $R_8$ = $R_9$ = $R_{10}$ = H,
- Verbindung I: $R_8$ = Cl, $R_7$ = $R_9$ = $R_{10}$ = H,
- Verbindung J: $R_9$ = Cl, $R_7$ = $R_8$ = $R_{10}$ = H,
- Verbindung K: $R_{10}$ = Cl, $R_7$ = $R_8$ = $R_9$ = H,
- Verbindung L: $R_9$ = Br, $R_7$ = $R_8$ = $R_{10}$ = H.

**7.** Pharmazeutische Zusammensetzungen, **dadurch gekennzeichnet, dass** diese als aktiven Wirkstoff / aktive Wirkstoffe wenigstens eines der in einem der Ansprüche 1 bis 6 definierten Derivate der allgemeinen Formel (III) in Verbindung mit einem physiologisch akzeptablen Träger umfassen.

**8.** Pharmazeutische Zusammensetzungen gemäß Anspruch 7, **dadurch gekennzeichnet, dass** diese oral verabreichbar sind, insbesondere in Form von Tabletten oder in parenteral verabreichbarer Form, insbesondere in Form von Präparaten, die intravenös, intramuskulär oder subkutan injizierbar sind, oder über die Luft verabreichbar sind, insbesondere durch die Lunge in Form von Aerosolen.

**9.** Pharmazeutische Zusammensetzungen gemäß Anspruch 7 oder Anspruch 8, **dadurch gekennzeichnet, dass** die Mengen des aktiven Wirkstoffs / der aktiven Wirkstoffe so sind, dass die tägliche Einnahmemenge, auf eine oder mehrere Dosen verteilt, von aktivem Wirkstoff / aktiven Wirkstoffen ungefähr 0,1 mg/kg bis 5 mg/kg beträgt, insbesondere ungefähr 3 mg/kg.

**10.** Derivate der Benzo[c]chinoliziniums der folgenden Formel (III):

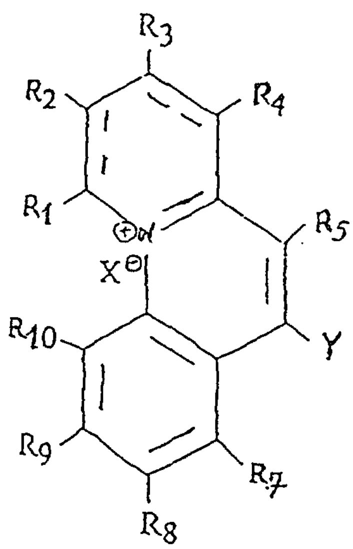

(III)

in welcher $R_1$, $R_2$, $R_3$, $R_4$, $R_5$, $R_7$, $R_8$, $R_9$, $R_{10}$, X und Y so wie in Anspruch 1 definiert sind, wobei die Verbindungen folgender Formel ausgeschlossen sind:

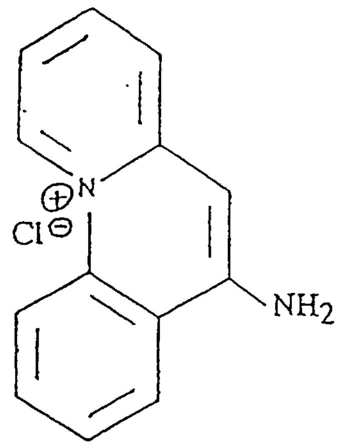

(Verbindung 11 oder MPB-26)

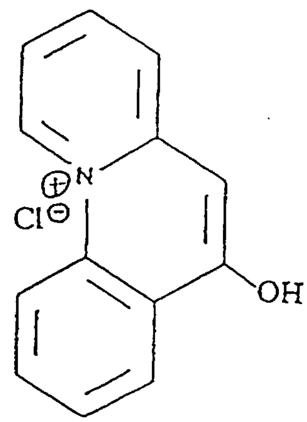

(Verbindung 12 oder MPB-05)

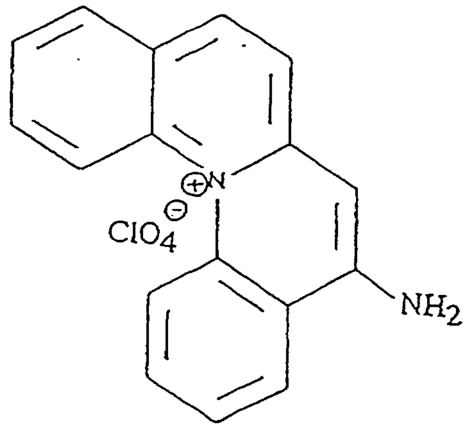

(Verbindung 22)

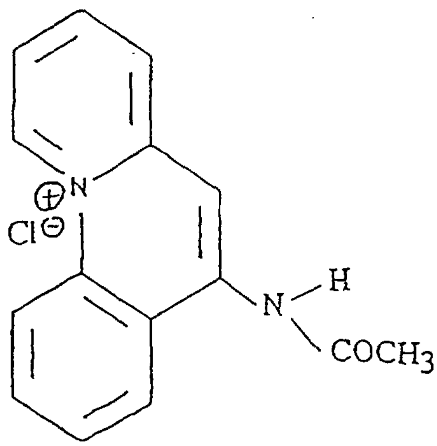

(Verbindung 23)

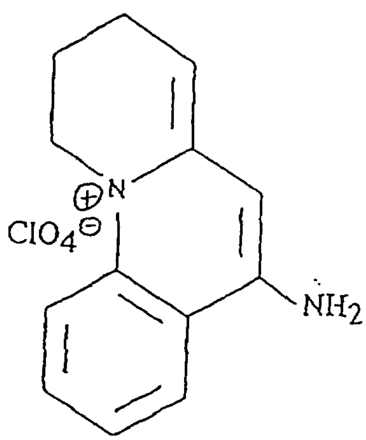

(Verbindung 24)

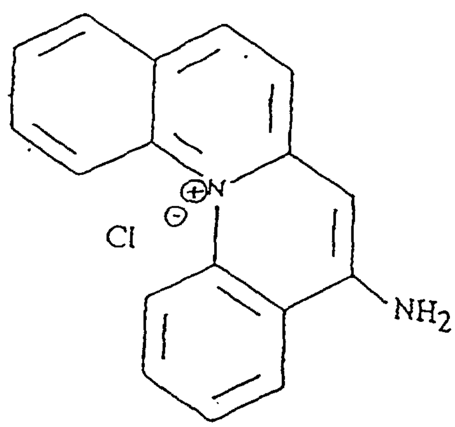

**11.** Derivate gemäß Anspruch 10 mit der folgenden allgemeinen Formel (IIIa):

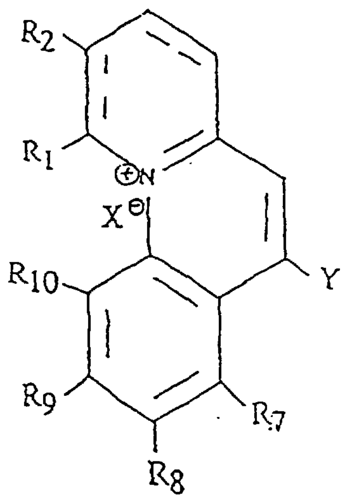

(IIIa)

in welcher:

- $R_1$ und $R_2$ ein Wasserstoffatom darstellen, oder in Verbindung mit $C_1$ und $C_2$ einen aromatischen Cyclus von 6 Kohlenstoffatomen bilden,
- Y eine Gruppe -OH oder $-NH_2$ oder $-NHCOCH_3$ darstellt,
- $R_7$, $R_8$, $R_9$ und $R_{10}$ ein Wasserstoffatom darstellen, oder eines von $R_7$, $R_8$, $R_9$ oder $R_{10}$ ein Halogenatom darstellen, insbesondere ein Chloratom, Bromatom oder Fluoratom,
- X ein Halogenatom in anionischer Form darstellt, insbesondere ein Bromatom $Br^-$, oder Chloratom $Cl^-$, oder eine Gruppe von Atomen in anionischer Form, insbesondere ein Perchlorat $ClO_4^-$.

**12.** Derivate gemäß Anspruch 11 mit der Formel (IIIa), in welcher:

- $R_1$ und $R_2$ ein Wasserstoffatom darstellen,
- Y eine Gruppe -OH darstellt,
- X ein Halogenatom in anionischer Form darstellt,
  insbesondere ein Bromatom $Br^-$, oder ein Chloratom $Cl^-$ oder eine Gruppe von Atomen in anionischer Form, insbesondere ein Perchlorat $ClO_4^-$,
- $R_7$, $R_8$, $R_9$ und $R_{10}$ unabhängig voneinander ein Wasserstoffatom oder ein Halogenatom, insbesondere ein Chloratom, Bromatom oder Fluoratom, darstellen.

**13.** Derivate der Benzo[c]chinoliziniums gemäß den Ansprüchen 10 bis 12 der allgemeinen Formel (IIIa), ausgewählt aus den folgenden Verbindungen:

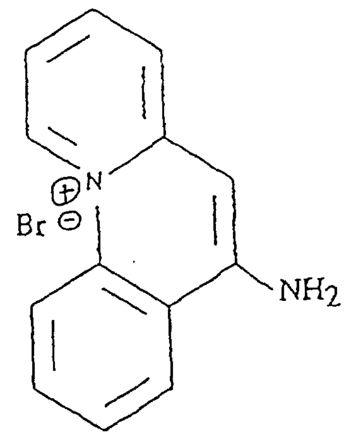

(Verbindung 13 oder MPB-01)

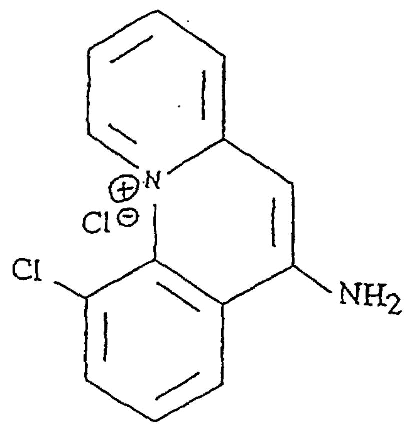

(Verbindung 14 oder MPB-02)

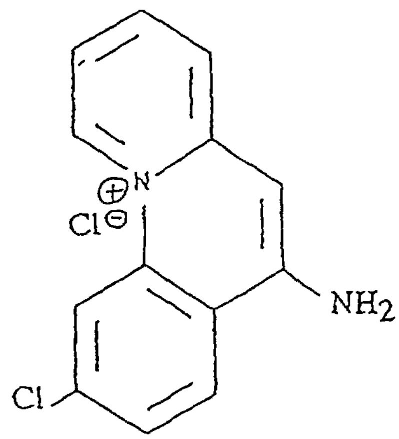

(Verbindung 15 oder MPB-03)

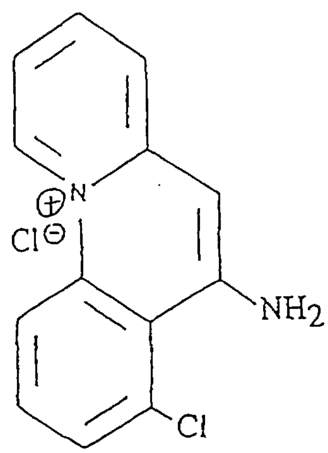

(Verbindung 16)

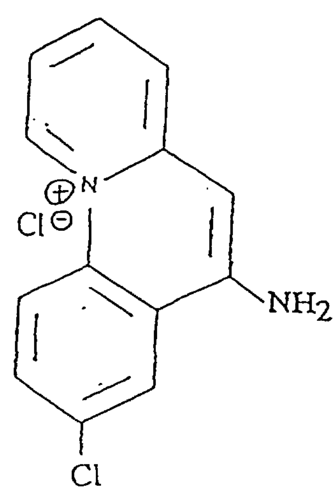

(Verbindung 17)

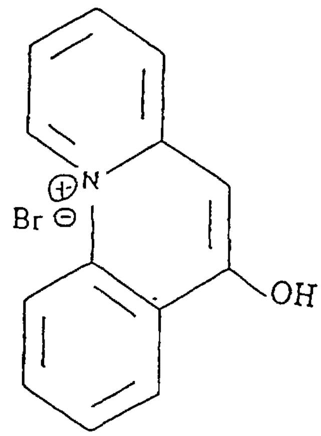

(Verbindung 18 oder MPB–06)

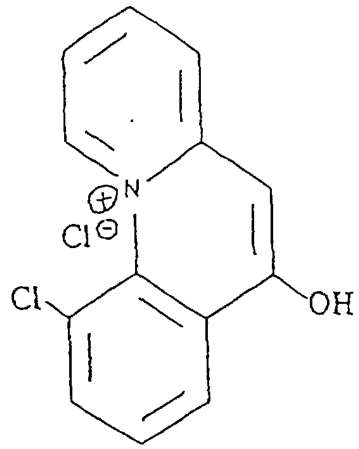

(Verbindung 19 oder MPB-07)

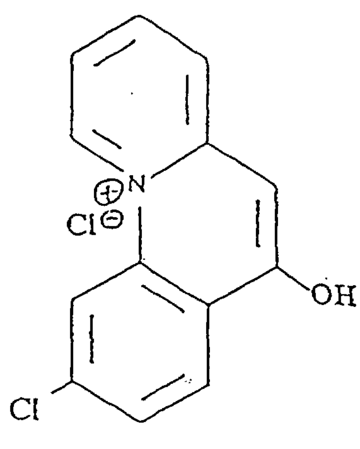

(Verbindung 20 oder MPB-08)

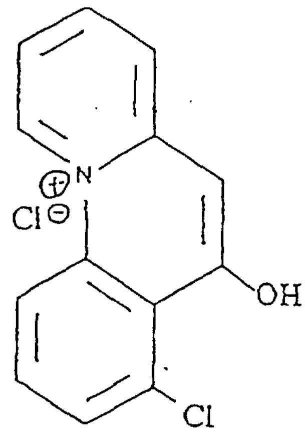

(Verbindung 21 oder MPB-27)

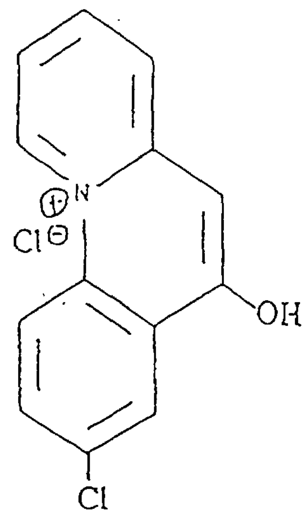

(Verbindung 25 oder MPB-30)

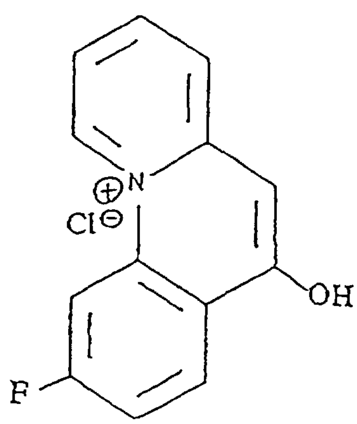

(Verbindung 26 oder MPB-29)

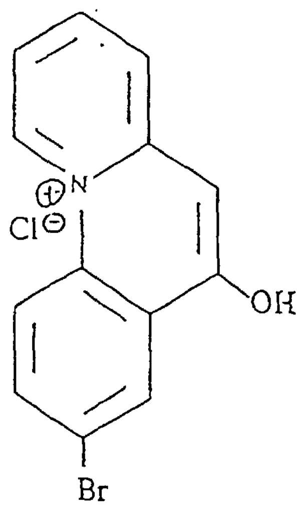

(Verbindung 27 oder MPB-32)

**14.** Derivate gemäß Anspruch 10, der folgenden allgemeinen Formel (IIIb):

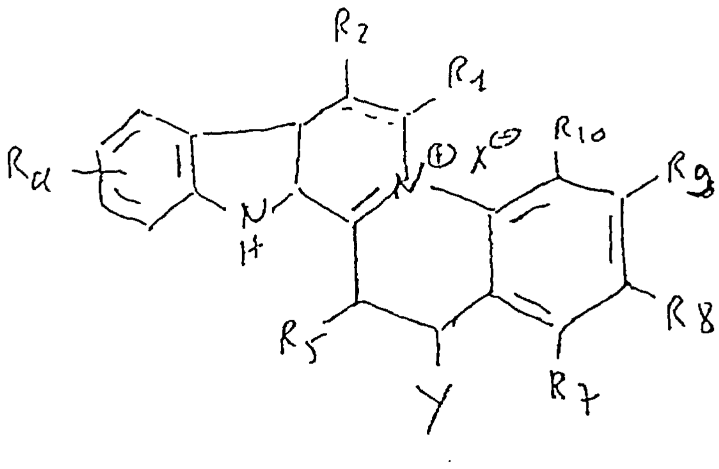

(IIIb)

in welcher $R_a$, $R_1$, $R_2$, $R_5$, $R_7$, $R_8$, $R_9$, $R_{10}$, X und Y so wie in Anspruch 1 definiert sind, und insbesondere die Verbindung der Formel (IIIb), in welcher:

- $R_a$ ein Wasserstoffatom darstellt,
- $R_1$ und $R_2$ ein Wasserstoffatom darstellen, und es keine Doppelbindung zwischen den beiden Kohlenstoffatomen gibt, die $R_1$ und $R_2$ tragen,
- $R_5$ ein Wasserstoffatom darstellt,
- $R_7$, $R_8$, $R_9$ und $R_{10}$ ein Wasserstoffatom darstellen, oder eines von $R_7$, $R_8$, $R_9$ oder $R_{10}$ ein Halogenatom darstellt, insbesondere ein Chloratom, Bromatom oder Fluoratom,
- Y $-NH_2$ darstellt,
- X ein Halogenatom in anionischer Form darstellt, insbesondere ein Bromatom $Br^-$, oder Chloratom $Cl^-$, oder eine Gruppe von Atomen in anionischer Form, insbesondere ein Perchlorat $ClO_4^-$.

**15.** Derivate gemäß Anspruch 14 der allgemeinen Formel (IIIb), ausgewählt aus den folgenden Verbindungen:

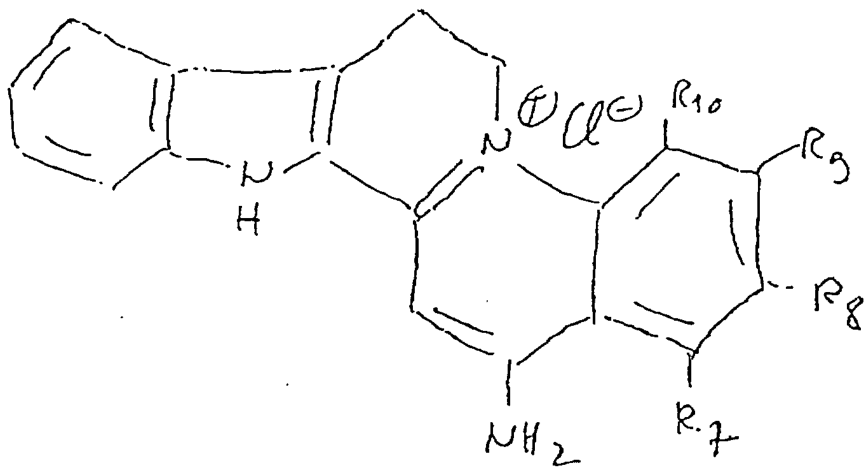

- Verbindung G: $R_7$ = Cl, $R_8$ = $R_9$ = $R_{10}$ = H,
- Verbindung H: $R_7$ = $R_8$ = $R_9$ = $R_{10}$ = H,
- Verbindung I: $R_8$ = Cl, $R_7$ = $R_9$ = $R_{10}$ = H,
- Verbindung J: $R_9$ = Cl, $R_7$ = $R_8$ = $R_{10}$ = H,
- Verbindung K: $R_{10}$ = Cl, $R_7$ = $R_8$ = $R_9$ = H,
- Verbindung L: $R_9$ = Br, $R_7$ = $R_8$ = $R_{10}$ = H.

**16.** Verfahren zur Herstellung von in Anspruch 1 definierten Derivaten, **dadurch gekennzeichnet, dass** dieses die folgenden Stufen umfasst:

- Behandlung des Derivats der Formel (A), in welcher $R_1$, $R_2$, $R_3$, $R_4$ und $R_5$ so wie in Anspruch 1 definiert sind, mit Phenyllithium oder dem Lithium-Diisopropylamid, vorteilhafter Weise in Ether oder in THF, was zum Erhalt von Derivaten der Formel (B) führt, in welcher $R_1$, $R_2$, $R_3$, $R_4$ und $R_5$ so wie in Anspruch 1 definiert sind, gemäß dem folgenden Reaktionsschema:

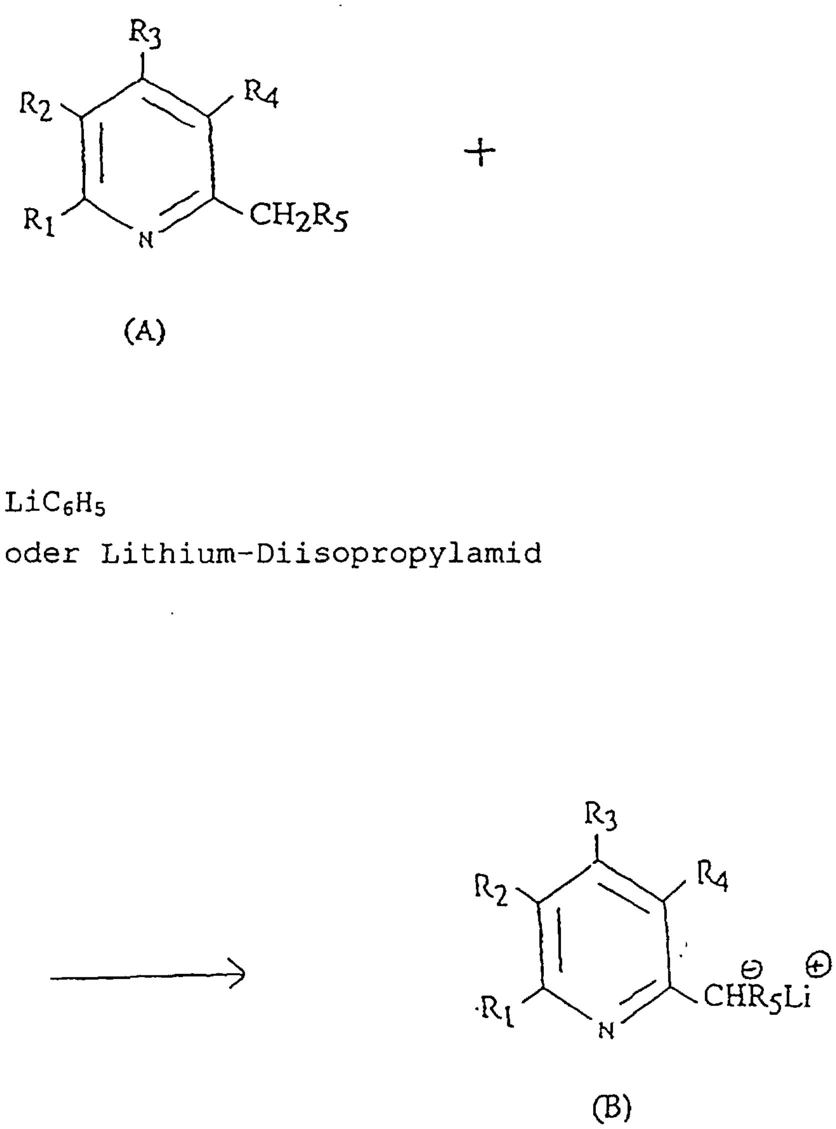


(A)

(B)

- Kondensation des in der vorhergehenden Stufe erhaltenen Derivats der. Formel (B) mit dem Derivat der Formel (C),

in welcher $R_7$, $R_8$, $R_9$, $R_{10}$ und X so wie in der Formel (I) definiert sind, was zum Erhalt von Derivaten der Formel (D) führt, in welcher $R_1$, $R_2$, $R_3$, $R_4$, $R_5$, $R_7$, $R_8$, $R_9$, $R_{10}$ und X so wie in Anspruch 1 definiert sind, gemäß dem folgenden Reaktionsschema:

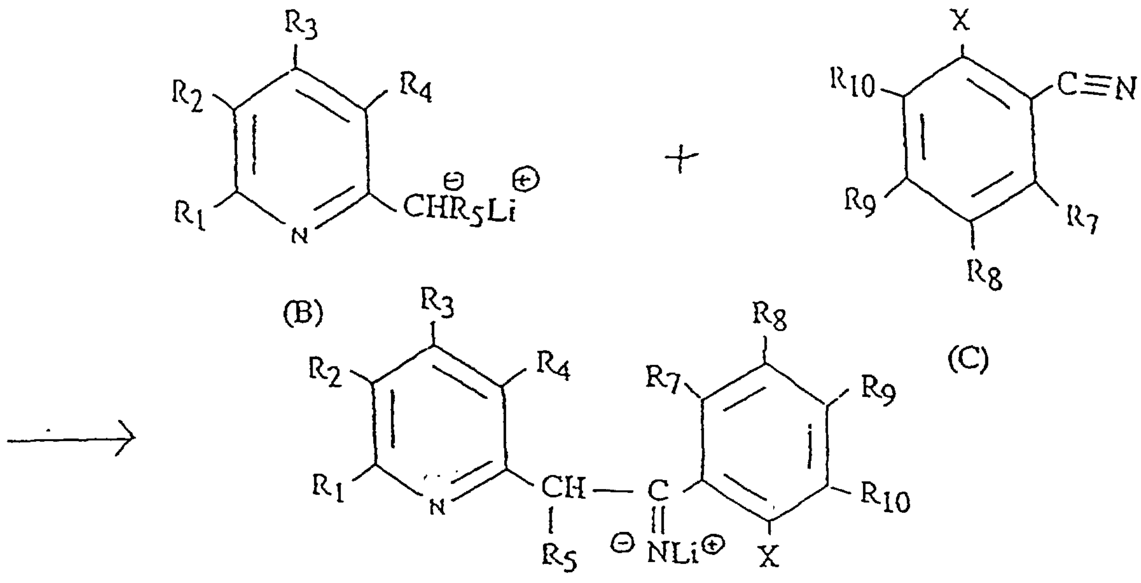

(B)

(C)

(D)

- Behandlung der Verbindung der Formel (D) durch Zugabe von $H_2O$, was zum Erhalt des Derivats der folgenden Formel (II) führt, in welcher $R_1$ bis $R_5$, $R_7$ bis $R_{10}$ und X so wie in Anspruch 1 definiert sind, und Y eine Gruppe $-NH_2$ darstellt,

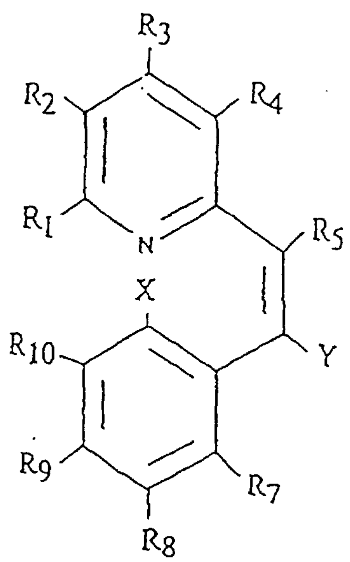

- gegebenenfalls Behandlung der Verbindung der vorstehend erwähnten Formel (II) durch ein Derivat, das die so wie in Anspruch 1 definierten Gruppen $R_e$ und $R_f$ trägt, wobei das Derivat gegenüber dem Stickstoffatom, das an das Kohlenstoff der Position 6 der Verbindung der vorstehend erwähnten Formel gebunden ist, reaktiv ist, insbesondere durch eine Halogenierung von $R_e$ und / oder von $R_f$, wobei, soweit notwendig, die anderen Funktionen, die in der Verbindung der vorstehend erwähnten Formel (II) vorhanden sind und die gegenüber dem die vorstehend erwähnten Gruppen $R_e$ und $R_f$ tragenden Derivat reaktiv sind, im vorhinein mit Schutzgruppen versehen werden, was zum Erhalt der Verbindung der folgenden Formel (II) führt, in welcher $R_1$ bis $R_5$, $R_7$ bis $R_{10}$ und X so wie vorstehend definiert sind, und Y eine so wie in Anspruch 1 definierte Gruppe

$-NR_eR_f$ darstellt,

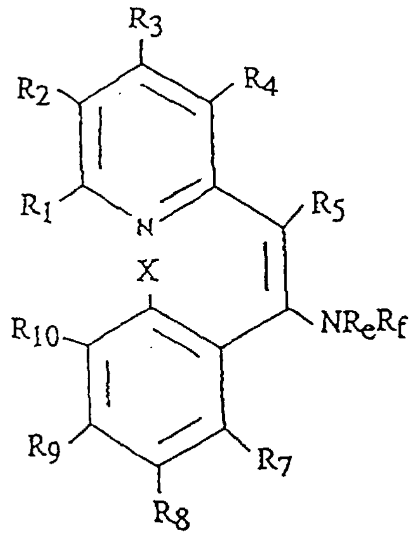

- gegebenenfalls, Hydrolyse, insbesondere mit Hilfe von Schwefelsäure (pH3) bei 40°C, der Verbindung der vorstehend erwähnten Formel (II), in welcher Y $NH_2$ darstellt, was zum Erhalt der Verbindung der folgenden Formel (II) führt, in welcher $R_1$ bis $R_5$, $R_7$ bis $R_{10}$ und X so wie in dem Anspruch 1 definiert sind, und Y eine Gruppe -OH darstellt,

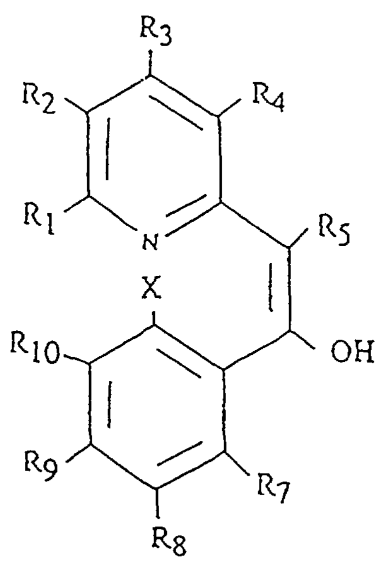

- gegebenenfalls Behandlung der oben erwähnten Formel (II), in welcher Y eine Gruppe -OH darstellt, durch ein Derivat, das die wie in Anspruch 1 definierte Gruppe $R_d$ trägt, wobei das Derivat gegenüber dem Sauerstoffatom reaktiv ist, das an das Kohlenstoffatom der Position 7 der Verbindung der oben erwähnten Formel (II) gelegen ist, insbesondere durch eine Halogenierung von $R_d$, wobei, wenn notwendig, die anderen Funktionen, die in der Verbindung der oben erwähnten Formel (II) vorhanden sind und die gegenüber dem die vorstehend erwähnte Gruppe $R_d$ tragendem Derivat reaktiv sind, im vorhinein mit einer Schutzgruppe versehen werden, was zum Erhalt der Verbindung der folgenden Formel (II') führt, in welcher $R_1$ bis $R_5$, $R_7$ bis $R_{10}$ und X so wie vorstehend definiert sind, und Y eine in Anspruch 1 definierte Gruppe $-OR_d$ darstellt,

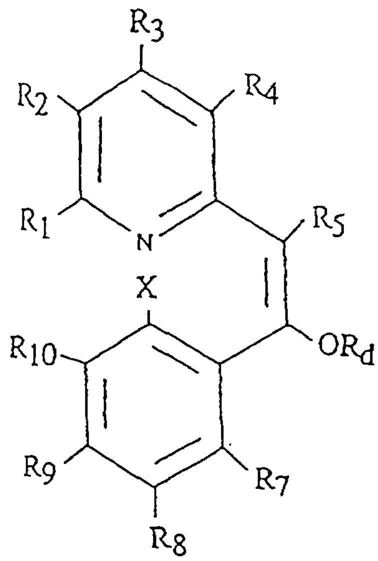

- Erwärmung, vorteilhafter Weise bis auf 200°C, der Verbindungen der vorstehend erwähnten Formel (II), in welcher Y $-NH_2$ oder -OH darstellt, was jeweils zu Verbindungen der folgenden Formel (III) führt, in welcher $R_1$ bis $R_5$, $R_7$ bis $R_7$ bis $R_{10}$ und X so wie in Anspruch 1 definiert sind, und Y eine Gruppe $-NH_2$ oder -OH darstellt.

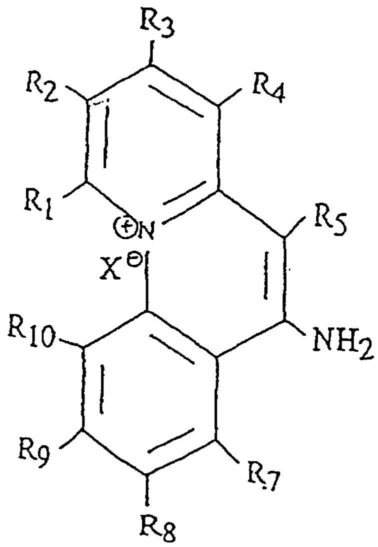

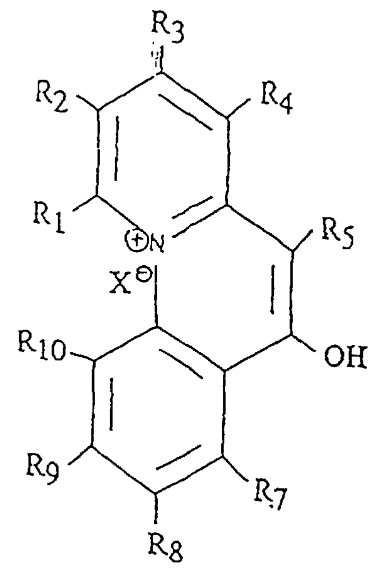

- gegebenenfalls Behandlung der Verbindung der vorstehend erwähnten Formel (III), in welcher Y eine Gruppe $-NH_2$ darstellt, durch ein wie in Anspruch 1 definiertes, die Gruppen $R_e$ und $R_f$ tragendes Derivat, wobei das Derivat gegenüber dem Stickstoffatom, das an dem Kohlenstoff der Position 6 der Verbindung der vorstehend erwähnten Formel (III) gebunden ist, reaktiv ist, insbesondere durch eine Halogenierung von $R_e$ und/oder von $R_f$, wobei, sofern notwendig, die anderen Funktionen, die in der Verbindung der vorstehend erwähnten Formel (III) vorhanden sind und die gegenüber dem Derivat, das die vorstehend erwähnten Gruppen $R_e$ und $R_f$ trägt, reaktiv sind, im vorhinein mit einer Schutzgruppe versehen werden, was zum Erhalt der Verbindung der folgenden Formel (III) führt, in welcher $R_1$ bis $R_5$, $R_7$ bis $R_{10}$ und X so wie vorstehend definiert sind, und Y eine in Anspruch 1 definierte Gruppe $-NR_eR_f$ darstellt,

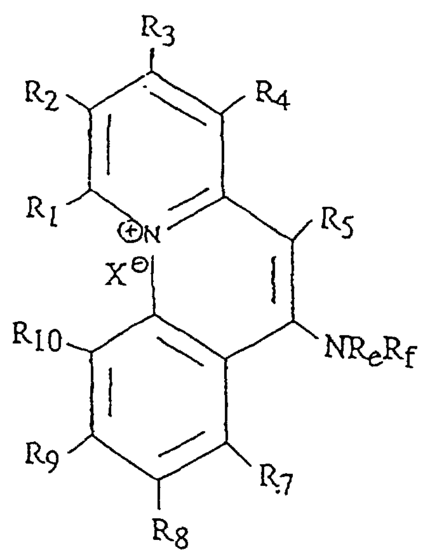

- gegebenenfalls Behandlung der Verbindung der vorstehend erwähnten Formel (III), in welcher Y eine Gruppe -OH darstellt, durch ein wie in Anspruch 1 definierte Gruppe $R_d$ tragendes Derivat, wobei das Derivat gegenüber dem Sauerstoffatom, das an den Kohlenstoff der Position 7 der Verbindung der vorstehend erwähnten Formel (III) gebunden ist, reaktiv ist, insbesondere durch eine Halogenierung von $R_d$, wobei, wenn notwendig, die anderen Funktionen, die in der Verbindung der vorstehend erwähnten Formel (III) vorhanden sind und die gegenüber dem die vorstehend erwähnte Gruppe $R_d$ tragenden Derivat reaktiv sind, im vorhinein mit Schutzgruppen versehen werden, was zum Erhalt der Verbindung der folgenden Formel (III) führt, in welcher $R_1$ bis $R_5$, $R_7$ bis $R_{10}$ und X so wie vorstehend definiert sind, und Y eine in Anspruch 1 definierte Gruppe $-OR_d$ darstellt,

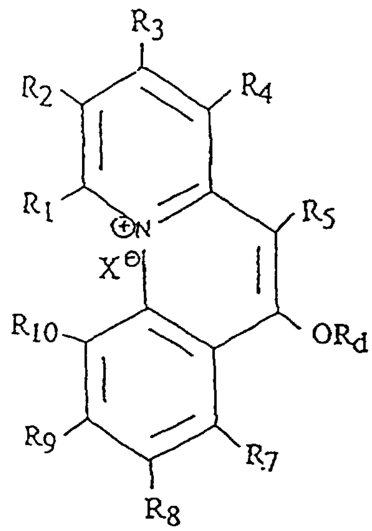

- gegebenenfalls Hydrierung der Verbindungen der vorstehend erwähnten Formeln (II) oder (III), insbesondere durch katalytische Hydrierung in Gegenwart von Platinoxid bei vermindertem Druck, was zum Erhalt der Verbindungen der folgenden Formeln (II) oder (III) führt:

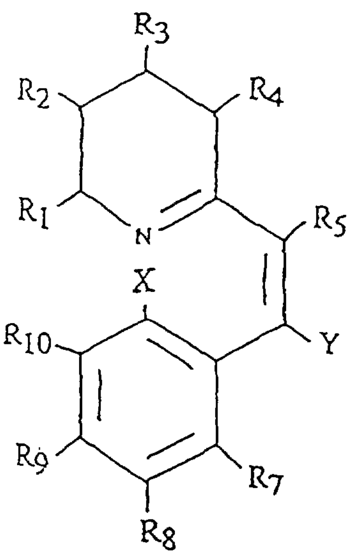

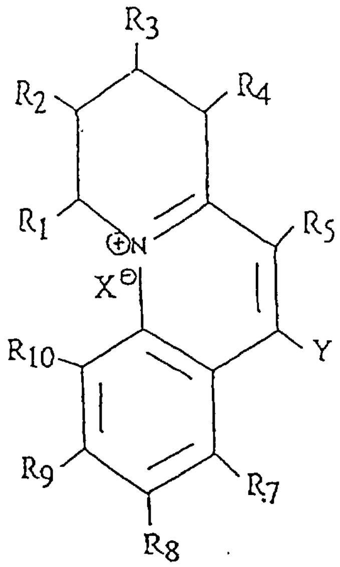

in welchen $R_1$ bis $R_5$, $R_7$ bis $R_{10}$, X und Y so wie vorstehend definiert sind.

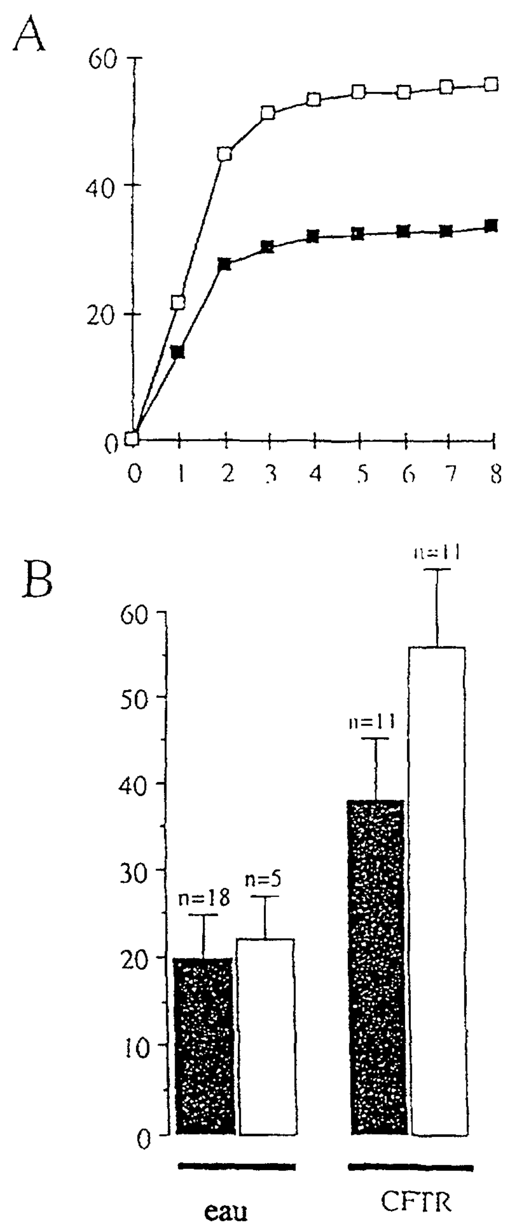


Figure 1

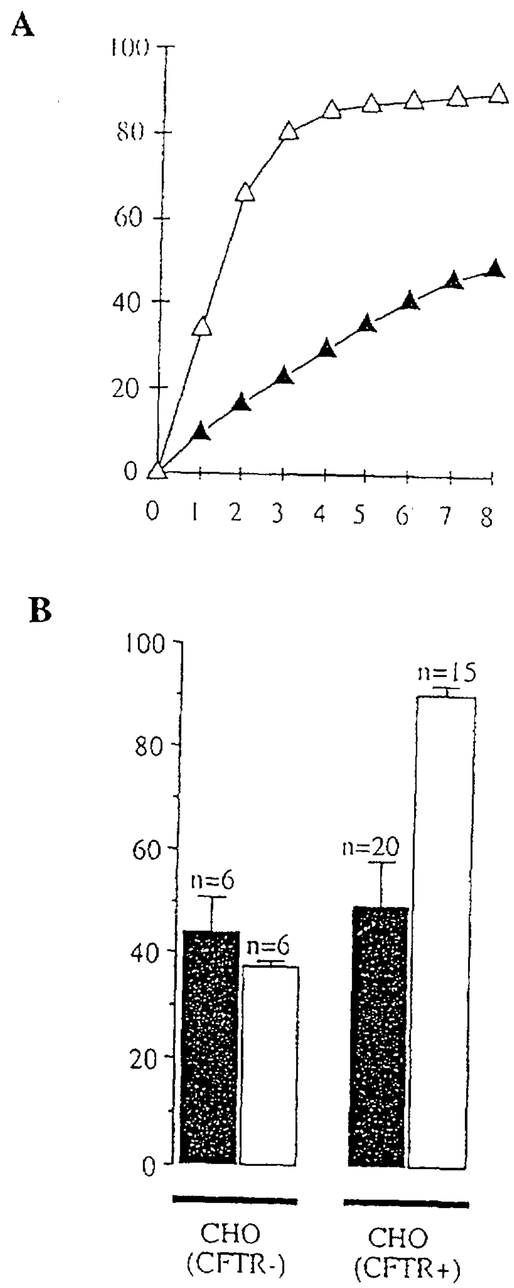
A
100
80
60
40
20
0
0 1 2 3 4 5 6 7 8
B
100
80
60
40
20
0
n=6
n=6
n=20
n=15
CHO
(CFTR-)
CHO
(CFTR+)

Figure 2

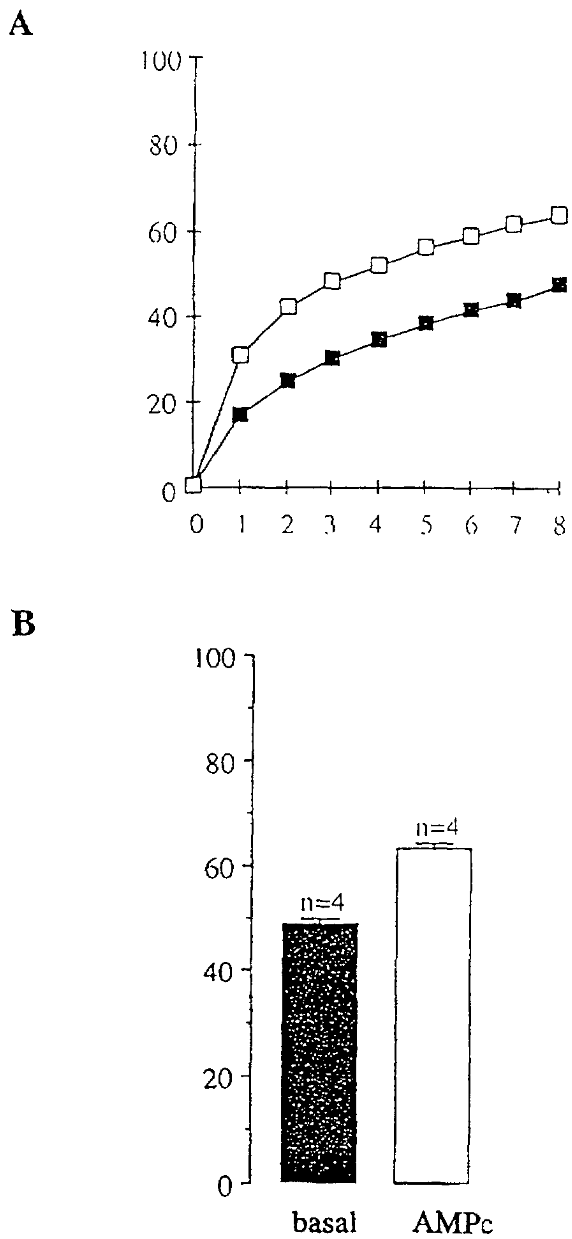


Figure 3

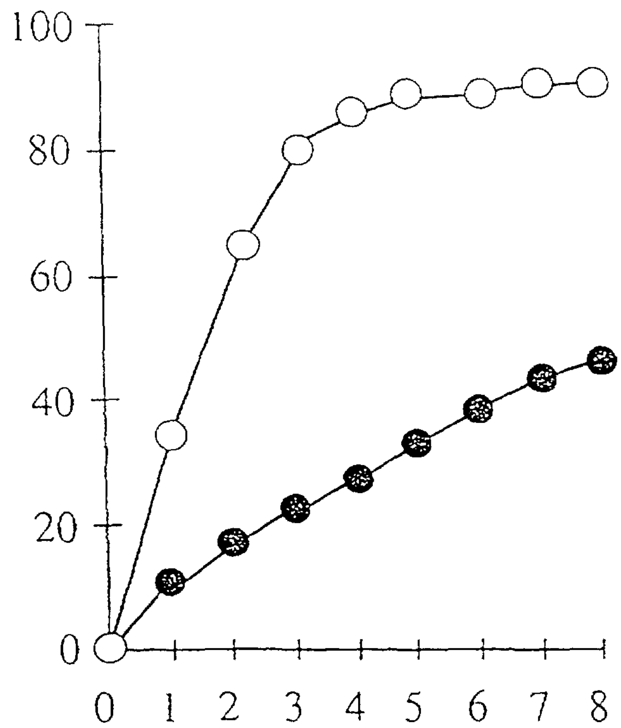


Figure 4

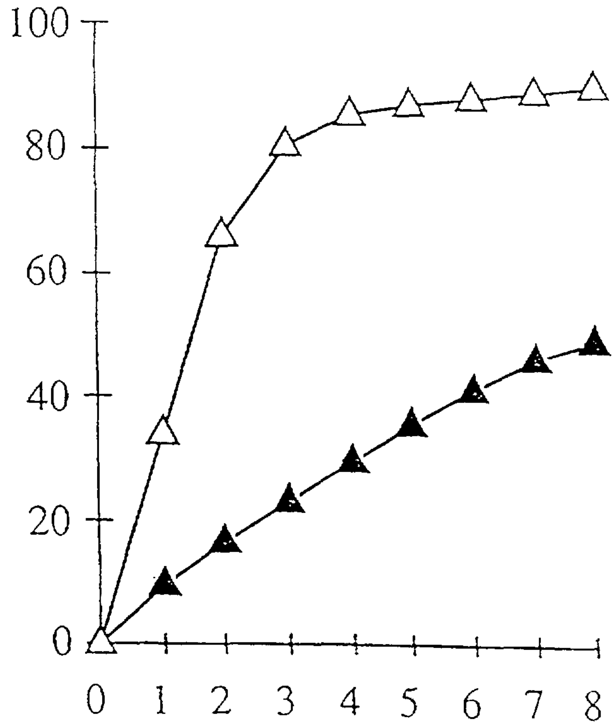


Figure 5

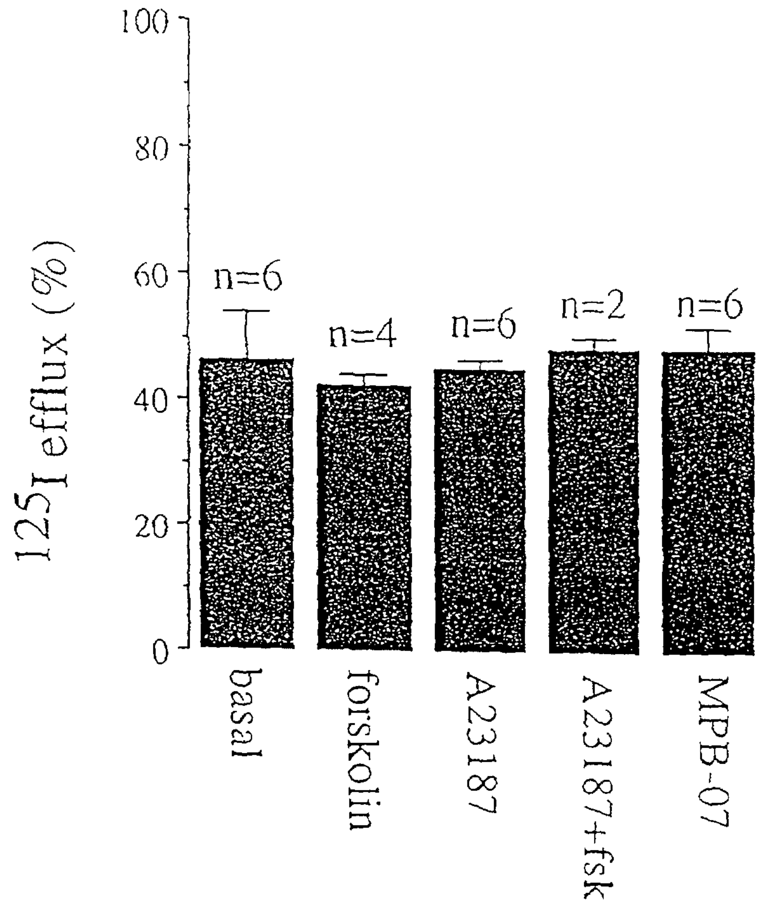

100
80
60
40
20
0
125I efflux (%)
n=6
n=4
n=6
n=2
n=6
basal
forskolin
A23187
A23187+fsk
MPB-07


Figure 6

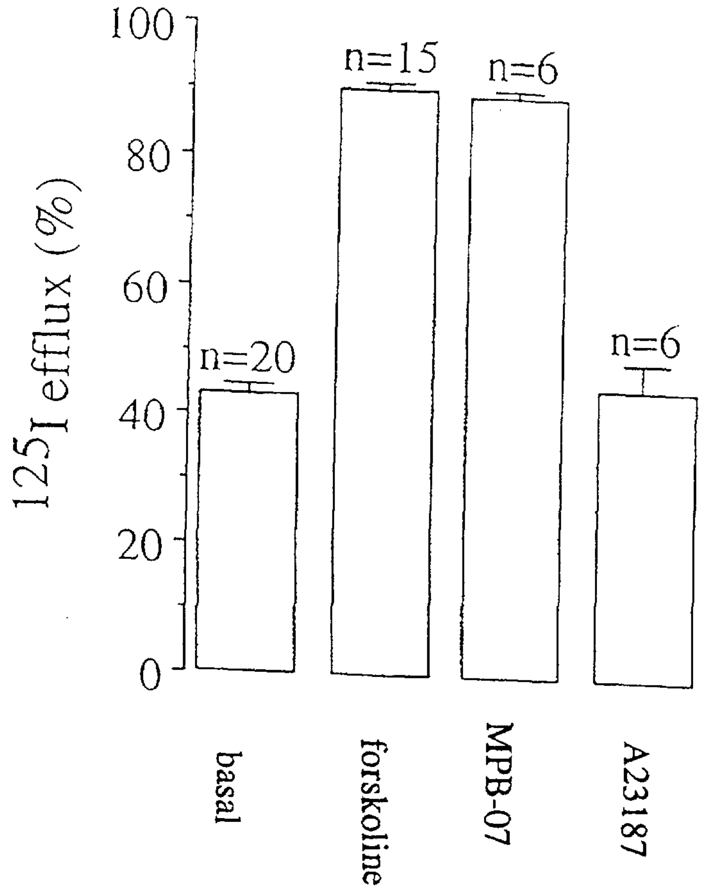
100
80
60
40
20
0
$^{125}$I efflux (%)
n=20
n=15
n=6
n=6
basal
forskoline
MPB-07
A23187

Figure 7

A

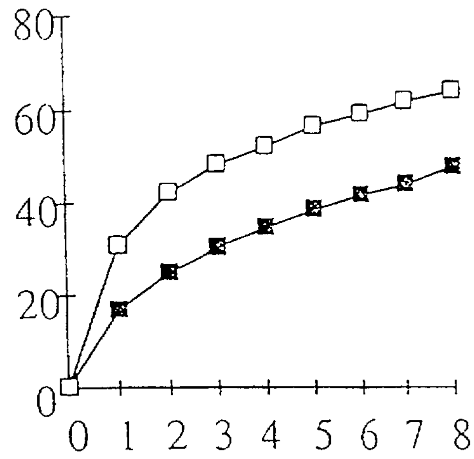


B

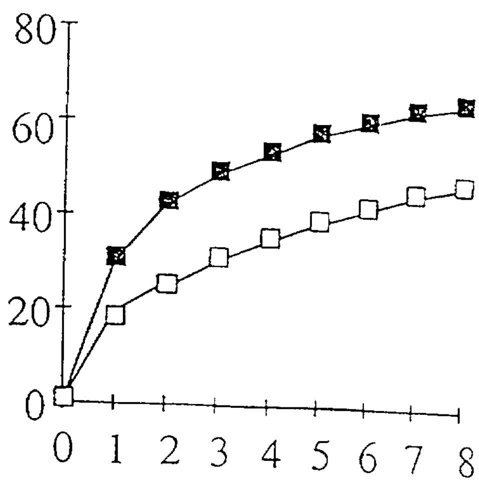


Figure 8

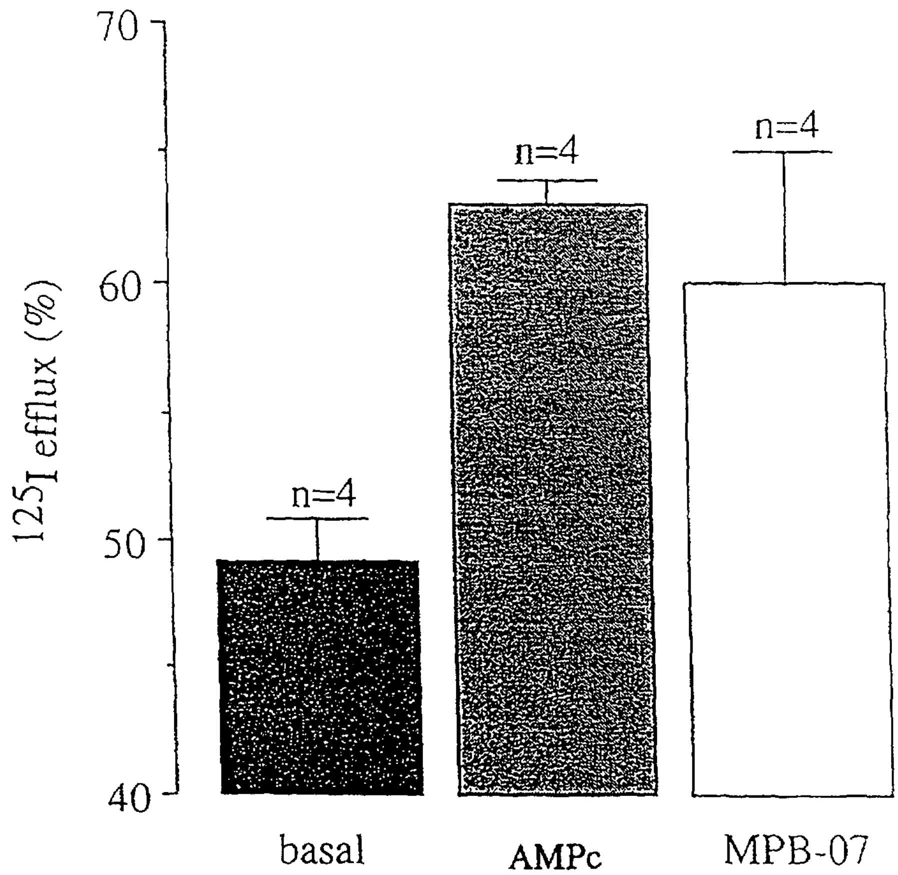


Figure 9

A

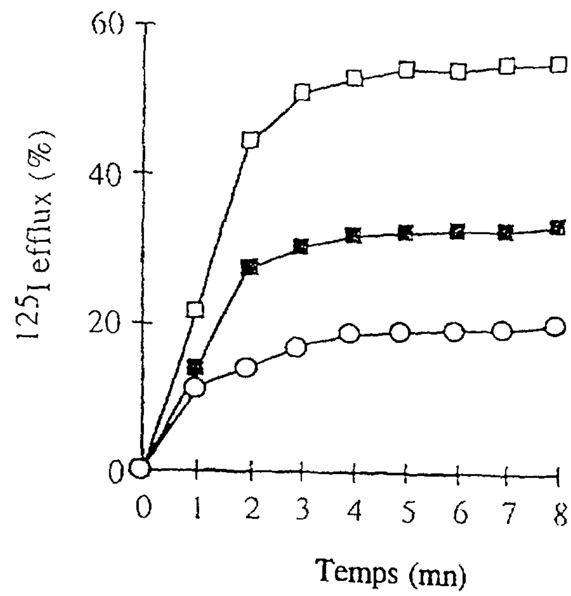
60
40
20
0
$^{125}$I efflux (%)
0 1 2 3 4 5 6 7 8
Temps (mn)

B

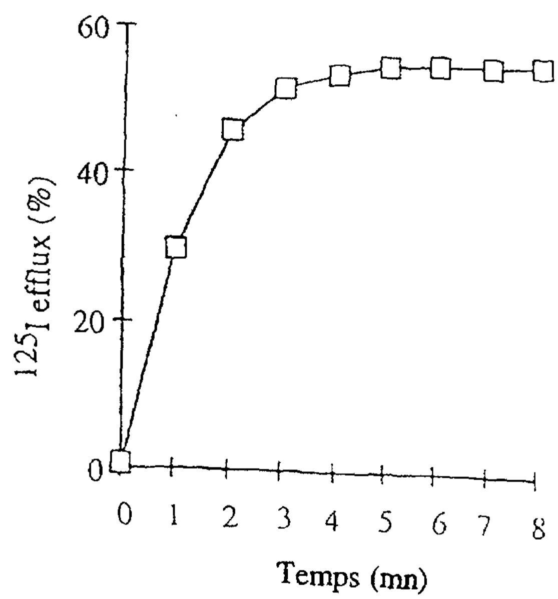
60
40
20
0
$^{125}$I efflux (%)
0 1 2 3 4 5 6 7 8
Temps (mn)

Figure 10

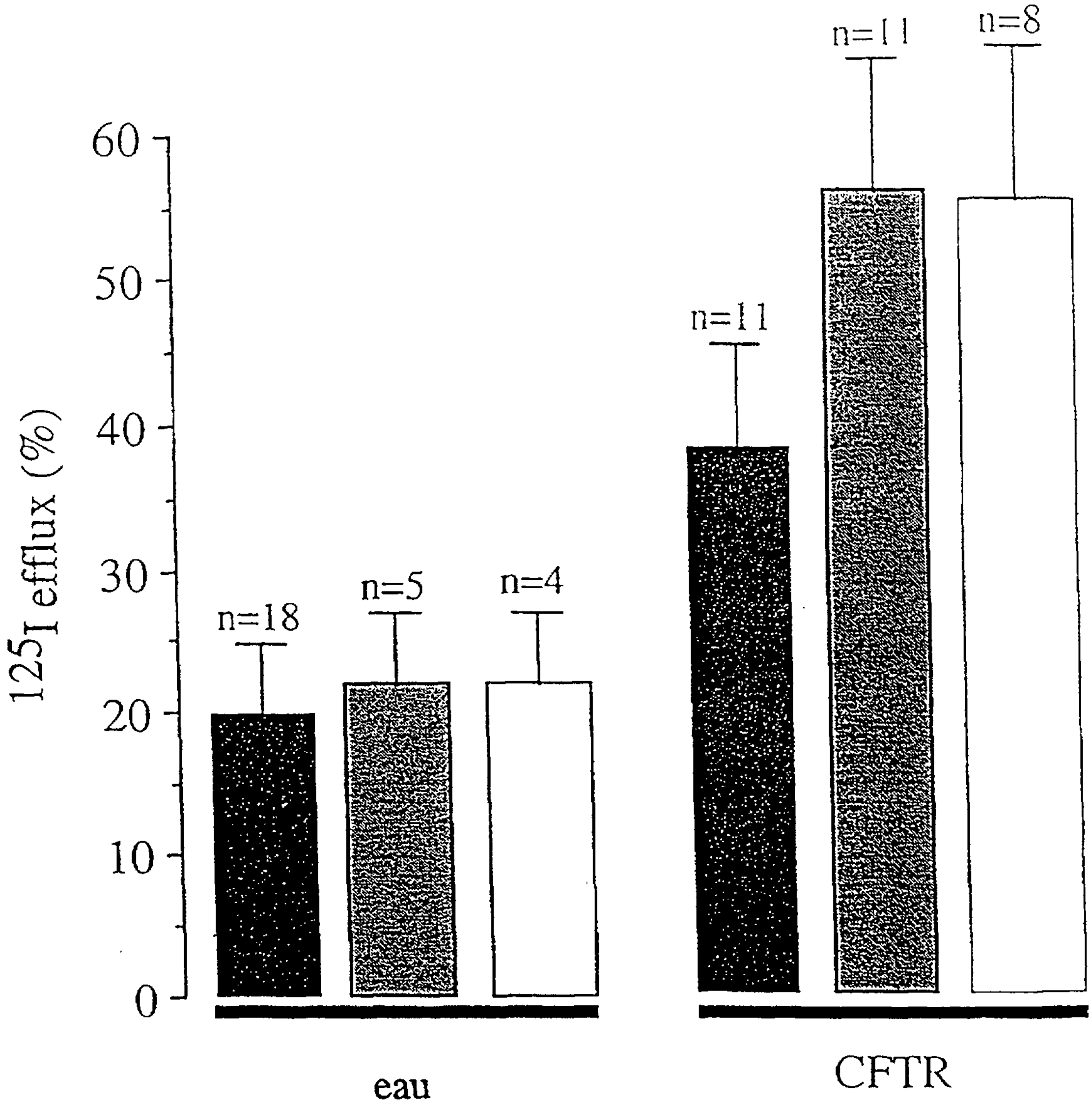


Figure 11

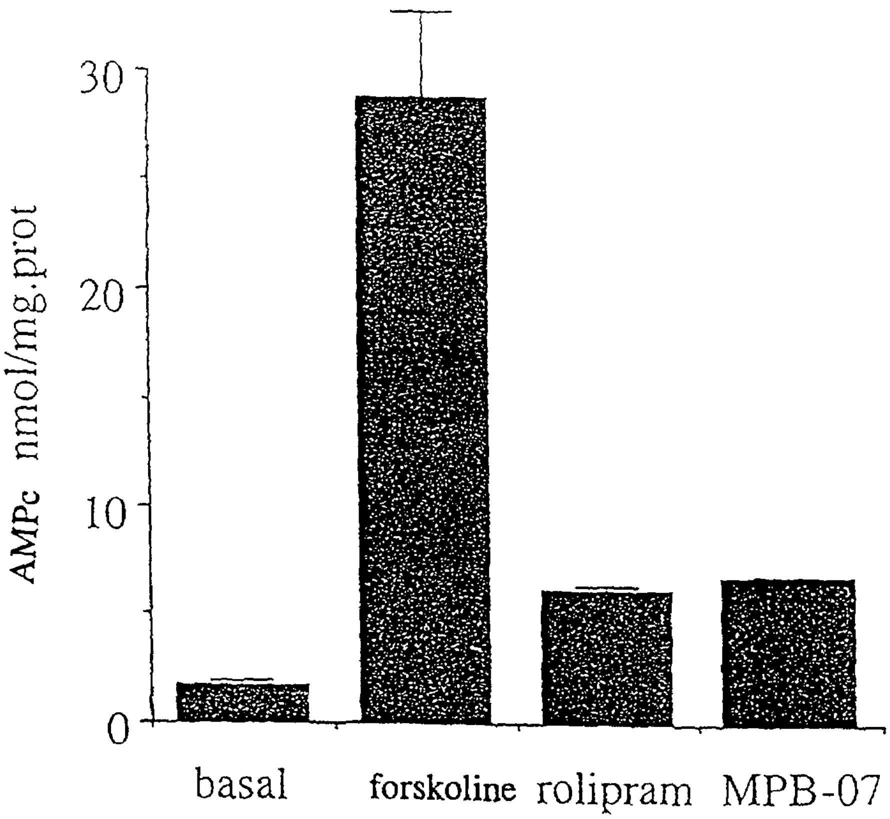


Figure 12